# FASCICULE DE BREVET EUROPEEN

(11) **EP 2 234 966 B1**
(45) Date de publication et mention de la délivrance du brevet: **11.09.2013**
(21) Numéro de dépôt: 08872952.0
(22) Date de dépôt: 16.12.2008
(51) Int. Cl.: C07D 205/04, C07D 413/12, A61K 31/397, A61P 1/00, A61P 3/00, A61P 9/00, A61P 29/00, A61P 35/00

(54) **DERIVES D'AZETIDINES, LEUR PREPARATION ET LEUR APPLICATION EN THERAPEUTIQUE**
AZETIDINDERIVATE, IHRE HERSTELLUNG UND IHRE THERAPEUTISCHE ANWENDUNG
AZETIDINE DERIVATIVES, THEIR PREPARATION AND THEIR APPLICATION IN THERAPY

(30) Priorité: 18.12.2007 FR 0708830; 06.05.2008 FR 0802492
(43) Date de publication de la demande: 06.10.2010
(73) Titulaire: SANOFI, 75008 Paris (FR)
(72) Inventeur: SABUCO, Jean-François, F-75013 Paris (FR)
(74) Mandataire: Le Coupanec, Pascale A.M.P.
(86) Numéro de dépôt international: PCT/FR2008/001748
(87) Numéro de publication internationale: WO 2009/106708

(56) Documents cités:
- WO-A-2004/096763
- WO-A-2007/067617

## Description

La présente invention se rapporte à des dérivés d'azétidines, à leur préparation et à leur application en thérapeutique dans le traitement ou la prévention de maladies impliquant les récepteurs aux cannabinoïdes CB1. WO 2004 096763 et WO 2007 067617 décrivent des composés antagonidés aux cannabinoïdes CB1.

La présente invention a pour objet des composés répondant à la formule (I) 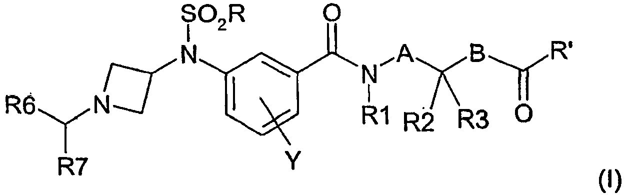 dans laquelle :
R représente un groupe (C₁-C₆)alkyle, halo(C₁-C₆)alkyle;
R' représente un groupe NR4R5, OR8 ;
A et B, s'ils sont présents, représentent indépendamment l'un de l'autre, un ou deux atomes de carbone, ces atomes de carbones étant substitués par un ou plusieurs hydrogènes, groupes (C₁-C₆)alkyle ; le ou les groupes (C₁-C₆)alkyle étant éventuellement substitués par un ou plusieurs groupes hydroxy, (C₁-C₆)alcoxy, halo(C₁-C₆)alkyle, (C₁-C₆)alkylS(O)ₚ, NR4R5, CONR4R5 ;
A+B représentent au maximum deux carbones ;
R1 représente un atome d'hydrogène ou un groupe (C₁-C₆)alkyle ;
R2 et R3 représentent, indépendamment l'un de l'autre, un atome d'hydrogène, un groupe (C₁-C₆)alkyle ; le groupe (C₁-C₆)alkyle étant éventuellement substitué par un ou plusieurs groupes hydroxy, (C₁-C₆)alcoxy, halo(C₁-C₆)alkyle, (C₁-C₆)alkylS(O)ₚ, NR4R5, CONR4R5 ;
R4 et R5 représentent indépendamment l'un de l'autre, un atome d'hydrogène, un groupe (C₁-C₆)alkyle, ou forment ensemble, avec l'atome d'azote qui les porte, un hétérocycle du type azétidine, pyrrolidine, pipéridine, azepane, pipérazine, homopipérazine, morpholine, thiomorpholine, thiomorpholine S-oxyde, thiomorpholine S-dioxyde, cet hétérocycle étant éventuellement substitué par un groupe (C₁-C₆)alkyle ;
R6 et R7 représentent chacun un groupe phényle, éventuellement substitué par un ou plusieurs atomes ou groupes choisis parmi un atome d'hydrogène, un halogène, un groupe (C₁-C₆)alkyle, halo(C₁-C₆)alkyle, (C₁-C₆)alcoxy, halo(C₁-C₆)alcoxy ou cyano;
Y représente un atome d'hydrogène, un halogène, un groupe (C₁-C₆)alkyle, halo(C₁-C₆)alkyle, (C₁-C₆)alcoxy, halo(C₁-C₆)alcoxy, (C₁-C₆)alkylS(O)ₚ ou cyano ;
R8 représente un atome d'hydrogène, un groupe (C₁-C₆)alkyle, un groupe halo(C₁-C₆)alkyle, un groupe allyle, un groupe phényle(C₁-C₆)alkyle, le groupe phényle étant éventuellement substitué par 1 ou 2 groupes O-méthyle,
p représente un nombre entier choisi parmi 0, 1 ou 2 ;
à l'état de base ou de sel d'addition à un acide ou à une base.

Les composés de formule (I) peuvent comporter un ou plusieurs atomes de carbones asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Parmi les composés de formule (I) objets de l'invention, un premier groupe de composés est constitué des composés pour lesquels :
R' est un NR4R5, OR8 ;
R représente un méthyle ;
A et B, s'ils sont présents, représentent indépendamment l'un de l'autre, un -CH₂-;
R1 représente un atome d'hydrogène ;
R2 et R3 représentent indépendamment l'un de l'autre, un atome d'hydrogène, un groupe (C₁-C₆)alkyle ; le groupe (C₁-C₆)alkyle étant éventuellement substitué par un groupe hydroxy ;
R4 et R5 représentent indépendamment l'un de l'autre un atome d'hydrogène ou un méthyle ou forment ensemble, avec l'atome d'azote qui les porte, une morpholine ;
R6 et R7 représentent chacun un groupe phényle, éventuellement substitué par un ou plusieurs atomes ou groupes choisis parmi un halogène, un groupe (C₁-C₆)alkyle, halo(C₁-C₆)alkyle, (C₁-C₆)alcoxy, halo(C₁-C₆)alcoxy ou cyano en position para ;
R8 représente un atome d'hydrogène, un groupe (C₁-C₆)alkyle ;
Y représente un hydrogène, un halogène, un groupe (C₁-C₆)alcoxy ; un groupe (C₁-C₆)alkyle ; un cyano ; un groupe halo(C₁-C₆)alkyle ;
sous forme d'énantiomères ou de diastéréoisomères ou de mélanges de ces formes ;
à l'état de base ou de sel d'addition à un acide ou à une base.

Parmi les composés de formule (I) objets de l'invention, un second groupe de composés est constitué des composés pour lesquels :
R' est un NR4R5, OR8 ;
R représente un méthyle ;
A et B, s'ils sont présents, représentent indépendamment l'un de l'autre, un -CH₂- ;
R1 représente un atome d'hydrogène ;
R2 et R3 représentent indépendamment l'un de l'autre, un atome d'hydrogène, un groupe (C₁-C₆)alkyle ; le groupe (C₁-C₆)alkyle étant éventuellement substitué par un groupe hydroxy ;
R4 et R5 représentent indépendamment l'un de l'autre un atome d'hydrogène ou un méthyle ou forment ensemble, avec l'atome d'azote qui les porte, une morpholine ;
R6 et R7 représentent chacun un groupe phényle substitué par un atome de chlore, fluor, brome, un groupe Me, OMe, CN, CF₃, OCF₃ en position para ;
R8 représente un atome d'hydrogène, un groupe (C₁-C₆)alkyle ;
Y représente un hydrogène, un halogène, un groupe (C₁-C₆)alcoxy ; un groupe (C₁-C₆)alkyle ; un cyano ; un groupe halo(C₁-C₆)alkyle ;
sous forme d'énantiomères ou de diastéréoisomères ou de mélanges de ces formes ;
à l'état de base ou de sel d'addition à un acide ou à une base.

Parmi les composés de formule (I) objets de l'invention, un troisème groupe de composés est constitué des composés pour lesquels :
R' est un NR4R5, OR8 ;
R représente un méthyle ;
A et B, s'ils sont présents, représentent indépendamment l'un de l'autre, un -CH₂- ;
R1 représente un atome d'hydrogène ;
R2 et R3 représentent indépendamment l'un de l'autre, un atome d'hydrogène, un groupe (C₁-C₆)alkyle ; le groupe (C₁-C₆)alkyle étant éventuellement substitué par un groupe hydroxy ;
R4 et R5 représentent indépendamment l'un de l'autre un atome d'hydrogène ou un méthyle ou forment ensemble, avec l'atome d'azote qui les porte, une morpholine,
R6 et R7 représentent chacun un groupe phényle, éventuellement substitué par un ou plusieurs atomes ou groupes choisis parmi un halogène, un groupe (C₁-C₆)alkyle, halo(C₁-C₆)alkyle, (C₁-C₆)alcoxy, halo(C₁-C₆)alcoxy ou cyano en position para ;
R8 représente un atome d'hydrogène, un groupe (C₁-C₆)alkyle ;
Y représente un atome d'hydrogène, de chlore, fluor, brome, un groupe Me, OMe, CN, CF₃,
sous forme d'énantiomères ou de diastéréoisomères ou de mélanges de ces formes ;
à l'état de base ou de sel d'addition à un acide ou à une base.

Parmi les composés de formule (I) objets de l'invention, un quatrième groupe de composés est constitué des composés pour lesquels :
R' est un NR4R5, OR8 ;
R représente un méthyle ;
A et B, s'ils sont présents, représentent indépendamment l'un de l'autre, un -CH₂- ;
R1 représente un atome d'hydrogène ;
R2 et R3 représentent indépendamment l'un de l'autre, un atome d'hydrogène, un groupe (C₁-C₆)alkyle ; le groupe (C₁-C₆)alkyle étant éventuellement substitué par un groupe hydroxy ;
R4 et R5 représentent indépendamment l'un de l'autre un atome d'hydrogène ou un méthyle ou forment ensemble, avec l'atome d'azote qui les porte, une morpholine ;
R6 et R7 représentent chacun un groupe phényle substitué par un atome de chlore, fluor, brome, un groupe Me, OMe, CN, CF₃, OCF₃ en position para ;
R8 représente un atome d'hydrogène, un groupe (C₁-C₆)alkyle ;
Y représente un atome d'hydrogène, de chlore, fluor, brome, un groupe Me, OMe, CN, CF₃,
sous forme d'énantiomères ou de diastéréoisomères ou de mélanges de ces formes ;
à l'état de base ou de sel d'addition à un acide ou à une base.

Dans le cadre de la présente invention, on entend par :
- un halogène : un fluor, un chlore, un brome ou un iode ;
- un groupe (C₁-C₆)alkyle: un groupe aliphatique saturé cyclique, ramifié ou linéaire pouvant éventuellement être substitué par un groupement alkyle cyclique. A titre d'exemples, on peut citer les groupes méthyle, éthyle, propyle, isopropyle, butyle, isobutyle, tertbutyle, pentyle, hexyle, cyclopropyle, cyclobutyle, cyclopentyle, cyclohexyle, cycloheptyle, cyclopropylméthyle, etc ;
- un groupe halo(C₁-C₆)alkyle : un groupe (C₁-C₆)alkyle tel que précédemment défini dont un ou plusieurs atomes d'hydrogène ont été substitués par un atome d'halogène. A titre d'exemples, on peut citer les groupes CF₃, CH₂CF₃, CHF₂, CCl₃ ;
- un groupe (C₁-C₆)alcoxy : un groupe -O-(C₁-C₆)alkyle où le groupe (C₁-C₆)alkyle est tel que précédemment défini.
- un groupe halo(C₁-C₆)alcoxy : un groupe halo(C₁-C₆)alkyle-O- où le groupe halo(C₁-C₆)alkyle est tel que précédemment défini.
- un groupe allyle est un CH2-CH=CH2

Les composés de formule (I) peuvent exister à l'état de bases ou d'acides ou de sels. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Parmi les composés de formule (I) objets de l'invention, on peut notamment citer les composés suivants ; la nomenclature utilisée correspond à la nomenclature IUPAC.
3-({1-[bis-(4-chloro-phényl)-méthyl]-azétidin-3-yl}-méthanesulfonyl-amino)-*N*-((S)-1-carbamoyl-éthyl)-benzamide
3-({1-[bis-(4-chloro-phényl)-méthyl]-azétidin-3-yl}-méthanesulfonyl-amino)-*N-*carbamoylméthyl-5-fluoro-benzamide
3-({1-[bis-(4-chloro-phényl)-méthyl]-azétidin-3-yl}-méthanesulfonyl-amino)-*N-*((S)-1-carbamoyl-éthyl)-5-fluoro-benzamide,
3-({1-[bis-(4-chloro-phényl)-méthyl]-azétidin-3-yl}-méthanesulfonyl-amino)*-N-*((R)-1-carbamoyl-éthyl)-5-fluoro-benzamide
3-({1-[bis-(4-chloro-phényl)-méthyl]-azétidin-3-yl}-méthanesulfonyl-amino)-*N*-((R)-1-carbamoyl-éthyl)-benzamide
3-({1-[bis-(4-chloro-phényl)-méthyl]-azétidin-3-yl}-méthanesulfonyl-amino)-*N*-((S)-1-carbamoyl-2-hydroxy-éthyl)-benzamide
3-({1-[bis-(4-chloro-phényl)-méthyl]-azétidin-3-yl}-méthanesulfonyl-amino)-*N-*(2-carbamoyl-éthyl)-benzamide
3-({1-[bis-(4-chloro-phényl)-méthyl]-azétidin-3-yl}-méthanesulfonyl-amino)-*N-*(2-morpholin-4-yl-2-oxo-éthyl)-benzamide
3-({1-[bis-(4-chloro-phényl)-méthyl]-azétidin-3-yl}-méthanesulfonyl-amino)-*N*-((S)-1-méthylcarbamoyl-éthyl)-benzamide
3-({1-[bis-(4-chloro-phényl)-méthyl]-azétidin-3-yl}-méthanesulfonyl-amino)-*N*-((S)-1-carbamoyl-propyl)-benzamide
3-({1-[bis-(4-chloro-phényl)-méthyl]-azétidin-3-yl}-méthanesulfonyl-amino)-*N*-((S)-1-carbamoyl-3-méthyl-butyl)-benzamide
3-({1-[bis-(4-chloro-phényl)-méthyl]-azétidin-3-yl}-méthanesulfonyl-amino)-*N*-((S)-1-carbamoyl-2-méthyl-propyl)-5-fluoro-benzamide
3-({1-[bis-(4-chloro-phényl)-méthyl]-azétidin-3-yl}-méthanesulfonyl-amino)-*N-*((S)-1-carbamoyl-2-hydroxy-éthyl)-5-fluoro-benzamide
3-({1-[bis-(4-chloro-phényl)-méthyl]-azétidin-3-yl}-méthanesulfonyl-amino)-*N-*((S)-1-carbamoyl-propyl)-5-fluoro-benzamide
3-({1-[bis-(4-chloro-phényl)-methyl]-azétidin-3-yl}-méthanesulfonyl-amino)-*N-*(méthylcarbamoyl-méthyl)-benzamide
3-({1-[bis-(4-chloro-phényl)-méthyl]-azétidin-3-yl}-méthanesulfonyl-amino)-*N*-(carbamoyl-méthyl)-benzamide
Ester tert-butylique de l'acide (S)-2-[3-({1-[bis-(4-chloro-phényl)-méthyl]-azétidin-3-yl}-méthanesulfonyl-amino)-benzoylamino]-propionique
Acide (S)-2-[3-({1-[bis-(4-chloro-phényl)-méthyl]-azétidin-3-yl}-méthanesulfonyl-amino)-benzoylamino]-propionique
Ester tert-butylique de l'acide 3-({1-[bis-(4-chloro-phényl)-méthyl]-azétidin-3-yl}-méthanesulfonyl-amino)-benzoylamino-acétique
Ester tert-butylique de l'acide 4-[3-({1-[bis-(4-chloro-phényl)-méthyl]-azétidin-3-yl}-méthanesulfonyl-amino)-5-fluoro-benzoylamino]-butyrique
Ester tert-butylique de l'acide (S)-2-[3-({1-[bis-(4-chloro-phényl)-méthyl]-azétidin-3-yl}-méthanesulfonyl-amino)-5-fluoro-benzoylamino]-propionique
3-({1-[bis-(4-chloro-phényl)-méthyl]-azétidin-3-yl}-méthanesulfonyl-amino)-*N*-((S)-1-carbamoyl-éthyl)-5-méthoxy-benzamide
3-({1-[bis-(4-chloro-phényl)-méthyl]-azétidin-3-yl}-méthanesulfonyl-amino)-*N*-((S)-1-diméthylcarbamoyl-éthyl)-benzamide
Ester tert-butylique de l'acide 4-[3-({1-[bis-(4-chloro-phényl)-méthyl]-azétidin-3-yl}-méthanesulfonyl-amino)-benzoylamino]-butyrique
Acide (S)-2-[3-({1-[bis-(4-chloro-phényl)-méthyl]-azétidin-3-yl}-méthanesulfonyl-amino)-5-fluoro-benzoylamino]-propionique et son sel de sodium
3-({1-[bis-(4-chloro-phényl)-méthyl]-azétidin-3-yl}-méthanesulfonyl-amino)-5-fluoro-*N-*((S)-1-méthylcarbamoyl-éthyl)-benzamide
3-({1-[bis-(4-chloro-phényl)-méthyl]-azétidin-3-yl}-méthanesulfonyl-amino)-*N*-((1S,2R)-1-carbamoyl-2-hydroxy-propyl)-benzamide
3-({1-[bis-(4-chloro-phényl)-méthyl]-azétidin-3-yl}-méthanesulfonyl-amino)-*N-*(3-carbamoyl-propyl)-5-fluoro-benzamide
Acide [3-({1-[bis-(4-chloro-phényl)-méthyl]-azétidin-3-yl}-méthanesulfonyl-amino)-benzoylamino]-acétique
3-({1-[Bis-(4-chloro-phenyl)-methyl]-azetidin-3-yl}-methanesulfonyl-amino)-*N-*((S)-1-carbamoyl-ethyl)-2-fluoro-benzamide
3-({1-[Bis-(4-chloro-phenyl)-methyl]-azetidin-3-yl}-methanesulfonyl-amino)-*N*-((S)-1-methylcarbamoyl-ethyl)-2-fluoro-benzamide
3-({1-[Bis-(4-chloro-phenyl)-methyl]-azetidin-3-yl}-methanesulfonyl-amino)-*N-*((S)-1-carbamoyl-ethyl)-6-fluoro-benzamide
3-({1-[Bis-(4-chloro-phenyl)-methyl]-azetidin-3-yl}-methanesulfonyl-amino)-*N-*((S)-1-carbamoyl-propyl)-6-fluoro-benzamide
3-({1-[Bis-(4-chloro-phenyl)-methyl]-azetidin-3-yl}-methanesulfonyl-amino)-*N-*((S)-1-carbamoyl-2-methyl-propyl)-6-fluoro-benzamide
3-({1-[Bis-(4-chloro-phenyl)-metllyl]-azetidin-3-yl}-methanesulfonyl-amino)-*N*-((S)-1-methylcarbamoyl-ethyl)-6-fluoro-benzamide
3-({1-[Bis-(4-chloro-phenyl)-methyl]-azetidin-3-yl}-methanesulfonyl-amino)-*N*-((S)-1-carbamoyl-propyl)-4-fluoro-benzamide
3-({1-[Bis-(4-chloro-phenyl)-methyl]-azetidin-3-yl}-methanesulfonyl-amino)-*N-*carbamoylmethyl-5-méthyl-benzamide
3-({1-[Bis-(4-chloro-phenyl)-methyl]-azetidin-3-yl}-methanesulfonyl-amino)-*N*-((S)-1-carbamoyl-ethyl)-5-méthyl-benzamide
3-({1-[Bis-(4-chloro-phenyl)-methyl]-azetidin-3-yl}-methanesulfonyl-amino)-*N*-((S)-1-carbamoyl-propyl)-5-méthyl-benzamide
3-({1-[Bis-(4-chloro-phenyl)-methyl]-azetidin-3-yl}-methanesulfonyl-amino)-*N-*((S)-1-carbamoyl-2-methyl-propyl)-5-méthyl-benzamide
3-({1-[Bis-(4-chloro-phenyl)-methyl]-azetidin-3-yl}-methanesulfonyl-amino)-*N*-((S)-1-methylcarbamoyl-ethyl)-5-méthyl-benzamide
3-({1-[Bis-(4-chloro-phenyl)-methyl]-azetidin-3-yl}-methanesulfonyl-amino)-*N-*((S)-1-carbamoyl-2-hydroxy-ethyl)-5-méthyl-benzamide
3-({1-[Bis-(4-chloro-phenyl)-methyl]-azetidin-3-yl}-methanesulfonyl-amino)-*N*-((S)-1-carbamoyl-ethyl)-5-chloro-benzamide
3-({1-[Bis-(4-chloro-phenyl)-methyl]-azetidin-3-yl}-methanesulfonyl-amino)-*N*-((S)-1-carbamoyl-propyl)-5-chloro-benzamide
3-({1-[Bis-(4-chloro-phenyl)-methyl]-azetidin-3-yl}-methanesulfonyl-amino)-*N-*((S)-1-carbamoyl-2-methyl-propyl)-5-chloro-benzamide
3-({1-[Bis-(4-chloro-phenyl)-methyl]-azetidin-3-yl}-methanesulfonyl-amino)-*N*-((S)-1-methylcarbamoyl-ethyl)-5-chloro-benzamide
3-({1-[Bis-(4-chloro-phenyl)-methyl]-azetidin-3-yl}-methanesulfonyl-amino)-*N*-((S)-1-carbamoyl-2-hydroxy-ethyl)-5-chloro-benzamide
3-({1-[Bis-(4-chloro-phenyl)-methyl]-azetidin-3-yl}-methanesulfonyl-amino)-*N-*((S)-1-carbamoyl-ethyl)-5-bromo-benzamide
3-({1-[Bis-(4-chloro-phenyl)-methyl]-azetidin-3-yl}-methanesulfonyl-amino)-*N*-((S)-1-carbamoyl-propyl)-5-bromo-benzamide
3-({1-[Bis-(4-chloro-phenyl)-methyl]-azetidin-3-yl}-methanesulfonyl-amino)-*N-*((S)-1-carbamoyl-2-methyl-propyl)-5-bromo-benzamide
3-({1-[Bis-(4-chloro-phenyl)-methyl]-azetidin-3-yl}-methanesulfonyl-amino)-*N*-((S)-1-methylcarbamoyl-ethyl)-5-bromo-benzamide
3-({1-[Bis-(4-chloro-phenyl)-methyl]-azetidin-3-yl}-methanesulfonyl-amino)-*N*-((S)-1-carbamoyl-2-hydroxy-ethyl)-5-bromo-benzamide
3-({1-[Bis-(4-chloro-phenyl)-methyl]-azetidin-3-yl}-methanesulfonyl-amino)-*N-*carbamoylmethyl-5-trifluorométhyl-benzamide
3-({1-[Bis-(4-chloro-phenyl)-methyl]-azetidin-3-yl}-methanesulfonyl-amino)-*N*-((S)-1-carbamoyl-ethyl)-5-trifluorométhyl-benzamide
3-({1-[Bis-(4-chloro-phenyl)-methyl]-azetidin-3-yl}-methanesulfonyl-amino)-*N*-((S)-1-carbamoyl-propyl)-5-trifluorométhyl-benzamide
3-({1-[Bis-(4-chloro-phenyl)-methyl]-azetidin-3-yl}-methanesulfonyl-amino)-*N*-((S)-1-carbamoyl-2-methyl-propyl)-5-trifluorométhyl-benzamide
3-({1-[Bis-(4-chloro-phenyl)-methyl]-azetidin-3-yl}-methanesulfonyl-amino)-*N*-((S)-1-methylcarbamoyl-ethyl)-5-trifluorométhyl-benzamide
3-({1-[Bis-(4-chloro-phenyl)-methyl]-azetidin-3-yl}-methanesulfonyl-amino)-*N-*((S)-1-carbamoyl-2-hydroxy-ethyl)-5-trifluorométhyl-benzamide
3-({1-[Bis-(4-chloro-phenyl)-methyl]-azetidin-3-yl}-methanesulfonyl-amino)-*N-*((S)-1-carbamoyl-ethyl)-5-cyano-benzamide
3-({1-[Bis-(4-chloro-phenyl)-methyl]-azetidin-3-yl}-methanesulfonyl-amino)-*N-*((S)-1-carbamoyl-propyl)-5-cyano-benzamide
3-({1-[Bis-(4-chloro-phenyl)-methyl]-azetidin-3-yl}-methanesulfonyl-amino)-*N*-((S)-1-carbamoyl-2-methyl-propyl)-5-cyano-benzamide
3-({1-[Bis-(4-chloro-phenyl)-methyl]-azetidin-3-yl}-methanesulfonyl-amino)-*N*-((S)-1-methylcarbamoyl-ethyl)-5-cyano-benzamide
3-({1-[Bis-(4-chloro-phenyl)-methyl]-azetidin-3-yl}-methanesulfonyl-amino)-*N*-((S)-1-carbamoyl-2-hydroxy-ethyl)-5-cyano-benzamide
Acide (S)-2-[3-({1-[bis-(4-chloro-phényl)-méthyl]-azétidin-3-yl}-méthanesulfonyl-amino)-6-fluoro-benzoylamino]-propionique et son sel de l'acide trifluoroacétique.
Acide (S)-2-[3-({1-[bis-(4-chloro-phényl)-méthyl]-azétidin-3-yl}-méthanesulfonyl-amino)-5-méthyl-benzoylamino]-propionique et son sel de l'acide trifluoroacétique.
Acide (S)-2-[3-({1-[bis-(4-chloro-phényl)-méthyl]-azétidin-3-yl}-méthanesulfonyl-amino)-5-chloro-benzoylamino]-propionique et son sel de l'acide trifluoroacétique.
Acide (S)-2-[3-({1-[bis-(4-chloro-phényl)-méthyl]-azétidin-3-yl}-méthanesulfonyl-amino)-5-bromo-benzoylamino]-propionique et son sel de l'acide trifluoroacétique
Acide (S)-2-[3-({1-[bis-(4-chloro-phényl)-méthyl]-azétidin-3-yl}-méthanesulfonyl-amino)-3-trifluorométhyl-benzoylamino]-propionique et son sel de l'acide trifluoroacétique
Acide (S)-2-[3-({1-[bis-(4-chloro-phényl)-méthyl]-azétidin-3-yl}-méthanesulfonyl-amino)-5-cyano-benzoylamino]-propionique et son sel de l'acide trifluoroacétique
3-({1-[Bis-(4-bromo-phenyl)-methyl]-azetidin-3-yl}-methanesulfonyl-amino)-*N-*((S)-1-carbamoyl-ethyl)-5-fluoro-benzamide
3-({1-[Bis-(4-trifluoromethyl-phenyl)-methyl]-azetidin-3-yl}-methanesulfonyl-amino)-*N*-((S)-1-carbamoyl-ethyl)-5-fluoro-benzamide
3-({1-[Bis-(4-cyano-phenyl)-methyl]-azetidin-3-yl}-methanesulfonyl-amino)-*N-*((S)-1-carbamoyl-ethyl)-5-fluoro-benzamide
3-({1-[Bis-(4-methoxy-phenyl)-methyl]-azetidin-3-yl}-methanesulfonyl-amino)-*N-*((S)-1-carbamoyl-ethyl)-5-fluoro-benzamide
3-({1-[Bis-(4-fluoro-phenyl)-methyl]-azetidin-3-yl}-methanesulfonyl-amino)-*N-*((S)-1-carbamoyl-ethyl)-5-fluoro-benzamide
Ester methylique de l'acide (S)-2-[3-({1-[Bis-(4-chloro-phenyl)-methyl]-azetidin-3-yl}-methanesulfonyl-amino)-5-fluoro-benzoylamino]-propionique
3-({1-[Bis-(4-methyl-phenyl)-methyl]-azetidin-3-yl}-methanesulfonyl-amino)-*N-*((S)-1-carbamoyl-propyl)-5-fluoro-benzamide
3-({1-[Bis-(4-bromo-phenyl)-methyl]-azetidin-3-yl}-methanesulfonyl-amino)-*N*-((S)-1-methylcarbamoyl-ethyl)-5-fluoro-benzamide
Acide (S)-2-[3-({1-[Bis-(4-bromo-phenyl)-methyl]-azetidin-3-yl}-methanesulfonyl-amino)-5-fluoro-benzoylamino]-propionique
Ester éthylique de l'acide (S)-2-[3-({1-[Bis-(4-chloro-phenyl)-methyl]-azetidin-3-yl}-methanesulfonyl-amino)-5-fluoro-benzoylamino]-propionique
3-({1-[Bis-(4-trifluoromethoxy-phenyl)-methyl]-azetidin-3-yl}-methanesulfonyl-amino)-*N*-((S)-1-carbamoyl-propyl)-5-fluoro-benzamide
leurs isomères optiques et leurs sels pharmaceutiquement acceptables.

La présente invention a également comme objet l'utilisation des composés de l'invention de formule (I) pour la préparation d'un médicament pour le traitement ou la prévention des maladies dans lesquelles le récepteur CB1 est impliqué.

La présente invention a également comme objet l'utilisation des composés de l'invention de formule (I) pour la préparation d'un médicament pour le traitement ou la prévention des désordres psychiatriques, de la dépendance et du sevrage à une substance, du sevrage tabagique, des troubles cognitifs et de l'attention et des maladies neurodégénératives aiguës et chroniques ; du métabolisme, des troubles de l'appétence, des troubles de l'appétit, de l'obésité, du diabète (de type I et/ou II), du syndrome métabolique, de la dyslipidémie, de l'apnée du sommeil ; de la douleur, de la douleur neuropathique, des douleurs neuropathiques induites par les anticancéreux ; des troubles gastro-intestinaux, des vomissements, de l'ulcère, des troubles diarrhéiques, des troubles vésicaux et urinaires, des troubles d'origine endocrinienne, des troubles cardiovasculaires, de l'hypotension, du choc hémorragique, du choc septique, des maladies du foie, de la cirrhose chronique du foie, des fibroses, de la stéatohépatite non alcoolique (NASH), de la stéatohépatite et de la stéatose hépatique, quelle que soit l'étiologie de ces affections (alcool, médicament, produit chimique, maladie auto-immune, obésité, diabète, maladie métabolique congénitale) ; des maladies du système immunitaire, de l'arthrite rhumatoïde, de la démyélinisation, de la sclérose en plaques, des maladies inflammatoires ; de la maladie d'Alzheimer, de Parkinson, de la schizophrénie, des troubles cognitifs associés à la schizophrénie, au diabète, à l'obésité, au syndrome métabolique ; l'asthme, des maladies pulmonaires chronique obstructive, du syndrome de Raynaud, du glaucome, des troubles de la fertilité ; des maladies infectieuses et virales telles que les encéphalites, des accidents vasculaires cérébraux, du syndrome de Guillain-Barré, de l'ostéoporose et l'apnée du sommeil et pour la chimiothérapie anticancéreuse ; les troubles liés aux traitements antipsychotiques (prise de poids, trouble du métabolisme).

Conformément à l'invention, on peut préparer les composés de formule générale (I) selon le procédé décrit dans le schéma 1 :

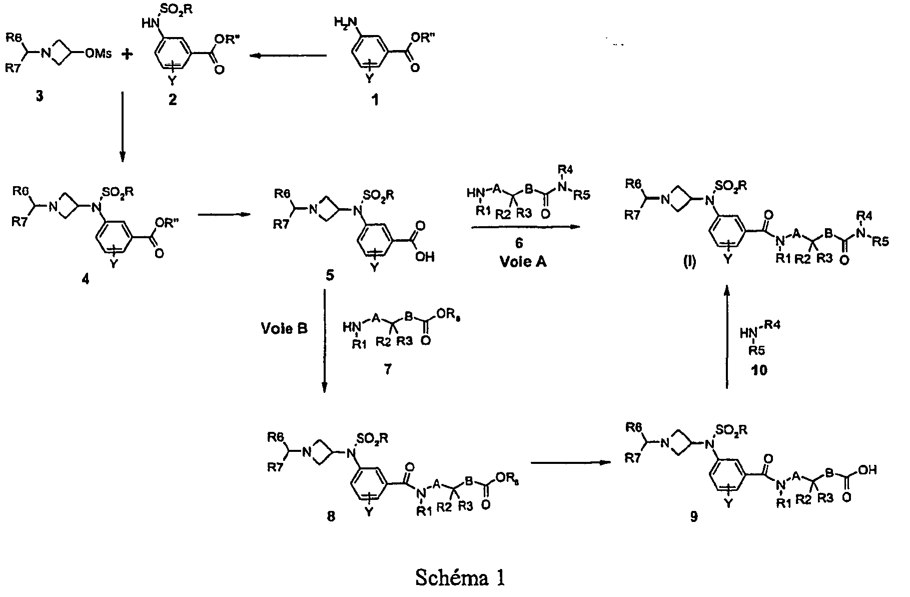

La mésylation du composé 1 en dérivé 2 peut se faire selon les méthodes connues de l'homme de l'art ou bien décrite dans T.W. GREENE, Protective Group in Organic Synthesis, third edition. Cette réaction peut se faire dans un solvant chloré tel que le dichlorométhane en présence d'une base telle que la pyridine et d'un dérivé mésylate tel que le chlorure de mésyle à une température comprise entre -10°C et 40°C.

Les dérivés 1 sont commerciaux ou synthétisés, selon les méthodes connues de l'homme du métier, à partir des précurseurs adéquats commerciaux, R" représente un groupement R8 à l'exception de l'atome d'hydrogène.

Le dérivé 4 est accessible par réaction du mésylate 2 avec l'azétidine 3. Cette étape s'effectue de préférence sous atmosphère inerte, au sein d'un solvant inerte tel que la 4-méthyl-2-pentanone en présence d'une base minérale comme le carbonate de potassium au reflux du mélange réactionnel.

La synthèse de l'azétidine 3 est décrite dans la demande de brevet WO01064634.

L'hydrolyse de l'ester 4 en acide 5 s'effectue d'après les méthodes connues de l'homme de l'art et, plus précisément, dans un mélange de solvants polaires tels que le tétrahydrofuranne et l'eau en présence d'une base telle que l'hydroxyde de lithium hydratée à une température voisine de 20°C.

La formation des composés de formule (I) peut se faire, selon la voie A, par réaction entre l'acide 5 et un dérivé aminé 6. Cette réaction peut être effectuée au sein d'un solvant inerte tel que le tétrahydrofuranne, un solvant chloré (dichlorométhane par exemple), en présence ou non d'une base telle qu'une trialkylamine (triéthylamine par exemple), d'un agent de couplage tel que le chlorhydrate de 1-(3-diméthylaminopropyl)-3-éthylcarbodiimide, en présence ou absence d'un additif évitant toute racémisation tel que le 1-hydroxybenzotriazole et en présence ou non d'un agent favorisant la synthèse peptidique via la formation d'un anhydride mixte tel que l'isobutylchloroformate, à une température comprise entre -20 °C et la température d'ébullition du solvant. Cette réaction peut également être effectuée dans un solvant inerte, en présence d'un agent de couplage et éventuellement d'un additif évitant la racémisation, le produit isolé étant éventuellement transformé en sel d'addition à un acide.

Les dérivés 6 sont commerciaux ou synthétisés, selon les méthodes connues de l'homme du métier, à partir des précurseurs adéquats commerciaux.

La formation des composés de formule 8 peut se faire, selon la voie B, par réaction entre l'acide 5 et un dérivé aminé 7. Cette réaction peut être effectuée selon les conditions précédemment décrites permettant d'accéder aux composés de formule (I) à partir de l'acide 5 et du dérivé aminé 6.

Les dérivés 7 sont commerciaux ou synthétisés, selon les méthodes connues de l'homme du métier, à partir des précurseurs adéquats commerciaux.

L'hydrolyse de l'ester 8 en acide 9 s'effectue d'après les méthodes connues de l'homme de l'art.

La formation des composés de formule (I) peut se faire par réaction entre l'acide 9 et une amine 10. Cette réaction peut être effectuée selon les conditions précédemment décrites permettant d'accéder aux composés de formule (I) à partir de l'acide 5 et du dérivé aminé 6.

Les dérivés 10 sont commerciaux ou synthétisés, selon les méthodes connues de l'homme du métier, à partir des précurseurs adéquats commerciaux.

Les composés de formule (I) peuvent être transformés en d'autres composés de formule (I) par changement de groupement CHR6R7 par débenzhydrylation par des méthodes connues de l'homme du métier suivi par une alkylation avec un groupe CHXR6R7 en présence d'une base, X étant un groupement partant (par exemple un halogène).

Les composés de formule (I) peuvent être purifiés par les méthodes connues habituelles, par exemple par cristallisation, chromatographie ou extraction.

Les énantiomères des composés de formule (I) peuvent être obtenus par dédoublement des racémiques, par exemple, par chromatographie sur colonne chirale selon PIRKLE W.H. et coll., Asymmetric Synthesis, vol. 1, Academic Press (1983) ou, par formation de sels ou par synthèse à partir des précurseurs chiraux. Les diastéréoisomères peuvent être préparés selon les méthodes classiques connues (cristallisation, chromatographie ou à partir des précurseurs chiraux).

La présente invention concerne également le procédé de préparation des intermédiaires.

L'invention, selon un autre de ses aspects, a également pour objet les composés de formules 4 et 5. Ces composés sont utiles comme intermédiaires de synthèse des composés de formule (I).

Les intermédiaires utiles pour la synthèse des composés de formule (I) sont illustrés de manière non limitative ci-dessous
Ester méthylique de l'acide 3-({1-[bis-(4-chloro-phényl)-méthyl]-azétidin-3-yl}-méthanesulfonyl-amino)-5-fluoro-benzoïque
Ester méthylique de l'acide 3-({1-[bis-(4-fluoro-phényl)-méthyl]-azétidin-3-yl}-méthanesulfonyl-amino)-5-fluoro-benzoïque
Acide 3-({1-[bis-(4-fluoro-phényl)-méthyl]-azétidin-3-yl}-méthanesulfonyl-amino)-5-fluoro-benzoïque
Ester éthylique de l'acide 3-({1-[bis-(4-bromo-phényl)-méthyl]-azétidin-3-yl}-méthanesulfonyl-amino)-5-fluoro-benzoïque
Acide 3-({1-[bis-(4-bromo-phényl)-méthyl]-azétidin-3-yl}-méthanesulfonyl-amino)-5-fluoro-benzoïque
Ester éthylique de l'acide 3-({1-[bis-(4-trifluorométhyl-phényl)-méthyl]-azétidin-3-yl}-méthanesulfonyl-amino)-5-fluoro-benzoïque
Acide 3-({1-[bis-(4-trifluorométhyl-phényl)-méthyl]-azétidin-3-yl}-méthanesulfonyl-amino)-5-fluoro-benzoïque
Ester éthylique de l'acide 3-({1-[bis-(4-méthoxy-phényl)-méthyl]-azétidin-3-yl}-méthanesulfonyl-amino)-5-fluoro-benzoïque
Acide 3-({1-[bis-(4-méthoxy-phényl)-méthyl]-azétidin-3-yl}-méthanesulfonyl-amino)-5-fluoro-benzoïque
Ester méthylique de l'acide 3-({1-[bis-(4-méthyl-phényl)-méthyl]-azétidin-3-yl}-méthanesulfonyl-amino)-5-fluoro-benzoïque
Acide 3-({1-[bis-(4-méthyl-phényl)-méthyl]-azétidin-3-yl}-méthanesulfonyl-amino)-5-fluoro-benzoïque
Ester éthylique de l'acide 3-({1-[bis-(4-cyano-phényl)-méthyl]-azétidin-3-yl}-méthanesulfonyl-amino)-5-fluoro-benzoïque
Acide 3-({1-[bis-(4-cyano-phényl)-méthyl]-azétidin-3-yl}-méthanesulfonyl-amino)-5-fluoro-benzoïque
Ester méthylique de l'acide 3-({1-[bis-(4-trifluorométhoxy-phényl)-méthyl]-azétidin-3-yl}-méthanesulfonyl-amino)-5-fluoro-benzoïque
Acide 3-({1-[bis-(4-trifluorométhoxy-phényl)-méthyl]-azétidin-3-yl}-méthanesulfonyl-amino)-5-fluoro-benzoïque
Ester méthylique de l'acide 3-({1-[bis-(4-chloro-phényl)-méthyl]-azétidin-3-yl}-méthanesulfonyl-amino)-2-fluoro-benzoïque
Acide 3-({1-[bis-(4-chloro-phényl)-méthyl]-azétidin-3-yl}-méthanesulfonyl-amino)-2-fluoro-benzoïque
Ester éthylique de l'acide 3-({1-[bis-(4-chloro-phényl)-méthyl]-azétidin-3-yl}-méthanesulfonyl-amino)-4-fluoro-benzoïque
Acide 3-({1-[bis-(4-chloro-phényl)-méthyl]-azétidin-3-yl}-méthanesulfonyl-amino)-4-fluoro-benzoïque
Ester méthylique de l'acide 3-({1-[bis-(4-chloro-phényl)-méthyl]-azétidin-3-yl}-méthanesulfonyl-amino)-6-fluoro-benzoïque
Acide 3-({1-[bis-(4-chloro-phényl)-méthyl]-azétidin-3-yl}-méthanesulfonyl-amino)-6-fluoro-benzoïque
Ester méthylique de l'acide 3-({1-[bis-(4-chloro-phényl)-méthyl]-azétidin-3-yl}-méthanesulfonyl-amino)-5-méthoxy-benzoïque
Acide 3-({1-[bis-(4-chloro-phényl)-méthyl]-azétidin-3-yl}-méthanesulfonyl-amino)-5-méthoxy-benzoïque
Ester Allylique de l'acide 3-({1-[bis-(4-chloro-phényl)-méthyl]-azétidin-3-yl}-méthanesulfonyl-amino)-5-méthyl-benzoïque
Acide 3-({1-[bis-(4-chloro-phényl)-méthyl]-azétidin-3-yl}-méthanesulfonyl-amino)-5-méthyl-benzoïque
Ester méthylique de l'acide 3-({1-[bis-(4-chloro-phényl)-méthyl]-azétidin-3-yl}-méthanesulfonyl-amino)-5-chloro-benzoïque
Acide 3-({1-[bis-(4-chloro-phényl)-méthyl]-azétidin-3-yl}-méthanesulfonyl-amino)-5-chloro-benzoïque
Ester méthylique de l'acide 3-({1-[bis-(4-chloro-phényl)-méthyl]-azétidin-3-yl}-méthanesulfonyl-amino)-5-bromo-benzoïque
Acide 3-({1-[bis-(4-chloro-phényl)-méthyl]-azétidin-3-yl}-méthanesulfonyl-amino)-5-bromo-benzoïque
Ester méthylique de l'acide 3-({1-[bis-(4-chloro-phényl)-méthyl]-azétidin-3-yl}-méthanesulfonyl-amino)-5-trifluorométhyl-benzoïque
Acide 3-({1-[bis-(4-chloro-phényl)-méthyl]-azétidin-3-yl}-méthanesulfonyl-amino)-5-trifluorométhyl-benzoïque
Ester méthylique de l'acide 3-({1-[bis-(4-chloro-phényl)-méthyl]-azétidin-3-yl}-méthanesulfonyl-amino)-5-cyano-benzoïque
Acide 3-({1-[bis-(4-chloro-phényl)-méthyl]-azétidin-3-yl}-méthanesulfonyl-amino)-5-cyano-benzoïque

Les composés de formules 4 et 5 ont été préparés à l'état de poudre ou d'huile, à l'état de base. Le tableau 1A rassemble quelques données physicochimiques de ces intermédiaires.

**Tableau 1A**

| | | | | |
|---|---|---|---|---|
| 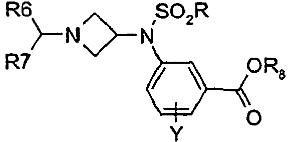 | | | | |

| R6 | R7 | Y | R" | RMN |
|---|---|---|---|---|
| 4-Cl-Ph | 4-Cl-Ph | 5-F | Me | 1H NMR (400 MHz, DMSO-d6) δ (ppm) 2.75 (m, 2 H) 3.01 (s, 3 H) 3.38 (m, 2 H) 3.89 (s, 3 H) 4.44 (s, 1H) 4.80 (m, 1 H) 7.32 (d, J=8.7 Hz, 4 H) 7.38 (d, J=8.7 Hz, 4 H) 7.59 (dt, J=9.5, 2.1 Hz, 1 H) 7.69 (ddd,J=8.5, 2.1, 1.6 Hz, 1 H) 7.74 (s large, 1 H) |
| 4-F-Ph | 4-F-Ph | 5-F | Me | 1H NMR (400 MHz, DMSO-d6) δ (ppm) 2.71 (m, 2 H) 2.98 (s, 3 H) 3.33 (m, 2 H) 3.87 (s, 3 H) 4.41 (s, 1H) 4.76 (m, 1 H) 7.07 (t, J=8.9 Hz, 4 H) 7.37 (dd, J=8.9, 5.6 Hz, 4 H) 7.56 (dt, J=9.5, 2.0 Hz, 1 H) 7.67(ddd, J=7.8, 2.0, 1.4 Hz, 1 H) 7.70 (s large, 1 H) |
| 4-F-Ph | 4-F-Ph | 5-F | H | 1H NMR (400 MHz, DMSO-d6) δ (ppm) : 3.02 (m, 3 H) 3.39 - 3.57 (m partiellement masqué, 2 H) 3.72 - 4.36 (m, 2 H) 5.00 - 5.40 (m, 1 H) 5.80 - 6.10 (m, 1 H) 6.90 - 7.80 (m, 11 H) 12.00 - 12.67 (m, 1 H) 13.56 (m, 1 H) |
| 4-Br-Ph | 4-Br-Ph | 5-F | Et | 1H NMR (400 MHz, DMSO-d6) δ (ppm) 1.32 (t, J=7.1 Hz, 3 H) 2.73 (m, 2 H) 2.98 (s, 3 H) 3.35 (m, 2 H) 4.34 (q, J=7.1 Hz, 2 H) 4.40 (s, 1 H) 4.76 (m, 1 H) 7.30 (d, J=8.7 Hz, 4 H) 7.45 (d, J=8.7 Hz, 4 H) 7.56 (dt, J=9.5, 2.3 Hz, 1 H) 7.66 (ddd, J=8.8, 2.3, 1.1 Hz, 1 H) 7.70 (s large, 1 H) |
| 4-Br-Ph | 4-Br-Ph | 5-F | H | 1H NMR (400 MHz, DMSO-d6) δ (ppm) 2.73 (m, 2 H) 2.97 (s, 3 H) 3.34 (m partiellement masqué, 2 H)4.40 (s, 1 H) 4.75 (m, 1 H) 7.30 (d, J=8.5 Hz, 4 H) 7.44 (d, J=8.5 Hz, 4 H) 7.47 (m partiellement masqué, 1H) 7.62 (d large J=9.0 Hz, 1 H) 7.68 (s large, 1 H) 12.95 (m étalé, 1 H) |
| 4-CF₃-Ph | 4-CF₃-Ph | 5-F | Et | 1H NMR (400 MHz, DMSO-d6) δ (ppm) 1.32 (t, J=7.1 Hz, 3 H) 2.81 (m, 2 H) 2.99 (s, 3 H) 3.41 (m, 2 H) 4.34 (q, J=7.1 Hz, 2 H) 4.69 (s, 1 H) 4.81 (m, 1 H) 7.57 (dt, J=9.5, 2.2 Hz, 1 H) 7.60 - 7.68 (m, 9 H) 7.71 (s large, 1 H) |
| 4-CF₃-Ph | 4-CF₃-Ph | 5-F | H | 1H NMR (400 MHz, DMSO-d6) δ (ppm) 2.81 (m, 2 H) 2.99 (s, 3 H) 3.40 (m, 2 H) 4.68 (s, 1 H) 4.81 (m, 1H) 7.51 (dt, J=9.5, 2.3 Hz, 1 H) 7.58 - 7.66 (m, 9 H) 7.71 (s large, 1 H) 13.39 (m étalé, 1H) |
| 4-OCH₃-Ph | 4-OCH₃-Ph | 5-F | Et | 1H NMR (400 MHz, DMSO-d6) δ (ppm) 1.33 (t, J=7.1 Hz, 3 H) 2.67 (m, 2 H) 2.98 (s, 3 H) 3.31 (m partiellement masqué, 2 H) 3.67 (s, 6 H) 4.22 (s, 1 H) 4.34 (q, J=7.1 Hz, 2 H) 4.73 (m, 1 H) 6.79 (d, J=8.8 Hz, 4 H) 7.23 (d, J=8.8 Hz, 4 H) 7.56 (dt, J=9.5, 2.3 Hz, 1 H) 7.66 (ddd, J=8.6, 2.3, 1.6 Hz, 1 H) 7.70 (s large, 1 H) |
| 4-OCH₃-Ph | 4-OCH₃-Ph | 5-F | H | 1H NMR (400 MHz, DMSO-d6) δ (ppm) 2.66 (m, 2 H) 2.96 (s, 3 H) 3.18 - 3.34 (m partiellement masqué,2 H) 3.67 (s, 6 H) 4.20 (s, 1 H) 4.71 (m, 1 H) 6.79 (d, J=8.8 Hz, 4 H) 7.22 (d, J=8.8 Hz, 4 H) 7.43 (d large,J=9.5 Hz, 1 H) 7.60 (d large, J=9.0 Hz, 1 H) 7.66 (s large, 1 H) 13.52 (m étalé, 1 H) |
| 4-CH₃-Ph | 4-CH₃-Ph | 5-F | Me | 1H NMR (400 MHz, DMSO-d6) δ (ppm) 2.20 (s, 6 H) 2.67 (m, 2 H) 2.97 (s, 3 H) 3.32 ( m, 2 H) 3.87 (s, 3H) 4.24 (s, 1 H) 4.74 (m, 1 H) 7.03 (d, J=8.1 Hz, 4 H) 7.20 (d, J=8.1 Hz, 4 H) 7.55 (dt, J=9.6, 2.2 Hz, 1 H) 7.67 (ddd, J=8.0, 2.2, 1.7 Hz, 1 H) 7.70 (s large, 1 H) |
| 4-CH₃-Ph | 4-CH₃-Ph | 5-F | H | 1H NMR (400 MHz, DMSO-d6) δ (ppm) 2.20 (s, 6 H) 2.68 (m, 2 H) 2.97 (s, 3 H) 3.32 (m, 2 H) 4.24 (s, 1H) 4.73 (m, 1 H) 7.03 (d, J=8.1 Hz, 4 H) 7.20 (d, J=8.1 Hz, 4 H) 7.50 (dt, J=9.5, 2.3 Hz, 1 H) 7.62 (ddd,J=8.8, 2.3, 1.5 Hz, 1 H) 7.68 (s large, 1 H) 13.52 (m étalé, 1 H) |
| 4-CN-Ph | 4-CN-Ph | 5-F | Et | 1H NMR (400 MHz, DMSO-d6) δ (ppm) 1.32 (t, J=7.1 Hz, 3 H) 2.78 (m, 2 H) 2.99 (s, 3 H) 3.38 (m, 2 H)4.34 (q, J=7.1 Hz, 2 H) 4.68 (s, 1 H) 4.80 (m, 1 H) 7.56 (m partiellement masqué, 1 H) 7.58 (d, J=8.5 Hz, 4H) 7.66 (d large, J=8.8 Hz, 1 H) 7.70 (s large, 1 H) 7.74 (d, J=8.5 Hz, 4 H) |
| 4-CN-Ph | 4-CN-Ph | 5-F | H | 1H NMR (400 MHz, DMSO-d6) δ (ppm) après addition d'une goutte d'acide trifluoroacétique deutéré CF3CO2D-d) 3.03 (s, 3 H) 4.07 (m, 2 H) 4.24 (m, 2 H) 5.15 (m, 1 H) 6.07 (s, 1 H) 7.53 (dt, J=9.3, 2.3 Hz,1 H) 7.63 (d, J=8.7 Hz, 4 H) 7.68 (ddd, J=8.7, 2.3, 1.5 Hz, 1 H) 7.78 (s large, 1 H) 7.88 (d, J=8.7 Hz, 4 H) |
| 4-OCF₃-Ph | 4-OCF₃-Ph | 5-F | Me | 1H NMR (400 MHz, DMSO-d6) δ (ppm) 2.76 (m, 2 H) 2.99 (s, 3 H) 3.36 (m, 2 H) 3.87 (s, 3 H) 4.53 (s, 1H) 4.78 (m, 1 H) 7.26 (d large, J=8.8 Hz, 4 H) 7.49 (d, J=8.8 Hz, 4 H) 7.56 (dt, J=9.5, 2.3 Hz, 1 H) 7.67(ddd, J=8.6, 2.5, 1.3 Hz, 1 H) 7.71 (s large, 1 H) |
| 4-OCF₃-Ph | 4-OCF₃-Ph | 5-F | H | 1H NMR (400 MHz, DMSO-d6) δ (ppm) 2.76 (m, 2 H) 2.98 (s, 3 H) 3.36 (m, 2 H) 4.53 (s, 1 H) 4.77 (m, 1H) 7.26 (d large, J=8.8 Hz, 4H) 7.49 (d, J=8.8 Hz, 4 H) 7.51 (m partiellement masqué, 1 H) 7.63 (ddd, J=8.7, 2.6, 1.3 Hz, 1 H) 7.70 (s large, 1 H) 13.53 (m étalé, 1 H) |
| 4-Cl-Ph | 4-Cl-Ph | 2-F | Me | 1H-NMR (300 MHz, DMSO-d6): δ(ppm) = 7.96-7.90 (m, 1H),7.78-7.72 (m, 1H), 7.42-7.37 (m, 1 H), 7.33-7.27 (m, 8H), 4.74-4.70 (m, 1H), 4.31 (s, 1H),3.86 (s, 3H), 3.37-3.32 (m, 2H), 3.09 (s, 3H), 2.67-2.63(m, 2H) |
| 4-Cl-Ph | 4-Cl-Ph | 2-F | H | 1H NMR (500 MHz, DMSO-d6) δ (ppm) pour ce lot, tous les signaux sont larges avec : 3.06 - 4.27 (m, 4H) 3.13 (s, 3 H) 4.80 - 6.18 (m, 2 H) 7.26-7.86 (m, 10 H) 7.94 (m, 1 H) 12.37 - 13.89 (m, 1H) |
| 4-Cl-Ph | 4-Cl-Ph | 4-F | Et | 1H-NMR (300 MHz, DMSO-d6): δ(ppm) = 8.07-8.02 (m, 1H),8.01-7.97 (m, 1H), 7.51 (t, J= 9.7, 1H), 7.34-7.27 (m, 8H), 4.76-4.72 (m, 1H), 4.37-4.30(m, 3H), 3.35-3.32 (m, 2H), 3.10 (s, 3H), 2.74-2.66 (m,2H), 1.33 (t, J= 7.1, 3H) |
| 4-Cl-Ph | 4-Cl-Ph | 4-F | H | 1H NMR (500 MHz, DMSO-d6) δ (ppm) avec : 2.62 - 4.36 (m, 4H) 3.12 (s, 3 H) 4.65 - 6.28 (m, 2 H) 7.12 - 7.77 (m, 9 H) 8.06 (m, 2 H) 12.23-14.18 (m, 1H) |
| 4-Cl-Ph | 4-Cl-Ph | 6-F | Me | 1H-NMR (300 MHz, DMSO-d6): δ(ppm) = 7.79-7.76 (m, 1H),7.64-7.60 (m, 1H), 7.43-7.29 (m, 9H), 4.76-4.72 (m, 1H), 4.41 (s, 1H), 3.86 (s, 3H), 3.33-3.29 (m, 2H), 2.96 (s, 3H), 2.72 (t, J= 7.4, 2H). |
| 4-Cl-Ph | 4-Cl-Ph | 6-F | H | 1H NMR (500 MHz, DMSO-d6) δ (ppm): 2.74 - 4.29 (m, 4H) 3.01 (s, 3 H) 4.75 - 6.30 (m, 2 H) 7.23 - 8.11 (m, 11 H) 11.79 - 14.10 (m, 1 H) |
| 4-Cl-Ph | 4-Cl-Ph | 5-OCH₃ | Me | 1H NMR (400 MHz, DMSO-d6) δ (ppm) 2.70 (m, 2 H) 2.95 (s, 3 H) 3.33 (m, 2 H) 3.82 (s, 3 H) 3.86 (s, 3H) 4.42 (s, 1 H) 4.76 (m, 1 H) 7.16 (t, J=2.3 Hz, 1 H) 7.31 (d, J=8.5 Hz, 4 H) 7.37 (d, J=8.5 Hz, 4 H) 7.40 (t, J=2.3 Hz, 1 H) 7.44 (t, J=2.3 Hz, 1 H) |
| 4-Cl-Ph | 4-Cl-Ph | 5-OCH₃ | H | 1H NMR (400 MHz, DMSO-d6) δ(ppm) 2.71 (m, 2 H) 2.94 (s, 3 H) 3.32 (m partiellement masqué, 2 H)3.80 (s, 3 H) 4.41 (s, 1 H) 4.75 (m, 1 H) 7.09 (s large, 1 H) 7.31 (d, J=8.6 Hz, 4 H) 7.36 (d, J=8.6 Hz, 4 H) 7.39 (dd, J=2.4, 1.5 Hz, 1 H) 7.42 (s large, 1 H) 13.20 (m étalé, 1 H) |
| 4-Cl-Ph | 4-Cl-Ph | 5-CH₃ | Allyl | 1H-NMR (300 MHz, DMSO-d6): δ(ppm) = 7.75 (s, 1H), 7.66 (s,1H), 7.44 (s, 1H), 7.37-7.29 (m, 8H), 6.09-5.97 (m, 1H), 5.38 (d, J= 17.2, 1H), 5.28 (d, J=10.5, 1H), 4.80 (d, J= 5.4, 2H), 4.79-4.73 (m, 1H), 4.41 (s, 1H), 3.33-3.29 (m, 2H, overlapped with the H2O signal), 2.95 (s, 3H), 2.69 (t, J= 7.5, 2H), 2.37 (s, 3H). |
| 4-Cl-Ph | 4-Cl-Ph | 5-CH₃ | H | 1H NMR (500 MHz, DMSO-d6) δ (ppm) avec : 2.38 (s, 3 H)2.85 - 4.15 (m, 4 H) 2.99 (s, 3 H) 4.76 - 6.30 (m, 2 H) 7.20 - 7.83 (m, 11 H) 12.30-13.50 (m, 1 H) |
| 4-Cl-Ph | 4-Cl-Ph | 5-Cl | Me | 1H-NMR (300 MHz, DMSO-d6): δ(ppm) = 7.88 (s, 1H), 7.80 (s,1H), 7.74 (s, 1H), 7.37-7.30 (m, 8H), 4.81-4.77 (m, 1H), 4.43 (s, 1H), 3.87 (s, 3H), 3.35-3.30 (m, 2H), 2.99 (s, 3H), 2.72 (t, J= 7.3, 2H). |
| 4-Cl-Ph | 4-Cl-Ph | 5-Cl | H | 1H NMR (400 MHz, DMSO-d6) δ (ppm) pour ce lot, tous les signaux sont larges avec : 2.70 - 4.20 (m, 4H) 3.03 (s, 3 H) 4.70 - 6.13 (m, 2 H) 7.30 - 7.70 (m, 8 H) 7.75 (s, 1 H) 7.98 (s, 2 H) 12.24 - 14.00 (m, 1 H) |
| 4-Cl-Ph | 4-Cl-Ph | 5-Br | Me | 1H-NMR (300 MHz, DMSO-d6): δ(ppm) = 8.00 (s, 1H), 7.86(s, 1H), 7.83 (s, 1H), 7.37-7.29 (m, 8H), 4.81-4.77 (m, 1H), 4.43 (s, 1H), 3.87 (s, 3H),3.34-3.29 (m, 2H overlapped with the H2O signal), 2.99 (s, 3H), 2.72 (t, J= 7.4,2H). |
| 4-Cl-Ph | 4-Cl-Ph | 5-Br | H | 1H NMR (400 MHz, DMSO-d6) δ (ppm) 2.72-4.30 (m, 4H) 3.03 (s, 3 H) 4.78 - 6.25 (m, 2 H) 7.29 - 7.71 (m, 8 H) 7.87 (s, 1 H) 7.90 (s, 1 H) 8.01 (s, 1 H) 11.94-14.11 (m, 1 H) |
| 4-Cl-Ph | 4-Cl-Ph | 5-CF₃ | Me | 1H-NMR (300 MHz, DMSO-d6): δ(ppm) = 8.13-8.11 (m, 2H),7.99 (s, 1H), 7.36-7.29 (m, 8H), 4.90-4.86 (m, 1H), 4.44 (s, 1H), 3.90 (s, 3H), 3.35-3.31 (m,2H overlapped with the H2O signal), 3.01 (s, 3H), 2.75 (t, J= 7.2, 2H). |
| 4-Cl-Ph | 4-Cl-Ph | 5-CF₃ | H | 1H NMR (400 MHz, DMSO-d6) δ (ppm) 2.77 (m, 2 H) 3.01 (s, 3 H) 3.30 (m masqué, 2 H) 4.44 (m, 1 H)4.87 (m, 1 H) 7.26 - 7.39 (m, 8 H) 7.96 (s, 1 H) 8.12 (s large, 2 H) 13.70 (m étalé, 1 H) |
| 4-Cl-Ph | 4-Cl-Ph | 5-CN | Me | 1H-NMR (300 MHz, DMSO-d6): δ(ppm) = 8.28 (s, 1H), 8.13 (s,1H), 8.10 (s, 1H), 7.37-7.29 (m, 8H), 4.81-4.77 (m, 1H), 4.42 (s, 1H), 3.89 (s, 3H), 3.38-3.32 (m, 2H overlapped with the H2O signal), 3.01 (s, 3H), 2.72 (t, J= 7.4, 2H). |
| 4-Cl-Ph | 4-Cl-Ph | 5-CN | H | 1H NMR (400 MHz, DMSO-d6) δ (ppm) 2.70-4.35 (m, 4H) 3.05 (s, 3 H) 4.83 - 6.34 (m, 2 H) 7.28 - 7.72 (m, 8 H) 8.11 (s, 1 H) 8.18 (s, 1 H) 8.27 (s, 1 H) 11.45 -13.35 (m, 1 H) |

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

### Exemple 1 : 3-({1-[bis-(4-chloro-phényl)-méthyl]-azétidin-3-yl}-méthanesulfonyl-amino)-N-((S)-1-carbamoyl-éthyl)-benzamide (Composé N°1)

A une solution de 500 mg d'acide 3-[{1-[bis(4-chlorophényl)méthyl]azétidin-3-yl}(méthanesulfonyl)amino]benzoïque, 150 mg de chlorhydrate de L-alaninamide dans 20 cm³ de tétrahydrofuranne, sont successivement additionnés 67 mg de 1-hydroxybenzotriazole, 0,4 cm³ de triéthylamine et 228 mg de chlorhydrate de 1-(3-diméthylaminopropyl)-3-éthylcarbodiimide. Le mélange réactionnel est laissé sous agitation pendant une nuit à une température voisine de 20°C. Après concentration à sec sous pression réduite du milieu réactionnel, la poudre blanche jaunâtre obtenue est dissoute dans du dichlorométhane. La phase organique est lavée avec de l'eau. Après décantation, la phase aqueuse est de nouveau extraite avec du dichlorométhane. Le brut réactionnel obtenu après concentration à sec sous pression réduite des phases organiques est purifié par chromatographie flash sur une cartouche de 30 g de silice Merck (gradient d'élution : dichlorométhane/méthanol 100/0 à 95/5). Après concentration des fractions sous pression réduite, on obtient une poudre blanche qui est lavée avec de l'éther éthylique et séchée à l'étuve sous vide. On obtient ainsi 430 mg de 3-({1-[bis-(4-chloro-phényl)-méthyl]-azétidin-3-yl}-méthanesulfonyl-amino)-*N-*((S)-1-carbamoyl-éthyl)-benzamide sous la forme d'un solide blanc.
Pf : 229°C
Spectre RMN 1H (300 MHz ; (δ en ppm) ; (DMSO-d6) ; référencé à 2,50 ppm): 1,34 (d, J=7,1 Hz, 3H) ; 2,71 (t, J=6,8 Hz, 2H) ; 2,97 (s, 3H) ; 3,26 - 3,38 (m partiellement masqué, 2H) ; 4,38 (s, 1H) ; 4,42 (m, 1H) ; 4,73 (quin, J=6,8 Hz, 1H) ; 6,98 (s large, 1H) ; 7,31 (d, J=8,5 Hz, 4H) ; 7,36 (d, J=8,5 Hz, 4H) ; 7,39 (s large, 1H) ; 7,44- 7,54 (m, 2H) ; 7,80 (s large, 1H) ; 7,89 (m, 1H) ; 8,50 (d, J=7,4 Hz, 1H)
Spectre de masse: ES m/z=575 MH⁺, pic de base), m/z=235 (C₁₃H₉Cl₂⁺), m/z=573 (MH)

### Analyse élémentaire:

Calculée: C: 56,35%- H: 4,90%- N: 9,73%- S: 5,57%
Mesurée: C: 56,72%- H: 4,99%- N: 9,50%- S: 5,65%
Pouvoir rotatoire : α_{D} = +10,3 +/- 0,6 (c=0,428, MeOH)

### Exemple 2 : 3-({1-[bis-(4-chloro-phényl)-méthyl]-azétidin-3-yl}-méthanesulfonyl-amino)-N-carbamoylméthyl-5-fluoro-benzamide (Composé N°2)

### 2a : Ester éthylique de l'acide 3-fluoro-5-méthanesulfbnylamino-benzoïque

A une solution de 4 g d'ester éthylique de l'acide 5-amino-3-fluoro benzoïque dans 100 cm³ de dichlorométhane agitée sous atmosphère d'argon, sont additionnés 2,65 cm³ de pyridine. Le milieu réactionnel est refroidi à une température voisine de 0°C à l'aide d'un bain de glace, puis, une solution de 1,78 cm³ de chlorure de méthane sulfonyle dans 2 cm³ de dichlorométhane est additionnée goutte à goutte. La solution orangée obtenue est laissée revenir à une température voisine de 20°C et est agitée à cette température pendant 20h. Après addition de 40 cm³ d'eau distillée et de 50 cm³ de dichlorométhane, suivie de décantation, la phase organique est successivement lavée avec 35 cm³ d'eau distillée puis 40 cm³ d'une solution aqueuse saturée en chlorure de sodium. La phase organique est séchée sur sulfate de sodium, filtrée sur verre fritté puis concentrée à sec sous pression réduite pour donner 5,8 g d'un solide orangé. Le brut réactionnel est purifié par chromatographie flash sur une cartouche de 400 g de silice Merck (granulométrie: 15-40 µm ; éluant: dichlorométhane/méthanol 98/2). Après concentration des fractions sous pression réduite, on obtient 5,09 g d'ester éthylique de l'acide 3-fluoro-5-méthanesulfonylamino-benzoïque sous la forme d'un solide blanc.
Spectre de masse: EI m/z=261 (M⁺, pic de base), m/z=233 [(M - C₂H₄)⁺], m/z=216 [(M-OC₂H₅)⁺], m/z=182 [(M-SO₂CH₃)⁺], m/z=138 [(m/z=182-OC₂H₄)⁺]

### 2b : Ester éthylique de l'acide 3-({1-[bis-(4-chloro-phényl)-méthyl]-azétidin-3-yl}-méthanesulfonyl-amino)-5-fluoro-benzoïque

A une suspension de 3,7 g de méthanesulfonate de 1-[bis-(4-chloro-phényl)-méthyl]-azétidin-3-yle, 3,5 g d'ester éthylique de l'acide 3-fluoro-5-méthanesulfonylamino-benzoïque dans 130 cm³ de 4-méthyl-2-pentanone sont additionnés 3,97 g de carbonate de potassium. Le milieu réactionnel est agité au reflux pendant 7 heures puis laissé revenir à une température voisine de 20°C pendant 16 heures. A la suspension crème obtenue sont ajoutés 50 cm³ d'eau distillée et 100 cm³ d'acétate d'éthyle. Après 30 minutes d'agitation suivie de décantation, la phase aqueuse est extraite deux fois par 100 cm³ d'acétate d'éthyle. Les phases organiques réunies sont lavées avec 80 cm³ d'une solution aqueuse saturée en chlorure de sodium, séchées sur sulfate de sodium, filtrées sur verre fritté puis concentrées à sec sous pression réduite pour donner 7,2 g d'un résidu orangé. Le brut réactionnel est purifié par chromatographie flash sur une cartouche de 400 g de silice Merck (granulométrie : 15-40 µm ; gradient d'élution : dichlorométhane/méthanol 98/2 à 95/5). Après concentration des fractions sous pression réduite, on obtient 4,03 g d'ester éthylique de l'acide 3-({1-[bis-(4-chloro-phényl)-méthyl]-azétidin-3-yl}-méthanesulfonyl-amino)-5-fluoro-benzoïque sous la forme d'une meringue blanche.
Spectre RMN 1H (400 MHz ; (δ en ppm) ; (DMSO-d6) ; référencé à 2,50 ppm): 1,32 (t, J=7,2 Hz, 3H) ; 2,73 (t, J=7,3 Hz, 2H) ; 2,98 (s, 3H) ; 3,35 (m, 2H) ; 4,34 (q, J=7,2 Hz, 2H) ; 4,43 (s, 1H) ; 4,77 (m, 1H) ; 7,31 (d, J=8,8 Hz, 4H) ; 7,37 (d, J=8,8 Hz, 4H) ; 7,56 (dt, J=9,8 ; 2,4 Hz, 1H) ; 7,66 (d large, J=9,1 Hz, 1H) ; 7,70 (s large,1H)
Spectre de masse: ES m/z=551 (MH⁺), m/z=235 (C₁₃H₉Cl₂⁺, pic de base)

### 2c : Acide 3-({1-[bis-(4-chloro-phényl)-méthyl]-azétidin-3-yl}-méthanesulfonyl-amino)-5-fluoro-benzoïque

A une solution de 2,5 g d'ester éthylique de l'acide 3-({1-[bis-(4-chloro-phényl)-méthyl]-azëtidin-3-yl}-méthanesulfonyl-amino)-5-fluoro-benzoïque dans un mélange constitué de 34 cm³ de tétrahydrofuranne et 9 cm³ d'eau agitée sous atmosphère d'argon, est additionné en deux fois, 0,222 g d'hydroxyde de lithium. Le milieu réactionnel est agité à une température voisine de 20°C pendant 24 heures. Puis, 100 cm³ d'une solution aqueuse saturée en hydrogénophosphate de sodium sont additionnés pour amener le pH à 5. La phase aqueuse est extraite quatre fois avec 200 cm³ d'acétate d'éthyle. Les phases organiques réunies sont séchées sur sulfate de sodium, filtrées sur verre fritté puis concentrées à sec sous pression réduite pour donner une meringue qui est reprise deux fois par 150 cm³ d'éther éthylique. Après concentration sous pression réduite, on obtient 2,3 g d'acide 3-({1-[bis-(4-chloro-phényl)-méthyl]-azétidin-3-yl}-méthanesulfonyl-amino)-5-fluoro-benzoïque sous la forme d'un solide blanc.
Spectre RMN 1H (400 MHz ; (δ en ppm) ; (DMSO-d6) ; référencé à 2,50 ppm): 2,74 (t, J=6,9 Hz, 2H) ; 2,98 (s, 3H) ; 3,33 (m masqué, 2H) ; 4,43 (s, 1H) ; 4,76 (quin, J=6,9 Hz, 1H) ; 7,31 (d, J=8,8 Hz, 4H) ; 7,36 (d, J=8,8 Hz, 4H) ; 7,51 (dt, J=9,4 ; 2,0 Hz, 1H) ; 7,64 (dt, J=8,9 ; 2,0 Hz, 1H) ; 7,70 (t, J=2,0 Hz, 1H) ; 13,25 (m très étalé, 1H)
Spectre de masse: ES m/z=523 (MH⁺), m/z=235 (C₁₃H₉Cl₂⁺, pic de base)

### 2d : 3-({1-[bis-(4-chloro-phényl)-méthyl]-azétidin-3-yl}-méthanesulfonyl-amino)-N-carbamoylméthyl-5-fluoro-benzamide (Composé N°2)

A une solution de 0,4 g d'acide 3-({1-[bis-(4-chloro-phényl)-méthyl]-azétidin-3-yl}-méthanesulfonyl-amino)-5-fluoro-benzoïque et 0,245 cm³ de triéthylamine dans 5 cm³ de tétrahydrofuranne est additionné 0,115 cm³ d'isobutylchloroformate. Un précipité apparaît et le milieu réactionnel est agité à une température voisine de 20°C pendant 30 minutes avant d'additionner en une seule fois 0,101 g de glycinamide avec 1 cm³ de tétrahydrofuranne. La suspension blanche obtenue est agitée à une température voisine de 20°C pendant 20 heures. Le milieu réactionnel est filtré sur verre fritté en rinçant avec 50 cm³ de tétrahydrofuranne. Le filtrat est concentré à sec sous vide pour donner 0,55 g d'une meringue jaune qui est purifiée par chromatographie flash sur une cartouche de 70 g de silice Merck (granulométrie : 15-40 µm ; éluant : dichlorométhane/méthanol 98/2). Après concentration des fractions sous pression réduite, on obtient une meringue qui est reprise à l'éther diéthylique, filtrée puis séchée sous vide à une température voisine de 50°C. Le solide ainsi obtenu est repris avec 20 cm³ de pentane et laissé dans le pentane pendant une nuit. Après filtration, le produit est séché sous vide. On obtient alors 0,222 g de 3-({1-[bis-(4-chloro-phényl)-méthyl]-azétidin-3-yl}-méthanesulfonyl-amino)-*N*-carbamoylméthyl-5-fluoro-benzamide sous la forme d'un solide jaunâtre.
Pf : 154-156°C
Spectre RMN 1H (400 MHz ; (δ en ppm) ; (DMSO-d6) ; référencé à 2,50 ppm): 2,73 (t, J=7,0 Hz, 2H) ; 3,00 (s, 3H) ; 3,36 (m, 2H) ; 3,81 (d, J=5,9 Hz, 2H) ; 4,40 (s, 1H) ; 4,72 (m, 1H) ; 7,04 (s large, 1H) ; 7,31 (d, J=8,8 Hz, 4H) ; 7,35 (d, J=8,8 Hz, 4H) ; 7,37-7,47 (m, 2H) ; 7,60 - 7,73 (m, 2H) ; 8,84 (t, J=5,9 Hz, 1H)
Spectre de masse: ES m/z=579 (MH⁺, pic de base), m/z=235 (C₁₃H₉Cl₂^{+.})

### Analyse élémentaire:

Calculée: C: 53,89%- H: 4,35%- N: 9,67%- S: 5,53%
Mesurée: C: 53,88%-H: 4,71%- N: 9,16%-S: 5,66%

Exemple 3 : 3-({1-[bis-(4-chloro-phényl)-méthyl]-azétidin-3-yl}-méthanesulfonyl-amino)-*N-*((S)-1-carbamoyl-éthyl)-5-fluoro-benzamide (Composé N°3).

A une solution de 0,35 g d'acide 3-({1-[bis-(4-chloro-phényl)-méthyl]-azétidin-3-yl}-méthanesulfonyl-amino)-5-fluoro-benzoïque et 0,214 cm³ de triéthylamine dans 5 cm³ de tétrahydrofuranne est additionné 0,1 cm³ d'isobutylchloroformate. Le milieu réactionnel est agité à une température voisine de 20°C pendant 1 heure 10 minutes avant d'additionner en une seule fois 0,113 g de chlorhydrate de L-alaninamide avec 2 cm³ de tétrahydrofuranne. La suspension blanche obtenue est agitée à une température voisine de 20°C pendant 48 heures. Le milieu réactionnel est filtré sur verre fritté en rinçant avec du tétrahydrofuranne. Le filtrat est concentré à sec sous vide pour donner 0,5 g d'une meringue blanche qui est purifiée par chromatographie flash sur une cartouche de 70 g de silice Merck (granulométrie : 15-40 µm ; éluant : dichlorométhane/méthanol 97/3). Après concentration des fractions sous pression réduite, on obtient une meringue qui est reprise à l'éther diéthylique, filtrée puis séchée sous vide à une température voisine de 50°C pour donner 0,307 g d'un solide blanc. Celui-ci est recristallisé à chaud dans un mélange éthanol/eau et séché sous vide à une température voisine de 45°C. On obtient ainsi 0,213 g de 3-({1-[bis-(4-chloro-phényl)-méthyl]-azétidin-3-yl}-méthanesulfonyl-amino)-*N-*((S)-1-carbamoyl-éthyl)-5-fluoro-benzamide sous forme d'un solide blanc.
Pf : 228-230°C
Spectre RMN 1H (400 MHz ; (δ en ppm) ; (DMSO-d6) ; référencé à 2,50 ppm): 1,33 (d, J=7,3 Hz, 3H) ; 2,73 (t, J=6,8 Hz, 2H) ; 3,00 (s, 3H) ; 3,24 - 3,43 (m partiellement masqué, 2H) ; 4,34 - 4,48 (m, 2H) ; 4,72 (quin, J=6,8 Hz, 1H) ; 6,99 (s large, 1H) ; 7,31 (d, J=8,5 Hz, 4H) ; 7,37 (d, J=8,5 Hz, 4H) ; 7,39 - 7,45 (m, 2H) ; 7,67 (s large, 1H) ; 7,74 (d large, J=8,8 Hz, 1H) ; 8,61 (d, J=7,3 Hz, 1H)
Spectre de masse: ES m/z=593 (MH⁺, pic de base), m/z=637 (MH⁻ + HCO₂H, m/z=591
(MH⁻, pic de base)

### Analyse élémentaire:

Calculée: C: 54,64%- H: 4,59%- N: 9,44%- S: 5,40%
Mesurée: C: 54,09%- H: 4,66%- N: 9,34%- S: 5,43% - H₂O : 1,71%
Pouvoir rotatoire : α_{D} = +11,3 +/- 0,7 (c=0,365, MeOH)

### Exemple 4 : 3-({1-[bis-(4-chloro-phényl)-méthyl]-azétidin-3-yl}-méthanesulfonyl-amino)-N-((R)-1-carbamoyl-éthyl)-5-fluoro-benzamide (Composé N°4)

A une solution de 0,35 g d'acide 3-({1-[bis-(4-chloro-phényl)-méthyl]-azétidin-3-yl}-méthanesulfonyl-amino)-5-fluoro-benzoïque et 0,216 cm³ de triéthylamine dans 7 cm³ de tétrahydrofuranne est additionné 0,1 cm³ d'isobutylchloroformate. Un précipité apparaît. Le milieu réactionnel est agité à une température voisine de 20°C pendant 1 heure 25 minutes avant d'additionner en une seule fois 0,108 g de chlorhydrate de D-alaninamide avec 2 cm³ de tétrahydrofuranne. La suspension blanche obtenue est agitée à une température voisine de 20°C pendant 24 heures. Le milieu réactionnel est filtré sur verre fritté en rinçant avec du tétrahydrofuranne. Le filtrat est concentré à sec sous vide pour donner 0,5 g d'une meringue blanche qui est purifiée par chromatographie flash sur une cartouche de 70 g de silice Merck (granulométrie : 15-40 µm ; éluant: dichlorométhane/méthanol 97/3). Après concentration des fractions sous pression réduite, on obtient une meringue qui est reprise à l'éther diéthylique, filtrée puis séchée sous vide à une température voisine de 50°C pour donner un solide. Celui-ci est recristallisé à chaud dans un mélange éthanol/eau et séché sous vide à une température voisine de 45°C. On obtient ainsi 0,095 g de 3-({1-[bis-(4-chloro-phényl)-méthyl]-azétidin-3-yl}-méthanesulfonyl-amino)-*N*-((R)-1-carbamoyl-éthyl)-5-fluoro-benzamide sous forme d'un solide blanc.
Pf: 219-221°C
Spectre RMN 1H (400 MHz ; (δ en ppm) ; (DMSO-d6) ; référencé à 2,50 ppm): 1,33 (d, J=6,8 Hz, 3H) ; 2,73 (t, J=7,1 Hz, 2H) ; 3,00 (s, 3H) ; 3,35 (m, 2H) ; 4,35 -4,45 (m, 2H) ; 4,73 (quin, J=6,8 Hz, 1H) ; 6,99 (s large, 1H) ; 7,31 (d, J=8,8 Hz, 4H) ; 7,37 (d, J=8,8 Hz, 4H) ; 7,39 - 7,45 (m, 2H) ; 7,68 (t, J=1,7 Hz, 1H) ; 7,74 (dt, J=9,0 ; 1,7 Hz, 1H) ; 8,62 (d, J=7,8 Hz, 1H)
Spectre de masse: ES m/z=593 (MH⁺, pic de base), m/z=235 (C₁₃H₉Cl₂^{+.})

### Analyse élémentaire:

Calculée: C: 54,64%- H: 4,59%- N: 9,44%- S: 5,40%
Mesurée: C: 54,44%- H: 4,54%- N: 9,48%- S: 5,36% - H₂O < 0,1%
Pouvoir rotatoire : α_{D} = - 23,3 +/- 0,7 (c=0,476, DMSO)

### Exemple 5 : 3-({1-[bis-(4-chloro-phényl)-méthyl]-azétidin-3-yl}-méthanesulfonyl-amino)-N-((R)-1-carbamoyl-éthyl)-benzamide (Composé N°5)

A une solution de 500 mg d'acide 3-({1-[bis-(4-chloro-phényl)-méthyl]-azétidin-3-yl}-méthanesulfonyl-amino-benzoïque, 150 mg de chlorhydrate de D-alaninamide dans 20 cm³ de tétrahydrofuranne, sont successivement additionnés 67 mg de 1-hydroxybenzotriazole, 0,4 cm³ de triéthylamine et 228 mg de chlorhydrate de 1-(3-diméthylaminopropyl)-3-éthylcarbodiimide. Le mélange réactionnel est laissé sous agitation pendant une nuit à une température voisine de 20°C. Après concentration à sec sous pression réduite du milieu réactionnel, la poudre blanche jaunâtre obtenue est dissoute avec du dichlorométhane. La phase organique est lavée avec de l'eau. Après décantation, la phase aqueuse est de nouveau extraite avec du dichlorométhane. Le brut réactionnel obtenu après concentration à sec sous pression réduite des phases organiques est purifié par chromatographie flash sur une cartouche de 30 g de silice Merck (gradient d'élution : dichlorométhane/méthanol 100/0 à 95/5). Après concentration des fractions sous pression réduite, on obtient un produit qui est lavé avec de l'éther diéthylique et séché à l'étuve sous vide. On obtient ainsi 220 mg de 3-({1-[bis-(4-chloro-phényl)-méthyl]-azétidin-3-yl}-méthanesulfonyl-amino)-*N* ((R)-1-carbamoyl-éthyl)-benzamide sous la forme de cristaux blancs.
Pf : 235°C
Spectre RMN 1H (300 MHz ; (δ en ppm) ; (DMSO-d6) ; référencé à 2,50 ppm): 1,34 (d, J=7,3 Hz, 3H) ; 2,71 (t, J=6,8 Hz, 2H) ; 2,97 (s, 3H) ; 3,32 (m masqué, 2H) ; 4,38 (s, 1H) ; 4,42 (m, 1H) ; 4,74 (quin, J=6,8 Hz, 1H) ; 6,98 (s large, 1H) ; 7,31 (d, J=8,8 Hz, 4H) ; 7,36 (d, J=8,8 Hz, 4H) ; 7,39 (s large, 1H) ; 7,45 - 7,54 (m, 2H) ; 7,80 (s large, 1H) ; 7,88 (m, 1H) ; 8,50 (d, J=7,6 Hz, 1H)
Spectre de masse: ES m/z= 575 (MH⁺, pic de base), m/z=235 (C₁₃H₉Cl₂^{+.})

### Analyse élémentaire:

Calculée: C: 56,35%- H: 4,90%- N: 9,73%- S: 5,57%
Mesurée: C: 56,84%- H: 5,17%- N: 9,56%- S: 5,51%
Pouvoir rotatoire : α_{D} = - 6,6 +/- 0,5 (c=0,493, DMSO)

### Exemple 6: 3-({1-[bis-(4-chloro-phényl)-méthyl]-azétidin-3-yl} méthanesulfonyl-amino)-N-((S)-1-carbamoyl-2-hydroxy-éthyl)-benzamide (Composé N°6)

A une solution de 0,4 g d'acide 3-({1-[bis-(4-chloro-phényl)-méthyl]-azétidin-3-yl}-méthanesulfonyl-amino)-benzoïque et 0,253 cm³ de triéthylamine dans 5 cm³ de tétrahydrofuranne agitée à une température comprise entre -10 et -15°C, est additionné 0,114 cm³ d'isobutylchloroformate. Un précipité apparaît. Le milieu réactionnel est agité à une température inférieure à 0°C pendant 1 heure 15 minutes avant d'additionner 0,166 g de chlorhydrate de L-serinamide avec 3 cm³ de tétrahydrofuranne tout en maintenant la température du milieu réactionnel entre -10 et -15°C. La suspension blanche obtenue est agitée à une température voisine de 20°C pendant une nuit. Le milieu réactionnel est de nouveau refroidi et maintenu à une température comprise entre -10°C et -15°C avant d'additionner : 22 µl de triéthylamine, 21 µl d'isobutylchloroformate et 22,2 mg de chlorhydrate de L-serinamide dans 2 cm³ de tétrahydrofuranne. Après 3 heures d'agitation à une température voisine de 20°C, le milieu réactionnel est filtré sur verre fritté et rincé avec du dichlorométhane. Le filtrat est concentré à sec sous pression réduite pour donner 0,726 g d'une meringue blanche qui est purifiée par chromatographie flash sur une cartouche de 70 g de silice Merck (granulométrie : 15-40 µm ; gradient d'élution : acétate d'éthyle/méthanol 99/1 à 98/2). Après concentration des fractions sous pression réduite, on obtient un résidu blanc qui est repris dans 35 cm³ d'éther diéthylique, filtré puis séché sous vide à une température voisine de 40°C. Cette opération est répétée une deuxième fois avec 6 cm³ d'éther diéthylique. On obtient ainsi 0,21 g de 3-({1-[bis-(4-chloro-phényl)-méthyl]-azétidin-3-yl}-méthanesulfonyl-amino)-N-((S)-1-carbamoyl-2-hydroxy-éthyl)-benzamide sous forme d'un solide blanc.
Pf : 245-247°C
Spectre RMN 1H (400 MHz ; (S en ppm) ; (DMSO-d6) ; référencé à 2,50 ppm): 2,71 (t, J=6,9 Hz, 2H) ; 2,97 (s, 3H) ; 3,33 (m masqué, 2H) ; 3,71 (m, 2H) ; 4,38 (s, 1H) ; 4,44 (m, 1H) ; 4,75 (quin, J=6,9 Hz, 1H) ; 4,93 (t large, J=5,9 Hz, 1H) ; 7,10 (s large, 1H); 7,31 (d, J=8,8 Hz, 4H) ; 7,36 (d, J=8,8 Hz, 4H) ; 7,41 (s large, 1H) ; 7,45 - 7,55 (m, 2H) ; 7,81 (s large, 1H) ; 7,88 (m, 1H) ; 8,30 (d, J=7,8 Hz, 1H)
Spectre de masse: ES m/z=591 (MH⁺, pic de base), m/z=235 (C₁₃H₉Cl₂^{+.})

### Analyse élémentaire:

Calculée: C: 54,83%- H: 4,77%- N: 9,47%- S: 5,42%
Mesurée: C: 53,60%- H: 5,04%- N: 9,00%- S: 4,90% - H₂O = 1,45%
Pouvoir rotatoire : α_{D} = +30,9 +/- 0,8 (c=0,412, DMSO)

### Exemple 7 : 3-({1-[bis-(4-chloro-phényl)-méthyl]-azétidin-3-yl}-méthanesulfonyl-amino)-N-(2-carbamoyl-éthyl)-benzamide (Composé N°7)

A une solution de 0,5 g d'acide 3-({1-[bis-(4-chloro-phényl)-méthyl]-azétidin-3-yl}-méthanesulfonyl-amino)-benzoïque et 0,317 cm³ de triéthylamine dans 6 cm³ de tétrahydrofuranne agitée à une température comprise entre -10 et -20°C, est additionné 0,142 cm³ d'isobutylchloroformate. Un précipité apparaît Le milieu réactionnel est agité à une température inférieure à 0°C pendant 2 heures avant d'additionner 0,184 g de chlorhydrate de β-alaninamide avec 2 cm³ de tétrahydrofuranne tout en maintenant la température du milieu réactionnel entre -10 et -20°C. La suspension blanche obtenue est agitée à une température voisine de 20°C pendant 3 heures. Le milieu réactionnel est de nouveau refroidi et maintenu à une température comprise entre -10°C et -20°C avant d'additionner : 27,5 µl de triéthylamine, 25,8 µl d'isobutylchloroformate et 24,6 mg de chlorhydrate de β-alaninamide dans 2 cm³ de tétrahydrofuranne. Après 15 heures d'agitation à une température voisine de 20°C, le milieu réactionnel est filtré sur verre fritté et rincé avec du dichlorométhane. Le filtrat est concentré à sec sous pression réduite pour donner 1 g d'une meringue blanche qui est purifiée par chromatographie flash sur une cartouche de 70 g de silice Merck (granulométrie : 15-40 µm ; éluant : acétate d'éthyle/méthanol 98/2). Après concentration des fractions sous pression réduite, on obtient un résidu blanc qui est repris dans 35 cm³ d'éther diéthylique, filtré puis séché sous vide à une température voisine de 40°C. On obtient ainsi 0,429 g de 3-({1-[bis-(4-chloro-phényl)-méthyl]-azétidin-3-yl}-méthanesulfonyl-amino)-*N-*(2-carbamoyl-éthyl)-benzamide sous forme d'un solide blanc.
Pf : 190-192°C
Spectre RMN 1H (400 MHz ; (δ en ppm) ; (DMSO-d6) ; référencé à 2,50 ppm): 2,35 (t, J=7,1 Hz, 2H) ; 2,69 (t, J=6,9 Hz, 2H) ; 2,95 (s, 3H) ; 3,34 (t, J=6,9 Hz, 2H) ; 3,44 (m, 2H) ; 4,37 (s, 1H) ; 4,73 (quin, J=6,9 Hz, 1H) ; 6,81 (s large, 1H) ; 7,31 (d, J=8,8 Hz, 4H) ; 7,35 (s masqué, 1H) ; 7,36 (d, J=8,8 Hz, 4H) ; 7,41 - 7,52 (m, 2H) ; 7,74 (s large, 1H) ; 7,80 (m, 1H) ; 8,58 (t, J=5,6 Hz, 1H)
Spectre de masse: ES m/z=575 (MH⁺, pic de base), m/z=235 (C₁₃H₉Cl₂^{+.})

### Analyse élémentaire:

Calculée: C: 56,35%-H: 4,90%- N: 9,73%- S: 5,57%
Mesurée: C: 56,36%- H: 5,10%- N: 9,46%- S: 5,29% - H2O = 0,34%

### Exemple 8 : 3-({1-[bis-(4-chloro-phényl)-méthyl]-azétidin-3-yl}-méthanesulfonyl-amino)-N-{2-morpholin-4-yl-2-oxo-éthyl)-benzamide (Composé N°8)

A une solution de 500 mg d'acide 3-({1-[bis-(4-chloro-phényl)-méthyl]-azétidin-3-yl}-méthanesulfonyl-amino]-benzoïque, 215 mg de chlorhydrate de 2-amino-1-morpholin-4-yl-éthanone dans 15 cm³ de tétrahydrofuranne, sont successivement additionnés 67 mg de 1-hydroxybenzotriazole, 0,14 cm² de triéthylamine et 270 mg de chlorhydrate de 1-(3-diméthylaminopropyl)-3-éthylcarbodiimide. Le mélange réactionnel est laissé sous agitation pendant une nuit à une température voisine de 20°C. Après concentration à sec sous pression réduite du milieu réactionnel, le résidu obtenu est repris avec du dichlorométhane. La phase organique est lavée avec de l'eau, séchée et concentrée à sec sous pression réduite pour donner un brut réactionnel qui est purifié par chromatographie flash sur une cartouche de 30 g de silice Merck (gradient d'élution : dichlorométhane/méthanol 100/0 à 95/5). Après concentration des fractions sous pression réduite, on obtient une mousse blanche qui cristallise après trituration avec de l'éther diéthylique. Après filtration et séchage à la pompe à palettes, on obtient ainsi 470 mg de 3-({1-[bis-(4-chloro-phényl)-méthyl]-azétidin-3-yl}-méthanesulfonyl-amino)-*N*-(2-morpholin-4-yl-2-oxo-éthyl)-benzamide sous la forme de cristaux blancs.
Pf : 176°C
Spectre RMN 1H (400 MHz ; (δ en ppm) ; (DMSO-d6) ; référencé à 2,50 ppm): 2,71 (t, J=6,9 Hz, 2H) ; 2,97 (s, 3H) ; 3,34 (t, J=6,9 Hz, 2H) ; 3,48 (m, 4H) ; 3,58 (m, 4H) ; 4,13 (d, J=5,5 Hz, 2H) ; 4,37 (s, 1H) ; 4,74 (quin, J=6,9 Hz, 1H) ; 7,31 (d, J=8,8 Hz, 4H) ; 7,35 (d, J=8,8 Hz, 4H) ; 7,47-7,55 (m, 2H) ; 7,78 (s large, 1H) ; 7,85 (m, 1H) ; 8,66 (t, J=5,5 Hz, 1H)
Spectre de masse: ES m/z= 631 (MH⁺, pic de base), m/z=235 (C₁₃H₉Cl₂⁺)

### Analyse élémentaire:

Calculée: C: 57,05%-H: 5,11%-N: 8,87%-S: 5,08%
Mesurée: C: 57,29%- H: 4,96%- N: 8,29%- S: 4,93%

### Exemple 9: 3-({1-[bis-(4-chloro-phényl)-méthyl]-azétidin-3-yl}-méthanesulfonyl-amino)-N-((S)-1-méthylcarbamoyl-éthyl)-benzamide (Composé N°9)

### VOIE A

A une solution de 0,4 g d'acide 3-({1-[bis-(4-chloro-phényl)-méthyl]-azétidin-3-yl}-méthanesulfonyl-amino)-benzoïque et 0,256 cm³ de triéthylamine dans 4 cm³ de tétrahydrofuranne agitée sous atmosphère inerte à une température comprise entre -10 et -20°C, est additionné 0,113 cm³ d'isobutylchloroformate. Un précipité apparaît. Le milieu réactionnel est agité à une température inférieure à 0°C pendant 1 heure avant d'additionner 0,143 g de chlorhydrate de L-alanine méthylamide avec 2 cm³ de tétrahydrofuranne tout en maintenant la température du milieu réactionnel entre -10 et -20°C. La suspension blanche obtenue est agitée à une température voisine de 20°C pendant 18 heures. Le milieu réactionnel est de nouveau refroidi et maintenu à une température comprise entre -10°C et -20°C avant d'additionner : 22 µl de triéthylamine, 20,6 µl d'isobutylchloroformate et 22 mg de chlorhydrate de L-alanine méthylamide dans 2 cm³ de tétrahydrofuranne. Après 24 heures d'agitation à une température voisine de 20°C, le milieu réactionnel est filtré sur verre fritté et rincé avec du dichlorométhane. Le filtrat est concentré à sec sous pression réduite pour donner 0,684 g d'une meringue blanche qui est purifiée par chromatographie flash sur une cartouche de 70 g de silice Merck (granulométrie : 15-40 µm ; éluant : acétate d'éthyle/méthanol 98/2). Après concentration des fractions sous pression réduite, on obtient un résidu blanc qui est recristallisé dans un mélange éthanol absolu/eau. Le solide obtenu est filtré sur verre fritté et séché sous vide à une température voisine de 40°C. On obtient ainsi 0,128 g de 3-({1-[bis-(4-chloro-phényl)-méthyl]-azétidin-3-yl}-méthanesulfonyl-amino)-*N-*((S)-1-méthylcarbamoyl-éthyl)-benzamide sous forme d'un solide blanc.
Pf: 171-173°C
Spectre RMN 1H (400 MHz ; (δ en ppm) ; (DMSO-d6) ; référencé à 2,50 ppm): 1,32 (d, J=7,0 Hz, 3H) ; 2,59 (d, J=4,9 Hz, 3H) ; 2,71 (t, J=7,6 Hz, 2H) ; 2,97 (s, 3H) ; 3,33 (t, J=7,6 Hz partiellement masqué, 2H) ; 4,38 (s, 1H) ; 4,44 (m, 1H) ; 4,74 (m, 1H) ; 7,31 (d, J=8,5 Hz, 4H) ; 7,35 (d, J=8,5 Hz, 4H) ; 7,44 - 7,52 (m, 2H) ; 7,80 (s, 1H) ; 7,83 - 7,91 (m, 2H) ; 8,59 (d, J=7,8 Hz, 1H)
Spectre de masse : ES m/z=589 (MH⁺, pic de base)

### Analyse. élémentaire:

Calculée: C: 57,05%- H: 5,13%- N: 9,50%- S: 5,44%
Mesurée: C: 55,49%- H: 5,50%- N: 9,14%- S: 5,10% - H₂O = 2,57%
Pouvoir rotatoire : α_{D} = + 25,4 +/- 0,7 (c=0,427, DMSO)

### VOIE B

### Voie B - a : Ester tert-butylique de l'acide (S)-2-[3-({1-[bis-(4-chloro-phényl)-méthyl]-azétidin-3-yl}-méthanesulfonyl-amino)-benzoylamino]-propionique

A une solution de 0,5 g d'acide 3-({1-[bis-(4-chloro-phényl)-méthyl]-azétidin-3-yl}-méthanesulfonyl-amino)-benzoïque dans 9 cm³ de tétrahydrofuranne anhydre agitée sous atmosphère inerte à une température voisine de -50°C, est additionné 0,345 cm³ de triéthylamine. Le milieu réactionnel est agité pendant 5 minutes avant d'ajouter, à la même température, 0,140 cm³ d'isobutylchloroformate. Après 1 heure d'agitation à une température voisine de -50°C, on additionne 0,270 g de chlorhydrate de l'ester tert-butylique de l'acide (S)-2-amino-propionique avec 6 cm³ de tétrahydrofuranne. Le milieu réactionnel est agité à une température voisine de - 50°C pendant 10 minutes puis est laissé revenir à une température voisine de 20°C. Après 3 heures d'agitation, le milieu est hydrolysé à une température comprise entre -20°C et -30°C avec 10 cm³ d'eau, puis agité 15 minutes à une température voisine de 20°C. La phase aqueuse est extraite trois fois avec 25 cm³ d'acétate d'éthyle. Les phases organiques réunies sont séchées sur sulfate de magnésium, filtrées sur verre fritté et concentrées à sec sous pression réduite pour donner 0,815 g d'un produit brut qui est purifié par chromatographie flash sur une cartouche de 70 g de silice Merck (granulométrie : 15-40 µm ; éluant : acétate d'éthyle/méthanol 99/1). Après concentration des fractions sous pression réduite, on obtient 0,418 g d'une meringue blanche qui est reprise dans du pentane. L'insoluble est filtré sur verre fritté puis séché sous vide à une température de 40°C. On obtient alors 0,364 g d'ester tert-butylique de l'acide (S)-2-[3-({1-[bis-(4-chloro- phényl)-méthyl]-azétidin-3-yl}-méthanesulfonyl-amino)-benzoylamino]-propionique sous forme d'un solide blanc.
Spectre RMN 1H (400 MHz ; (δ en ppm) ; (DMSO-d6) ; référencé à 2,50 ppm): 1,38 (m masqué, 3H) ; 1,39 (s, 9H) ; 2,71 (m, 2H) ; 2,97 (s, 3H) ; 3,34 (m partiellement masqué, 2H) ; 4,35 (m, 1H) ; 4,37 (s, 1H) ; 4,74 (m, 1H) ; 7,30 (d, J=8,5 Hz, 4H) ; 7,34 (d, J=8,5 Hz, 4H) ; 7,43 - 7,55 (m, 2H) ; 7,79 (s large, 1H) ; 7,86 (m, 1H) ; 8,72 (d, J=7,3 Hz, 1H) Spectre de masse : ES m/z=632 (MH⁺, pic de base)
Pouvoir rotatoire : α_{D} = + 3,6 +/- 0,5 (c=0,448, DMSO)

### Voie B - b _{:} Acide (S)-2-[3-({1-[bis-(4-chloro-phényl)-méthyl]-azétidin-3-yl}-méthanesulfonyl-amino)-benzoylamino]-propionique

A une solution de 0,5 g d'ester tert-butylique de l'acide (S)-2-[3-({1-[bis-(4-chlorophényl)-méthyl]-azétidin-3-yl}-méthanesulfonyl-amino)-benzoylamino]-propionique dans 11 cm³ de dichlorométhane agitée à une température voisine de 20°C sous atmosphère inerte sont additionnés goutte à goutte 2,72 cm³ d'acide trifluoroacétique. Le milieu réactionnel est agité à une température voisine de 20°C pendant 19 heures avant d'être concentré à sec sous pression réduite. Afin d'éliminer l'excès d'acide trifluoroacétique, on effectue deux entraînements avec 15 cm³ de toluène. Le résidu obtenu est dissous dans 10 cm³ d'eau. La phase aqueuse est alcalinisée à pH = 7 avec 3 gouttes d'une solution aqueuse de soude 1M puis extraite trois fois avec 20 cm³ de dichlorométhane. Les phases organiques réunies sont séchées sur sulfate de magnésium, filtrées sur verre fritté et concentrées à sec sous pression réduite pour donner 0,43 g d'une meringue jaunâtre qui est purifiée par chromatographie flash sur une cartouche de 25 g de silice Merck (granulométrie : 15-40 µm ; éluant : acétate d'éthyle 100). Après concentration des fractions sous pression réduite, on obtient 0,207 g d'un produit qui est trituré dans un minimum de pentane. Après concentration à sec sous pression réduite et séchage, on obtient 0,169 g d'acide (S)-2-[3-({1-[bis-(4-chloro-phényl)-méthyl]-azétidin-3-yl}-méthanesulfonyl-amino)-benzoylamino]-propionique sous forme d'une poudre blanche.
Spectre RMN 1H (400 MHz ; (δ en ppm) ; (DMSO-d6) ; référencé à 2,50 ppm): 1,39 (d, J=7,3 Hz, 3H) ; 2,71 (m, 2H) ; 2,97 (s, 3H) ; 3,23 - 3,45 (m masqué, 2H) ; 4,38 (s, 1H) ; 4,39 (m, 1H) ; 4,74 (m, 1H) ; 7,30 (d, J=8,3 Hz, 4H) ; 7,35 (d, J=8,3 Hz, 4H) ; 7,44 - 7,56 (m, 2H) ; 7,80 (s large, 1H) ; 7,86 (m, 1H) ; 8,70 (d, J=7,3 Hz, 1H)
Spectre de masse : ES m/z=576 (MH⁺, pic de base)

### Analyse élémentaire:

Calculée: C: 56,25%- H: 4,72%- N: 7,29%- S: 5,56%
Mesurée: C: 55,68%- H: 4,99%- N: 6,89%- S: 5,01% - H₂O = 0,82%
Pouvoir rotatoire : α_{D} = + 10 (c=0,195, DMSO)

### Voie B - c : 3-({1-[bis-(4-chloro-phényl)-méthyl]-azétidin-3-yl}-méthanesulfonyl-amino)-N-((S)-1-méthylcarbamoyl-éthyl)-benzamide (Composé N°9)

A une solution de 0,5 g d'acide (S)-2-[3-({1-[bis-(4-chloro-phényl)-méthyl]-azétidin-3-yl}-méthanesulfonyl-amino)-benzoylamino]-propionique dans 15 cm³ de tétrahydrofuranne agitée à une température voisine de 20°C sous atmosphère inerte sont successivement additionnés 60 mg de 1-hydroxybenzotriazole, 200 mg de chlorhydrate de 1-(3-diméthylaminopropyl)-3-éthylcarbodümide, 0,700 cm³ d'une solution de méthylamine dans le tétrahydrofuranne 2M, 0,370 cm³ de triéthylamine et 8 cm³ de tétrahydrofuranne. Le milieu réactionnel est agité à une température voisine de 20°C pendant 18 heures. Après concentration à sec sous pression réduite, le résidu est dissous dans un mélange constitué de dichlorométhane/eau 30/15 cm³. Après décantation, la phase aqueuse est extraite deux fois avec 15 cm³ de dichlorométhane. Les phases organiques sont réunies, lavées avec 15 cm³ d'une solution aqueuse saturée en chlorure de sodium, séchées sur sulfate de magnésium, filtrées puis concentrées à sec pour donner 0,415 g d'un brut qui est purifié par chromatographie flash sur une cartouche de 25 g de silice Merck (granulométrie : 15-40 µm ; éluant : acétate d'éthyle 100). Après concentration des fractions sous pression réduite, on obtient 0,293 g d'un produit qui est trituré dans un minimum de pentane. Après filtration, concentration à sec sous pression réduite et séchage, on obtient 0,247 g d'un solide crème. 147 mg de ce solide crème sont injectés sur une colonne de 6 cm de diamètre contenant 700 g de phase stationnaire chirale Chiralpak IA 20 µM. L'élution est effectuée à 80 cm³ par minute avec une phase mobile composée de 95% d'acétonitrile et 5% de dichlorométhane. L'énantiomère dextrogyre est élué en deuxième position. Après concentration de la phase mobile, on obtient 105 mg de 3-({1-[bis-(4-chloro-phényl)-méthyl]-azétidin-3-yl}-méthanesulfonyl-amino)-*N*-((S)-1-méthylcarbamoyl-éthyl)-benzamide sous la forme d'une poudre blanche amorphe.

Le tableau 1 qui suit illustre les structures chimiques (I) et les propriétés physiques (tableau 1A) de quelques exemples de composés selon l'invention. Dans ce tableau :
- Sel Na correspond au sel de sodium ; « Me » correspond à un groupe méthyle ; « Et correspond à un groupe éthyle » ; « iPr » correspond à un groupe isopropyle ; « iBu » correspond à un groupe isobutyle ; « tBu » correspond à un groupe tertio-butyle ; « Ph » représente un groupe phényle ;
- R représente un groupe méthyle;

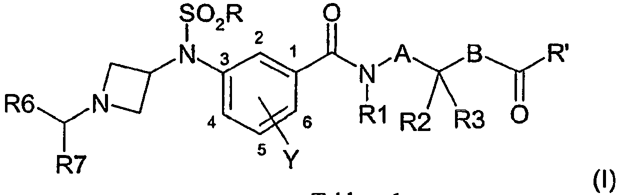

**Tableau 1**

| N° | R1 | A | B | R2, R3 | R' | R6 | R7 | Y | Sel/Chiralité |
|---|---|---|---|---|---|---|---|---|---|
| 1 | H | - | - | Me, H | NH₂ | 4-Cl-Ph | 4-Cl-Ph | H | Chiral (S) |
| 2 | H | - | - | H, H | NH₂ | 4-Cl-Ph | 4-Cl-Ph | 5-F | - |
| 3 | H | - | - | Me, H | NH₂ | 4-Cl-Ph | 4-Cl-Ph | 5-F | Chiral (S) |
| 4 | H | - | - | Me, H | NH₂ | 4-Cl-Ph | 4-Cl-Ph | 5-F | Chiral (R) |
| 5 | H | - | - | Me, H | NH₂ | 4-Cl-Ph | 4-Cl-Ph | H | Chiral (R) |
| 6 | H | - | - | CH₂OH, H | NH₂ | 4-Cl-Ph | 4-Cl-Ph | H | Chiral (S) |
| 7 | H | CH₂ | - | H, H | NH₂ | 4-Cl-Ph | 4-Cl-Ph | H | - |
| 8 | H | - | - | H, H | 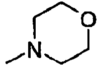 | 4-Cl-Ph | 4-Cl-Ph | H | - |
| 9 | H | - | - | H, Me | NHMe | 4-Cl-Ph | 4-Cl-Ph | H | Chiral (S) |
| 10 | H | - | - | H, Et | NH₂ | 4-Cl-Ph | 4-Cl-Ph | H | Chiral (S) |
| 11 | H | - | - | H, iBu | NH₂ | 4-Cl-Ph | 4-Cl-Ph | H | Chiral (S) |
| 12 | H | - | - | H, iPr | NH₂ | 4-Cl-Ph | 4-Cl-Ph | 5-F | Chiral (S) |
| 13 | H | - | - | CH₂OH, H | NH₂ | 4-Cl-Ph | 4-Cl-Ph | 5-F | Chiral (S) |
| 14 | H | - | - | Et, H | NH₂ | 4-Cl-Ph | 4-Cl-Ph | 5-F | Chiral (S) |
| 15 | H | - | - | H, H | NHMe | 4-Cl-Ph | 4-Cl-Ph | H | - |
| 16 | H | - | - | H, H | NH₂ | 4-Cl-Ph | 4-Cl-Ph | H | - |
| 17 | H | - | - | Me, H | OtBu | 4-Cl-Ph | 4-Cl-Ph | H | Chiral (S) |
| 18 | H | - | - | Me, H | OH | 4-Cl-Ph | 4-Cl-Ph | H | Chiral (S) |
| 19 | H | - | - | H, H | OtBu | 4-Cl-Ph | 4-Cl-Ph | H | - |
| 20 | H | CH₂ | CH₂ | H, H | OtBu | 4-Cl-Ph | 4-Cl-Ph | 5-F | - |
| 21 | H | - | - | H, Me | OtBu | 4-Cl-Ph | 4-Cl-Ph | 5-F | Chiral (S) |
| 22 | H | - | - | H, Me | NH₂ | 4-Cl-Ph | 4-Cl-Ph | 5-OMe | Chiral (S) |
| 23 | H | - | - | H, Me | NMe₂ | 4-Cl-Ph | 4-Cl-Ph | H | Chiral (S) |
| 24 | H | CH₂ | CH₂ | H, H | OtBu | 4-Cl-Ph | 4-Cl-Ph | H | - |
| 25 | H | - | - | H,Me | OH, | 4-Cl-Ph | 4-Cl-Ph | 5-F | sel Na Chiral (S) |
| 26 | H | - | - | H, Me | NHMe | 4-Cl-Ph | 4-Cl-Ph | 5-F | Chiral (S) |
| 27 | H | - | - | H, CH(OH)Me | NH₂ | 4-Cl-Ph | 4-Cl-Ph | H | Chiral (1S, 2R) |
| 28 | H | CH₂ | CH₂ | H, H | NH₂ | 4-Cl-Ph | 4-Cl-Ph | 5-F | - |
| 29 | H | - | - | H, H | OH | 4-Cl-Ph | 4-Cl-Ph | H | - |
| 30 | H | - | - | H,Me | NH₂ | 4-Cl-Ph | 4-Cl-Ph | 2-F | Chiral (S) |
| 31 | H | - | - | H, Me | NHMe | 4-Cl-Ph | 4-Cl-Ph | 2-F | Chiral (S) |
| 32 | H | - | - | H, Me | NH₂ | 4-Cl-Ph | 4-Cl-Ph | 6-F | Chiral (S) |
| 33 | H | - | - | H, Et | NH₂ | 4-Cl-Ph | 4-Cl-Ph | 6-F | Chiral (S) |
| 34 | H | - | - | H,iPr | NH₂ | 4-Cl-Ph | 4-Cl-Ph | 6-F | Chiral (S) |
| 35 | H | - | - | H, Me | NHMe | 4-Cl-Ph | 4-Cl-Ph | 6-F | Chiral (S) |
| 36 | H | - | - | H, Et | NH₂ | 4-Cl-Ph | 4-Cl-Ph | 4-F | Chiral (S) |
| 37 | H | - | - | H, H | NH₂ | 4-Cl-Ph | 4-Cl-Ph | 5-Me | - |
| 38 | H | - | - | H, Me | NH₂ | 4-Cl-Ph | 4-Cl-Ph | 5-Me | Chiral (S) |
| 39 | H | - | - | H, Et | NH₂ | 4-Cl-Ph | 4-Cl-Ph | 5-Me | Chiral (S) |
| 40 | H | - | - | H, iPr | NH₂ | 4-Cl-Ph | 4-Cl-Ph | 5-Me | Chiral (S) |
| 41 | H | - | - | H, Me | NHMe | 4-Cl-Ph | 4-Cl-Ph | 5-Me | Chiral (S) |
| 42 | H | - | - | H, CH₂OH | NH₂ | 4-Cl-Ph | 4-Cl-Ph | 5-Me | Chiral (S) |
| 43 | H | - | - | H, Me | NH₂ | 4-Cl-Ph | 4-Cl-Ph | 5-Cl | Chiral (S) |
| 44 | H | - | - | H, Et | NH₂ | 4-Cl-Ph | 4-Cl-Ph | 5-Cl | Chiral (S) |
| 45 | H | - | - | H, iPr | NH₂ | 4-Cl-Ph | 4-Cl-Ph | 5-Cl | Chiral (S) |
| 46 | H | - | - | H, Me | NHMe | 4-Cl-Ph | 4-Cl-Ph | 5-Cl | Chiral (S) |
| 47 | H | - | - | H, CH₂OH | NH₂ | 4-Cl-Ph | 4-Cl-Ph | 5-Cl | Chiral (S) |
| 48 | H | - | - | H, Me | NH₂ | 4-Cl-Ph | 4-Cl-Ph | 5-Br | Chiral (S) |
| 49 | H | - | - | H, Et | NH₂ | 4-Cl-Ph | 4-Cl-Ph | 5-Br | Chiral (S) |
| 50 | H | - | - | H, iPr | NH₂ | 4-Cl-Ph | 4-Cl-Ph | 5-Br | Chiral (S) |
| 51 | H | - | - | H, Me | NHMe | 4-Cl-Ph | 4-Cl-Ph | 5-Br | Chiral (S) |
| 52 | H | - | - | H, CH₂OH | NH₂ | 4-Cl-Ph | 4-Cl-Ph | 5-Br | Chiral (S) |
| 53 | H | - | - | H, H | NH₂ | 4-Cl-Ph | 4-Cl-Ph | 5-CF₃ | - |
| 54 | H | - | - | H, Me | NH₂ | 4-Cl-Ph | 4-Cl-Ph | 5-CF₃ | Chiral (S) |
| 55 | H | - | - | H, Et | NH₂ | 4-Cl-Ph | 4-Cl-Ph | 5-CF₃ | Chiral (S) |
| 56 | H | - | - | H, iPr | NH₂ | 4-Cl-Ph | 4-Cl-Ph | 5-CF₃ | Chiral (S) |
| 57 | H | - | - | H, Me | NHMe | 4-Cl-Ph | 4-Cl-Ph | 5-CF₃ | Chiral (S) |
| 58 | H | - | - | H, CH₂OH | NH₂ | 4-Cl-Ph | 4-Cl-Ph | 5-CF₃ | Chiral (S) |
| 59 | H | - | - | H, Me | NH₂ | 4-Cl-Ph | 4-Cl-Ph | 5-CN | Chiral (S) |
| 60 | H | - | - | H, Et | NH₂ | 4-Cl-Ph | 4-Cl-Ph | 5-CN | Chiral (S) |
| 61 | H | - | - | H, iPr | NH₂ | 4-Cl-Ph | 4-Cl-Ph | 5-CN | Chiral (S) |
| 62 | H | - | - | H, Me | NHMe | 4-Cl-Ph | 4-Cl-Ph | 5-CN | Chiral (S) |
| 63 | H | - | - | H, CH₂OH | NH₂ | 4-Cl-Ph | 4-Cl-Ph | 5-CN | Chiral (S) |
| 64 | H | - | - | H, Me | OH | 4-Cl-Ph | 4-Cl-Ph | 6-F | Sel CF₃CO₂⁻ Chiral (S) |
| 65 | H | - | - | H, Me | OH | 4-Cl-Ph | 4-Cl-Ph | 5-Me | Sel CF₃CO₂⁻ Chiral (S) |
| 66 | H | - | - | H, Me | OH | 4-Cl-Ph | 4-Cl-Ph | 5-Cl | Sel CF₃CO₂⁻ Chiral (S) |
| 67 | H | - | - | H, Me | OH | 4-Cl-Ph | 4-Cl-Ph | 5-Br | Sel CF₃CO₂⁻ Chiral (S) |
| 68 | H | - | - | H, Me | OH | 4-Cl-Ph | 4-Cl-Ph | 5-CF₃ | Sel CF₃CO₂⁻ Chiral (S) |
| 69 | H | - | - | H, Me | OH | 4-Cl-Ph | 4-Cl-Ph | 5-CN | Sel CF₃CO₂⁻ Chiral (S) |
| 70 | H | - | - | H, Me | NH₂ | 4-Br-Ph | 4-Br-Ph | 5-F | Chiral (S) |
| 71 | H | - | - | H, Me | NH₂ | 4-CF₃-Ph | 4-CF₃-Ph | 5-F | Chiral (S) |
| 72 | H | - | - | H, Me | NH₂ | 4-CN-Ph | 4-CN-Ph | 5-F | Chiral (S) |
| 73 | H | - | - | H, Me | NH₂ | 4-MeO-Ph | 4-MeO-Ph | 5-F | Chiral (S) |
| 74 | H | - | - | H, Me | NH₂ | 4-F-Ph | 4-F-Ph | 5-F | Chiral (S) |
| 75 | H | - | - | H, Me | OMe | 4-Cl-Ph | 4-Cl-Ph | 5-F | Chiral (S) |
| 76 | H | - | - | H, Et | NH₂ | 4-Me-Ph | 4-Me-Ph | 5-F | Chiral (S) |
| 77 | H | - | - | H, Me | NHMe | 4-Br-Ph | 4-Br-Ph | 5-F | Chiral (S) |
| 78 | H | - | - | H, Me | OH | 4-Br-Ph | 4-Br-Ph | 5-F | Chiral (S) |
| 79 | H | - | - | H, Me | OEt | 4-Cl-Ph | 4-Cl-Ph | 5-F | Chiral (S) |
| 80 | H | - | - | H, Et | NH₂ | 4-OCF₃-Ph | 4-OCF₃-Ph | 5-F | Chiral (S) |

**Tableau 1A**

| N° | Caractérisations |
|---|---|
| 1 | Pf : 229°C ; Spectre RMN 1H (300 MHz ; (δ en ppm) ; (DMSO-d6) ; référencé à 2,50 ppm): 1,34 (d, J=7,1 Hz, 3H) ; 2,71 (t, J=6,8 Hz, 2H) ; 2,97 (s, 3H) ; 3,26 - 3,38 (m partiellement masqué, 2H) ; 4,38 (s, 1H) ; 4,42 (m, 1H) ; 4,73 (quin, J=6,8 Hz, 1H) ; 6,98 (s large, 1H) ; 7,31 (d, J=8,5 Hz, 4H) ; 7,36 (d, J=8,5 Hz, 4H) ; 7,39 (s large, 1H) ; 7,44-7,54 (m, 2H) ; 7,80 (s large, 1H) ; 7,89 (m, 1H) ; 8,50 (d, J=7,4 Hz, 1H); Spectre de masse: ES m/z=575 (MH⁺, pic de base), m/z=235 (C₁₃H₉Cl₂⁺), m/z=573 (MH⁻) ; Analyse élémentaire: Calculée: C: 56,35%- H: 4,90%- N: 9,73%- S: 5,57% ; Mesurée: C: 56,72%- H: 4,99%- N: 9,50%- S: 5,65% ; Pouvoir rotatoire : α_{D} = + 10,3 (c=0,428, MeOH) |
| 2 | Pf : 154-156°C ; Spectre RMN 1H (400 MHz ; (δ en ppm) ; (DMSO-d6) ; référencé à 2,50 ppm): 2,73 (t, J=7,0 Hz, 2H) ; 3,00 (s, 3H) ; 3,36 (m, 2H) ; 3,81 (d, J=5,9 Hz, 2H) ; 4,40 (s, 1H) ; 4,72 (m, 1H) ; 7,04 (s large, 1H) ; 7,31 (d, J=8,8 Hz, 4H) ; 7,35 (d, J=8,8 Hz, 4H) ; 7,37 - 7,47 (m, 2H) ; 7,60 - 7,73 (m, 2H) ; 8,84 (t, J=5,9 Hz, 1H); Spectre de masse: ES m/z=579 (MH⁺, pic de base), m/z=235 (C₁₃H₉Cl₂⁺) ; Analyse élémentaire: Calculée: C: 53,89%- H: 4,35%-N: 9,67%- S: 5,53%; Mesurée: C: 53,88%- H: 4,71%- N: 9,16%- S: 5,66% |
| 3 | Pf : 228-230°C ; Spectre RMN 1H (400 MHz ; (δ en ppm) ; (DMSO-d6) ; référencé à 2,50 ppm): 1,33 (d, J=7,3 Hz, 3H) ; 2,73 (t, J=6,8 Hz, 2H) ; 3,00 (s, 3H) ; 3,24- 3,43 (m partiellement masqué, 2H) ; 4,34-4,48 (m, 2H) ; 4,72 (quin, J=6,8 Hz, 1H) ; 6,99 (s large, 1H) ; 7,31 (d, J=8,5 Hz, 4H) ; 7,37 (d, J=8,5 Hz, 4H) ; 7,39 - 7,45 (m, 2H) ; 7,67 (s large, 1H) ; 7,74 (d large, J=8,8 Hz, 1H) ; 8,61 (d, J=7,3 Hz, 1H); |
| | Spectre de masse: ES m/z=593 (MH⁺, pic de base), m/z=637 (MH⁻ + HCO₂H), m/z=591 (MH⁻, pic de base) ; Analyse élémentaire: Calculée: C: 54,64%- H: 4,59%- N: 9,44%- S: 5,40% ; Mesurée: C: 54,09%- H: 4,66%- N: 9,34%- S: 5,43% - H₂O = 1,71%; Pouvoir rotatoire : α_{D} = + 11,3 (c=0,365, MeOH) |
| 4 | Pf : 219-221°C ; Spectre RMN 1H (400 MHz ; (δ en ppm) ; (DMSO-d6] ; référencé à 2,50 ppm): 1,33 (d, J=6,8 Hz, 3H) ; 2,73 (t, J=7,1 Hz, 2H) ; 3,00 (s, 3H) ; 3,35 (m, 2H) ; 4,35 - 4,45 (m, 2H) ; 4,73 (quin, J=6,8 Hz, 1H) ; 6,99 (s large, 1H) ; 7,31 (d, J=8,8 Hz, 4H) ; 7,37 (d, J=8,8 Hz, 4H) ; 7,39 - 7,45 (m, 2H) ; 7,68 (t, J=1,7 Hz, 1H) ; 7,74 (dt, J=9,0; 1,7 Hz, 1H) ; 8,62 (d, J=7,8 Hz, 1H); Spectre de masse: ES m/z=593 (MH⁺, pic de base), m/z=235 (C₁₃H₉Cl₂⁺_{˙}); Analyse élémentaire: Calculée: C: 54,64%- H: 4,59%- N: 9,44%- S: 5,40% ; Mesurée: C: 54,44%- H: 4,54%- N: 9,48%- S: 5,36% - H₂O<0,1%; Pouvoir rotatoire : α_{D} = - 23,3 (c=0,476, DMSO) |
| 5 | Pf : 235°C ; Spectre RMN 1H (300 MHz ; (δ en ppm) ; (DMSO-d6) ; référencé à 2,50 ppm): 1,34 (d, J=7,3 Hz, 3H) ; 2,71 (t, J=6,8 Hz, 2H) ; 2,97 (s, 3H) ; 3,32 (m masqué, 2H) ; 4,38 (s, 1H) ; 4,42 (m, 1H) ; 4,74 (quin, J=6,8 Hz, 1H) ; 6,98 (s large, 1H) ; 7,31 (d, J=8,8 Hz, 4H) ; 7,36 (d, J=8,8 Hz, 4H) ; 7,39 (s large, 1H) ; 7,45 - 7,54 (m, 2H) ; 7,80 (s large, 1H) ; 7,88 (m, 1H) ; 8,50 (d, J=7,6 Hz, 1H) ; Spectre de masse : ES m/z=575 (MH⁺, pic de base), m/z=235 (C₁₃H₉Cl₂⁺_{˙}); Analyse élémentaire: Calculée: C: 56,35%- H: 4,90%- N: 9,73%- S: 5,57% ; Mesurée: C: 56,84%- H: 5,17%- N: 9,56%- S: 5,51%; Pouvoir rotatoire : α_{D} = - 6,6 (c=0,493, DMSO) |
| 6 | Pf : 245-247°C ; Spectre RMN 1H (400 MHz ; (δ en ppm) ; (DMSO-d6) ; référencé à 2,50 ppm): 2,71 (t, J=6,9 Hz, 2H) ; 2,97 (s, 3H) ; 3,33 (m masqué, 2H) ; 3,71 (m, 2H) ; 4,38 (s, 1H) ; 4,44 (m, 1H) ; 4,75 (quin, J=6,9 Hz, 1H) ; 4,93 (t large, J=5,9 Hz, 1H) ; 7,10 (s large, 1H) ; 7,31 (d, J=8,8 Hz, 4H) ; 7,36 (d, J=8,8 Hz, 4H) ; 7,41 (s large, 1H) ; 7,45 - 7,55 (m, 2H) ; 7,81 (s large, 1H) ; 7,88 (m, 1H) ; 8,30 (d, J=7,8 Hz, 1H) ; Spectre de masse : ES m/z=591 (MH⁺, pic de base), m/z=235 (C₁₃H₉Cl₂⁺_{˙}) ; Analyse élémentaire: Calculée: C: 54,83%- H: 4,77%- N: 9,47%- S: 5,42% ; Mesurée: C: 53,60%- H: 5,04%- N: 9,00%- S: 4,90% - H₂O= 1,45%; Pouvoir rotatoire : α_{D} = + 30,9 (c=0,412, DMSO) |
| 7 | Pf : 190-192°C ; Spectre RMN 1H (400 MHz ; (δ en ppm) ; (DMSO-d6) ; référencé à 2,50 ppm): 2,35 (t, J=7,1 Hz, 2H) ; 2,69 (t, J=6,9 Hz, 2H) ; 2,95 (s, 3H) ; 3,34 (t, J=6,9 Hz, 2H) ; 3,44 (m, 2H) ; 4,37 (s, 1H) ; 4,73 (quin, J=6,9 Hz, 1H) ; 6,81 (s large, 1H) ; 7,31 (d, J=8,8 Hz, 4H) ; 7,35 (s masqué, 1H) ; 7,36 (d, J=8,8 Hz, 4H) ; 7,41 - 7,52 (m, 2H) ; 7,74 (s large, 1H) ; 7,80 (m, 1H) ; 8,58 (t, J=5,6 Hz, 1H) ; Spectre de masse : ES m/z=575 (MH⁺, pic de base), m/z=235 (C₁₃H₉Cl₂⁺_{˙}); Analyse élémentaire: Calculée: C: 56,35%- H: 4,90%- N: 9,73%- S: 5,57% ; Mesurée: C: 56,36%- H: 5,10%- N: 9,46%- S: 5,29% - H₂O = 0,34% |
| 8 | Pf : 176°C ; Spectre RMN 1H (400 MHz ; (δ en ppm) ; (DMSO-d6) ; référencé à 2,50 ppm): 2,71 (t, J=6,9 Hz, 2H) ; 2,97 (s, 3H) ; 3,34 (t, J=6,9 Hz, 2H) ; 3,48 (m, 4H) ; 3,58 (m, 4H) ; 4,13 (d, J=5,5 Hz, 2H) ; 4,37 (s, 1H) ; 4,74 (quin, J=6,9 Hz, 1H) ; 7,31 (d, J=8,8 Hz, 4H) ; 7,35 (d, J=8,8 Hz, 4H) ; 7,47-7,55 (m, 2H) ; 7,78 (s large, 1H) ; 7,85 (m, 1H) ; 8,66 (t, J=5,5 Hz, 1H) ; Spectre de masse : ES m/z=631 (MH⁺, pic de base), m/z=235 (C₁₃H₉Cl₂⁺_{˙}) ; Analyse élémentaire: Calculée: C: 57,05%- H: 5,11%- N: 8,87%- S: 5,08% ; Mesurée: C: 57,29%- H: 4,96%-N: 8,29%- S: 4,93% |
| 9 VoieA | Pf : 171-173°C ; Spectre RMN 1H (400 MHz ; (δ en ppm) ; (DMSO-d6) ; référencé à 2,50 ppm): 1,32 (d, J=7,0 Hz, 3H) ; 2,59 (d, J=4,9 Hz, 3H) ; 2,71 (t, J=7,6 Hz, 2H); 2,97 (s, 3H) ; 3,33 (t, J=7,6 Hz partiellement masqué, 2H) ; 4,38 (s, 1H) ; 4,44 (m, 1H) ; 4,74 (m, 1H) ; 7,31 (d, J=8,5 Hz, 4H) ; 7,35 (d, J=8,5 Hz, 4H) ; 7,44-7,52 (m, 2H) ; 7,80 (s, 1H) ; 7,83 - 7,91 (m, 2H) ; 8,59 (d, J=7,8 Hz, 1H) ; Spectre de masse : ES m/z=589 (MH⁺, pic de base) ; Analyse élémentaire: Calculée: C: 57,05%- H: 5,13%- N: 9,50%- S: 5,44% ; Mesurée: C: 55,49%- H: 5,50%- N: 9,14%- S: 5,10% - H₂O = 2,57% ; Pouvoir rotatoire : α_{D} = + 25,4 (c=0,427, DMSO) |
| 9 VoieB | Spectre RMN 1H (400 MHz ; (δ en ppm) ; (DMSO-d6) ; référencé à 2,50 ppm): 1,32 (d, J=7,1 Hz, 3H) ; 2,59 (d, J=4,6 Hz, 3H) ; 2,71 (t, J=7,3 Hz, 2H) ; 2,97 (s, 3H) ; 3,33 (t, J=7,3 Hz, 2H) ; 4,38 (s, 1H) ; 4,44 (m, 1H) ; 4,74 (m, 1H) ; 7,30 (d, J=8,6 Hz, 4H) ; 7,35 (d, J=8,6 Hz, 4H) ; 7,45 - 7,53 (m, 2H) ; 7,80 (s large, 1H) ; 7,82 - 7,92 (m, 2H) ; 8,57 (d, J=7,3 Hz, 1H) ; Spectre de masse : ES m/z=589 (MH⁺, pic de base) ; Analyse élémentaire: Calculée: C: 57,05%- H: 5,13%-N: 9,50%- S: 5,44% ; Mesurée: C: 56,83%- H: 5,51%- N: 8,95%- S: 4,99% - H₂O= 0,57% ; Pouvoir rotatoire : α_{D} = + 25,9 (c=0,415, DMSO) |
| 10 | Pf : 220-222°C ; Spectre RMN 1H (400 MHz ; (δ en ppm) ; (DMSO-d6) ; référencé à 2,50 ppm): 0,90 (t, J=7,3 Hz, 3H) ; 1,63 - 1,86 (m, 2H) ; 2,71 (t, J=6,9 Hz, 2H) ; 2,97 (s, 3H) ; 3,31 (m masqué, 2H) ; 4,32 (m, 1H) ; 4,38 (s, 1H) ; 4,74 (quin, J=6,9 Hz, 1H) ; 7,01 (s large, 1H) ; 7,30 (d, J=8,8 Hz, 4H) ; 7,36 (d, J=8,8 Hz, 4H) ; 7,42 (s large, 1H) ; 7,45- 7,56 (m, 2H) ; 7,80 (s large, 1H) ; 7,88 (m, 1H) ; 8,40 (d, J=7,8 Hz, 1H) ; Spectre de masse : ES m/z=589 (MH⁺, pic de base), m/z=235 (C₁₃H₉Cl₂⁺_{˙}); Analyse élémentaire: Calculée: C: 57,05%- H: 5,13%- N: 9,50%- S: 5,44% ; Mesurée: C: 56,59%- H: 5,43%- N: 9,20%- S: 4,93% - H₂O= 0,56% ; Pouvoir rotatoire : α_{D} = + 17,4 (c=0,427, MeOH) |
| 11 | Spectre RMN 1H (400 MHz ; (δ en ppm) ; (DMSO-d6) ; référencé à 2,50 ppm): 0,88 (d, J=6,4 Hz, 3H) ; 0,90 (d, J=6,4 Hz, 3H) ; 1,50-1,73 (m, 3H) ; 2,71 (m, 2H) ; 2,97 (s, 3H) ; 3,33 (m, 2H) ; 4,37 (s, 1H) ; 4,46 (m, 1H) ; 4,74 (quin, J=6,9 Hz, 1H) ; 6,97 (s large, 1H) ; 7,30 (d, J=8,8 Hz, 4H) ; 7,35 (d, J=8,8 Hz, 4H) ; 7,42 (s large, 1H) ; 7,45 - 7,55 (m, 2H) ; 7,81 (s large, 1H) ; 7,90 (m, 1H) ; 8,46 (d, J=8,2 Hz, 1H) ; Spectre de masse : ES m/z=617 (MH⁺, pic de base) ; Analyse élémentaire: Calculée: C: 58,34%- H: 5,55%- N: 9,07%- S: 5,19% ; Mesurée: C: 58,74%- H: 5,68%- N: 8,69%- S: 4,77% ; Pouvoir rotatoire : α_{D} =+ 20,5 (c=0,401, DMSO) |
| 12 | Pf : 197-199°C ; Spectre RMN 1H (400 MHz ; (δ en ppm) ; (DMSO-d6) ; référencé à 2,50 ppm) : 0,91 (d, J=6,8 Hz, 3H) ; 0,93 (d, J=6,8 Hz, 3H) ; 2,10 (m, 1H) ; 2,74 (t, J=7,2 Hz, 2H) ; 3,00 (s, 3H) ; 3,35 (t, J=7,2 Hz, 2H) ; 4,26 (t, J=8,3 Hz, 1H) ; 4,41 (s, 1H) ; 4,73 (m, 1H); 7,06 (s large, 1H) ; 7,31 (d, J=8,3 Hz, 4H) ; 7,36 (d, J=8,3 Hz, 4H) ; 7,42 (dt, J=9,3 ; 2,2 Hz, 1H) ; 7,48 (s large, 1H) ; 7,67 (t, J=2,2 Hz, 1H) ; 7,76 (dt, J=9,3 ; 2,2 Hz, 1H) ; 8,38 (d, J=8,3 Hz, 1H) ; Spectre de masse : ES m/z=621 (MH⁺, pic de base) ; Analyse élémentaire: Calculée: C: 56,04%- H: 5,03%- N: 9,01%- S: 5,16% ; Mesurée: C: 55,96%- H: 4,67%- N: 9,07%- S: 5,31% ; Pouvoir rotatoire : α_{D} = + 12,1 (c=0,480, DMSO) |
| 13 | Pf : 212-214°C ; Spectre RMN 1H (400 MHz ; (δ en ppm) ; (DMSO-d6) ; référencé à 2,50 ppm) : 2,73 (t, J=7,6 Hz, 2H) ; 3,00 (s, 3H) ; 3,36 (t, J=7,6 H₂, 2H) ; 3,72 (m, 2H) ; 4,41 (s, 1H) ; 4,43 (m, 1H) ; 4,73 (m, 1H) ; 4,91 (m large, 1H) ; 7,10 (s large, 1H) ; 7,32 (d, J=8,4 Hz, 4H) ; 7,37 (d, J=8,4 Hz, 4H) ; 7,41 - 7,46 (m, 2H) ; 7,68 (s large, 1H) ; 7,75 (d large, J=9,1 Hz, 1H) ; 8,44 (d, J=8,3 Hz, 1H) ; Spectre de masse : ES m/z=609 (MH⁺, pic de base); Analyse élémentaire: Calculée: C: 53,21%- H: 4,47%- N: 9,19%- S: 5,26% ; Mesurée: C: 53,01%- H: 4,43%- N: 9,09%- S: 5,01%; Pouvoir rotatoire : α_{D} = + 29,5 (c=0,386, DMSO) |
| 14 | Pf : 212-214°C ; Spectre RMN 1H (400 MHz ; (δ en ppm) ; (DMSO-d6) ; référencé à 2,50 ppm) : 0,90 (t, J=7,3 Hz, 3H) ; 1,68 (m, 1H) ; 1,81 (m, 1H) ; 2,74 (t, J=7,3 Hz, 2H); 3,00 (s, 3H) ; 3,35 (m partiellement masqué, 2H) ; 4,30 (m, 1H) ; 4,41 (s, 1H) ; 4,73 (m, 1H) ; 7,02 (s large, 1H) ; 7,31 (d, J=8,3 Hz, 4H) ; 7,36 (d, J=8,3 Hz, 4H) ; 7,39 - 7,47 (m, 2H) ; 7,68 (s large, 1H) ; 7,76 (d large, J=9,3 Hz, 1H); 8,51 (d, J=8,3 Hz, 1H) ; Spectre de masse : ES m/z=607 (MH⁺, pic de base) ; Analyse élémentaire: Calculée: C: 55,36%- H: 4,81%-N: 9,22%- S: 5,28% ; Mesurée: C: 55,34%- H: 4,86%- N: 9,21%- S: 4,87%; Pouvoir rotatoire : α_{D} =+ 19,7 (c=0,547, DMSO) |
| 15 | Pf : 216°C ; Spectre RMN 1H (400 MHz ; (δ en ppm) ; (DMSO-d6) ; référencé à 2,50 ppm) : 2,60 (d, J=4,6 Hz, 3H) ; 2,71 (t, J=7,5 Hz, 2H) ; 2,98 (s, 3H) ; 3,34 (m masqué, 2H) ; 3,83 (d, J=6,1 Hz, 2H) ; 4,39 (s, 1H) ; 4,74 (m, 1H) ; 7,32 (d, J=8,3 Hz, 4H) ; 7,37 (d, J=8,3 Hz, 4H) ; 7,45 - 7,56 (m, 2H) ; 7,80 (s large, 1H) ; 7,82 - 7,90 (m, 2H) ; 8,84 (t, J=6,1 Hz, 1H) ; Spectre de masse : ES m/z=575 (MH⁺, pic de base) ; Analyse élémentaire: Calculée: C: 56,35%- H: 4,90%- N: 9,73%- S: 5,57% ; Mesurée: C: 56,72%- H: 4,89%- N: 9,65%- S: 5,29% |
| 16 | Pf : 196-198°C; Spectre RMN 1H (400 MHz ; (δ en ppm) ; (DMSO-d6) ; référencé à 2,50 ppm): 2,71 (t, J=7,2 Hz, 2H) ; 2,97 (s, 3H) ; 3,34 (m, 2H) ; 3,82 (d, J=5,9 Hz, 2H) ; 4,37 (s, 1H) ; 4,73 (m, 1H) ; 7,02 (s large, 1H) ; 7,30 (d, J=8,8 Hz, 4H) ; 7,35 (d, J=8,8 Hz, 4H) ; 7,37 (s large, 1H) ; 7,45 - 7,54 (m, 2H) ; 7,79 (t, J=1,8 Hz, 1H) ; 7,86 (dt, J=7,0 ; 1,8 Hz, 1H) ; 8,73 (t, J=5,9 Hz, 1H) ; Spectre de masse: ES m/z=561 (MH⁺, pic de base) ; Analyse élémentaire: Calculée: C: 55,62%- H: 4,67%- N: 9,98%- S: 5,71%- Cl : 12,63% ; Mesurée: C: 55,33%- H: 4,64%- N: 9,68%- S: 5,78%- Cl : 13,08% - H₂O < 0,10% |
| 17 | Spectre RMN 1H (400 MHz ; (δ en ppm) ; (DMSO-d6) ; référencé à 2,50 ppm): 1,38 (m masqué, 3H) ; 1,39 (s, 9H) ; 2,71 (m, 2H) ; 2,97 (s, 3H) ; 3,34 (m partiellement masqué, 2H) ; 4,35 (m, 1H) ; 4,37 (s, 1H) ; 4,74 (m, 1H) ; 7,30 (d, J=8,5 Hz, 4H) ; 7,34 (d, J=8,5 Hz, 4H) ; 7,43-7,55 (m, 2H) ; 7,79 (s large, 1H) ; 7,86 (m, 1H) ; 8,72 (d, J=7,3 Hz, 1H) ; Spectre de masse : ES m/z=632 (MH⁺, pic de base); Pouvoir rotatoire: α_{D} = + 3,6 (c=0,448, DMSO) |
| 18 | Spectre RMN 1H (400 MHz ; (δ en ppm) ; (DMSO-d6) ; référencé à 2,50 ppm): 1,39 (d, J=7,3 Hz, 3H) ; 2,71 (m, 2H) ; 2,97 (s, 3H) ; 3,23-3,45 (m masqué, 2H) ; 4,38 (s, 1H) ; 4,39 (m, 1H) ; 4,74 (m, 1H) ; 7,30 (d, J=8,3 Hz, 4H) ; 7,35 (d, J=8,3 Hz, 4H) ; 7,44 - 7,56 (m, 2H) ; 7,80 (s large, 1H) ; 7,86 (m, 1H) ; 8,70 (d, J=7,3 Hz, 1H) ; Spectre de masse : ES m/z=576 (MH⁺, pic de base) ; Analyse élémentaire: Calculée: C: 56,25%- H: 4,72%- N: 7,29%- S: 5,56% Mesurée: C: 55,68%- H: 4,99%- N: 6,89%- S: 5,01% - H₂O = 0,82% ; Pouvoir rotatoire : α_{D} = + 10 (c=0,195, DMSO) |
| 19 | Analyse élémentaire: Calculée: C: 58,25%- H: 5,38%- N: 6,79%- S: 5,18% Mesurée: C: 57,38%- H: 5,93%- N: 6,58%- S: 4,74% - H₂O = 1,49% ; Spectre de masse: ES m/z=618 (MH⁺), m/z=616 (M-H)⁻, m/z=662 ([M+HCO₂H-H]⁻, pic de base) |
| 20 | Analyse élémentaire: Calculée: C: 57,83%- H: 5,46%- N: 6,32%- S: 4,82% Mesurée: C: 57,65%- H: 5,80%- N: 6,17%- S: 4,68% ; Spectre de masse: ES m/z=664 (MH⁺), m/z=662 (M-H)⁻, m/z=708 ([M+HCO₂H-H]⁻, pic de base) |
| 21 | Pouvoir rotatoire : α_{D} = + 8,6 +/- 0,6 (c=0,401, DMSO) ; Spectre de masse : ES m/z=650 (MH⁺), m/z=648 (M-H)⁻, m/z=694 ([M+HCO₂H-H]⁻, pic de base) |
| 22 | Pf : 183-185°C; Analyse élémentaire: Calculée: C: 55,54%- H: 4,99%- N: 9,25%- S: 5,30% Mesurée: C: 54,77%- H: 5,04%- N: 8,99%- S: 4,80% - H₂O= 0,98% ; Pouvoir rotatoire : α_{D} = + 27,7 (c=0,406, DMSO) |
| 23 | Pouvoir rotatoire : α_{D} = + 8,1 +/- 0,7 (c=0,417, DMSO) ; Spectre de masse : ES m/z=603 (MH⁺), m/z=601 (M-H)⁻, m/z=647 ([M+HCO₂H-H]⁻, pic de base) |
| 24 | Analyse élémentaire: Calculée: C: 59,44%- H: 5,77%- N: 6,50%- S: 4,96%- Cl: 10,97% Mesurée: C: 59,27%- H: 5,93%- N: 6,41%- S: 4,67%- Cl: 11,13% ; Spectre de masse : ES m/z=646 (MH⁺) |
| 25 | Pouvoir rotatoire : α_{D} = + 24,2 +/- 0,7 (c=0,435, DMSO) ; Spectre de masse : ES m/z=594 (MH⁺), m/z=592 (M-H)⁻, m/z=1185 (2M-H)⁻ |
| 26 | Pf : 150°C; Analyse élémentaire: Calculée: C: 55,36%- H: 4,81%- N: 9,22%- S: 5,28% Mesurée: C: 55,35%- H: 5,02%- N: 8,82%- S: 4,88% ; Pouvoir rotatoire : α_{D} =+ 29,2 (c=0,363, DMSO) |
| 27 | Analyse élémentaire: Calculée: C: 55,54%- H: 4,99%- N: 9,25%- S: 5,30% Mesurée: C: 55,43%- H: 5,20%- N: 8,85%- S: 4,88% ; Pouvoir rotatoire : α_{D} = + 25,0 (c=0,447, DMSO) |
| 28 | Pf : 186°C; Analyse élémentaire: Calculée: C: 55,36%- H: 4,81%- N: 9,22%- S: 5,28% Mesurée: C: 55,10%- H: 5,09%- N: 8,98%- S: 4,92% |
| 29 | Analyse élémentaire: Calculée: C: 55,52%- H: 4,48%- N: 7,47%- S: 5,70% Mesurée: C: 54,98%- H: 4,92%- N: 6,75%- S: 5,08% - H₂O = 1,97% ; Spectre de masse : ES m/z=562 (MH⁺, pic de base), m/z=1123 (2M-H)⁻ |
| 30 | Spectre RMN 1H (500 MHz ; (δ en ppm) ; (DMSO-d6)) ; référencé à 2,50 ppm), pour ce lot, nous observons un mélange 50-50 de rotamères avec : 1,30 (d, J=7,2 Hz, 1,5H); 1,31 (d, J=7,2 Hz, 1,5H); 2,61 - 2,69 (m, 1H); 2,75 (t, J=7,1 Hz, 1H); 3,10 (s, 1,5H); 3,12 (s, 1,5 H); 3,33 (m partiellement masqué, 2H); 4,32 (s, 0,5H); 4,33 (s, 0,5 H); 4,41 (m, 1H); 4,70 (m, 1H); 7,10 (s large, 1H); 7,29 - 7,37 (m, 9H); 7,46 (m, 1H); 7,58 (m, 1H); 7,68 (m, 1H); 8,42 (dd, J=7,4; 2,2Hz, 0,5 H); 8,47 (dd, J=7,4; 2,2 Hz, 0,5H) ; Spectre de masse : ES m/z = 593 [M+H]⁺, m/z = 591 [M-H]⁻; Pouvoir rotatoire : α_{D} = + 31,3 (c=0,5716, DMSO) |
| 31 | Spectre RMN 1H (500 MHz ; (δ en ppm) ; (DMSO-d6)), il s'agit d'un mélange 50-50 de rotamères avec : 1,29 (d dédoublé, J=6,7 Hz, 3H); 2,59 - 2,71 (m, 4H); 2,77 (t, J=7,0 Hz, 1H); 3,10 (s, 1,5H); 3,12 (s, 1,5H); 3,33 (m, 2H); 4,32 (s, 0,5H); 4,33 (s, 0,5H); 4,42 (m, 1H); 4,70 (m, 1H); 7,23 - 7,36 (m, 9H); 7,58 (m, 1H); 7,68 (m, 1H); 7,92 (m, 1H) 8,42 - 8,53 (m, 1H); Spectre de masse : ES m/z = 607 [M+H]⁺, m/z = 605 [M-H]⁻; Pouvoir rotatoire : α_{D} = + 25,8 (c=0,562, DMSO) |
| 32 | Spectre RMN 1H (500 MHz ; (δ en ppm) ; (DMSO-d6)) : 1,32 (d, J=7,1 Hz, 3H); 2,71 (J=7,1 Hz, 2H); 2,97 (s, 3H); 3,33 (partiellement masqué, 2H); 4,41 (m, 2H); 4,71 (m, 1H); 7,09 (s large, 1H); 7,27-7,34 (m, 5H); 7,36 (d, J=8,5 Hz, 4H); 7,44 (s large, 1H); 7,48 (ddd, J=8,9; 4,5; 2,8 Hz, 1H); 7,59 (dd, J=6,4; 2,8 Hz, 1H); 8,32 (dd, J=7,4; 3,8 Hz, 1H); Spectre de masse : ES m/z = 593 [M+H]⁺, m/z = 591 [M-H]⁻; Pouvoir rotatoire : α_{D} = + 18,0 (c=0,549, DMSO) |
| 33 | Spectre RMN 1H (500 MHz ; (δ en ppm) ; (DMSO-d6)) : 0,89 (t, J=7,4 Hz, 3H); 1,65 (m, 1H); 1,80 (m, 1H); 2,71 (m, 2H); 2,97 (s, 3H); 3,32 (m partiellement masqué, 2H); 4,35 (m, 1H); 4,40 (s, 1H); 4,72 (m, 1H); 7,11 (s large, 1H); 7,24 - 7,34 (m, 5H); 7,35 - 7,40 (d, J=8,5 Hz, 4H); 7,43 - 7,52 (m, 2H); 7,57 (dd, J=6,3; 3,0 Hz, 1H); 8,24 (dd, J=6,3; 2,6 Hz, 1H); Spectre de masse : ES m/z = 607 [M+H]⁺, m/z = 605 [M-H]⁻; Pouvoir rotatoire : α_{D} = + 16,8 (c=0,578, DMSO) |
| 34 | Spectre RMN 1H (500 MHz ; (δ en ppm) ; (DMSO-d6)) : 0,89 (d, J=6,8 Hz, 3H); 0,93 (d, J=6,8 Hz, 3H); 2,06 (m, 1H); 2,71 (t, J=7,6 Hz, 2H); 2,96 (s, 3H); 3,32 (m partiellement masqué, 2H); 4,33 (dd, J=8,8; 6,7 Hz, 1H); 4,41 (s, 1H); 4,72 (m, 1H); 7,12 (s large, 1H); 7,30 - 7,33 (m, 5H); 7,36 (d, J=8,5 Hz, 4H); 7,45 - 7,52 (m, 2H); 7,54 (dd, J=6,3; 2,7 Hz, 1H); 8,14 (dd, J=8,7; 3,7 Hz, 1H); Spectre de masse : ES m/z = 621 [M+H]⁺, m/z = 619 [M-H]⁻; Pouvoir rotatoire : α_{D} = + 17,7 (c=0,499, DMSO) |
| 35 | Spectre RMN 1H (500 MHz ; (δ en ppm) ; (DMSO-d6)) : 1,29 (d, J=7,1 Hz, 3H); 2,62 (d, J=4,7 Hz, 3H); 2,71 (t, J=7,5Hz, 2H); 2,97 (s, 3H); 3,32 (m partiellement masqué, 2H); 4,40 (s, 1H); 4,43 (m, 1H); 4,71 (m, 1H); 7,30 - 7,34 (m, 5H); 7,36 (d, J=8,5 Hz, 4H); 7,48 (ddd, J=8,9; 4,4; 2,9 Hz, 1H); 7,57 (dd, J=6,3; 2,9 Hz, 1H); 7,93 (q, J=4,7 Hz, 1H; 8,38 (dd, J=7,2; 3,3 Hz, 1H); Spectre de masse : ES m/z = 607 [M+H]+, m/z = 605 [M-H]-; Pouvoir rotatoire : α_{D} = + 11,8 (c=0,568, DMSO) |
| 36 | Spectre RMN 1H (500 MHz ; (δ en ppm) ; (DMSO-d6)) : il s'agit d'un mélange de rotamères 50-50 avec : 0,91 (m, 3H); 1,71 (m, 1H); 1,81 (m, 1H); 2,65 - 2,80 (m, 2H); 3,11 (s, 1,5H); 3,13 (s, 1,5H); 3,34 (m, 2H); 4,25 - 4,38 (m, 2H); 4,72 (m, 1H); 7,02 (s large, 1H); 7,30 (m, 8H); 7,43 - 7,49 (m, 2H); 7,96 - 8,10 (m, 2H); 8,49 (d dédoublé, J= 8,1 Hz, 1H); Spectre de masse : ES m/z = 607 [M+H]⁺, m/z = 605 [M-H]⁻; Pouvoir rotatoire : α_{D} = + 22,4 (c=0,588, DMSO) |
| 37 | Spectre RMN 1H (500 MHz ; (δ en ppm) ; (DMSO-d6)) : 2,36 (s, 3H); 2,70 (t, J=7,5 Hz, 2H); 2,96 (s, 3H); 3,31 - 3,35 (m, 2H); 3,80 (d, J=6,0 Hz, 2H); 4,38 (s, 1H); 4,70 (m, 1H); 7,02 (s large, 1H); 7,31 (m, 5H); 7,36 (m, 5H); 7,58 (s large, 1H); 7,68 (s large, 1H); 8,66 (t, J=6,0 Hz, 1H); Spectre de masse : ES m/z = 575 [M+H]⁺; m/z = 573 [M-H]⁻ |
| 38 | Spectre RMN 1H (500 MHz ; (δ en ppm) ; (DMSO-d6)) : 1,33 (d, J=7,4 Hz, 3H); 2,36 (s, 3H); 2,70 (m, 2H); 2,96 (s, 3H); 3,31 - 3,36 (m, 2H); 4,38 (s, 1H); 4,41 (m, 1H); 4,71 (m, 1H); 6,98 (s large, 1H); 7,28 - 7,40 (m, 10H); 7,59 (s large, 1H); 7,71 (s large, 1H); 8,42 (d, J=7,4 Hz, 1H); Spectre de masse : ES m/z = 589 [M+H]⁺, m/z = 587 [M-H]⁻; Pouvoir rotatoire : α_{D} = + 26,4 (c=0,503, DMSO) |
| 39 | Spectre RMN 1H (500 MHz ; (δ en ppm) ; (DMSO-d6)) : 0,90 (t, J=7,4 Hz, 3H); 1,68 (m, 1H); 1,81 (m, 1H); 2,36 (s, 3H); 2,71 (m, 2H); 2,96 (s, 3H); 3,31 - 3,34 (m, 2H); 4,32 (td, J=8,3; 5,2 Hz, 1H); 4,38 (s, 1H); 4,71 (m, 1H); 7,00 (s large, 1H); 7,31 (m, 5H; 7,35 (d, J=8,5 Hz, 4H); 7,40 (s large, 1H); 7,59 (s large, 1H); 7,73 (s large, 1H); 8,31 (d, J=8,3 Hz, 1H); Spectre de masse : ES m/z = 603 [M+H]⁺, m/z = 601 [M-H]⁻; Pouvoir rotatoire : α_{D} = + 20,4 (c=0,529, DMSO) |
| 40 | Spectre RMN 1H (500 MHz ; (δ en ppm) ; (DMSO-d6)) : 0,91 (d, J=6,9 Hz, 3H); 0,93 (d, J=6,9 Hz, 3H); 2,09 (m, 1H); 2,37 (s, 3H); 2,71 (m, 2H); 2,96 (s, 3H); 3,31 - 3,35 (m, 2H); 4,27 (t, J=8,4 Hz, 1H); 4,38 (s, 1H); 4,71 (m, 1H); 7,06 (s large, 1H); 7,27 - 7,33 (m, 5H); 7,35 (d, J=8,6 Hz, 4H); 7,47 (s large, 1H); 7,58 (s large, 1H); 7,72 (s large, 1H); 8,17 (d, J=8,4 Hz, 1H); Spectre de masse : ES m/z= 617 [M+H]⁺, m/z = 615 [M-H]⁻; Pouvoir rotatoire : α_{D} = + 19,1 (c=0,483, DMSO) |
| 41 | Spectre RMN 1H (500 MHz ; (δ en ppm) ; (DMSO-d6)) : 1,31 (d, J=7,4 Hz, 3H); 2,36 (s, 3H); 2,59 (d, J=4,6 Hz, 3H); 2,70 (m, 2H); 2,96 (s, 3H); 3,33 (m, 2H); 4,38 (s, 1H); 4,43 (m, 1H); 4,70 (m, 1H); 7,31 (m, 5H); 7,35 (d, J=8,5 Hz, 4H); 7,59 (s, 1H); 7,73 (s, 1H); 7,83 (q, J=4,6 Hz, 1H); 8,49 (d, J=7,4 Hz, 1H); Spectre de masse : ES m/z = 603 [M+H]⁺, m/z = 601 [M-H]⁻; Pouvoir rotatoire : α_{D} = + 27,4 (c=0,507, DMSO) |
| 42 | Spectre RMN 1H (500 MHz ; (δ en ppm) ; (DMSO-d6)) : 2,37 (s, 3H); 2,70 (t, J=7,5 Hz, 2H); 2,96 (s, 3H); 3,33 (m, 2H); 3,67 - 3,75 (m, 2H); 4,38 (s, 1H); 4,43 (m, 1H); 4,71 (m, 1H); 4,90 (t, J=5,9 Hz, 1H); 7,09 (s large, 1H); 7,29 - 7,32 (m, 5H); 7,36 (d, J=8,5 Hz, 4H) ; 7,39 (s large, 1H); 7,60 (s, 1H); 7,72 (s, 1H); 8,21 (d, J=8,0 Hz, 1H); Spectre de masse : ES m/z = 605 [M+H]⁺, m/z = 603 [M-H]⁻; Pouvoir rotatoire : α_{D} = + 23,1 (c=0,521, DMSO) |
| 43 | Spectre RMN 1H (400 MHz; (δ en ppm) ; (DMSO-d6)) : 1,33 (d, J=7,4 Hz, 3H); 2,74 (t, J=7,5 Hz, 2H); 3,01 (s, 3H); 3,31- 3,37 (m partiellement masqué, 2H); 4,36 - 4,44 (m, 2H); 4,73 (m, 1H); 6,98 (s large, 1H); 7,32 (d, J=8,5 Hz, 4H); 7,34 (d, J=8,5 Hz, 4H); 7,40 (s large, 1H); 7,61 (t, J=2,0 Hz, 1H); 7,77 (t, J=2,0 Hz, 1H); 7,98 (t, J=2,0 Hz, 1H); 8,65 (d, J=7,4 Hz, 1H); Spectre de masse : ES m/z = 611 [M+H]⁺, m/z = 609 [M-H]⁻; Pouvoir rotatoire : α_{D} = + 27,2 (c=0,569, DMSO) |
| 44 | Spectre RMN 1H (400 MHz ; (δ en ppm) ; (DMSO-d6)) : 0,90 (t, T=7,4 Hz, 3H); 1,69 (m, 1H); 1,81 (m, 1H); 2,75 (t, J=7,3 Hz, 2H); 3,00 (s, 3H); 3,31 - 3,36 (m partiellement masqué, 2H); 4,30 (m, 1H); 4,41 (s, 1H); 4,74 (m, 1H); 7,00 (s large, 1H); 7,31 (d, J=8,5 Hz, 4H); 7,34 (d, J=8,5 Hz, 4H); 7,43 (s large, 1H); 7,61 (t, J=2,0 Hz, 1H); 7,77 (t, J=2,0 Hz, 1H); 8,00 (t, J=2,0 Hz, 1H); 8,55 (d, J=8,2 Hz, 1H); Spectre de masse : ES m/z = 625 [M+H]⁺, m/z = 623 [M-H]⁻; Pouvoir rotatoire : α_{D} = + 22,5 (c=0,498, DMSO) |
| 45 | Spectre RMN 1H (400 MHz ; (δ en ppm) ; (DMSO-d6)) : 0,90 (d, J=6,6 Hz, 3H); 0,92 (d, J=6,6 Hz, 3H); 2,11 (m, 1H); 2,74 (m, 2H); 3,00 (s, 3H); 3,33 (m, 2H); 4,28 (m, 1H); 4,41 (s, 1H); 4,75 (m, 1H); 7,05 (s large, 1H); 7,31 (d, J=8,5 Hz, 4H); 7,35 (d, J=8,5 Hz, 4H); 7,48 (s large, 1H); 7,61 (t, J=1,9 Hz, 1H); 7,76 (t, J=1,9 Hz, 1H); 7,99 (t, J=1,9 Hz, 1H); 8,44 (d, J=8,6 Hz, 1H); Spectre de masse : ES m/z = 639 [M+H]⁺; m/z = 637 [M-H]⁻; Pouvoir rotatoire : α_{D} = + 22,3 (c=0,558, DMSO) |
| 46 | Spectre RMN 1H (400 MHz ; (δ en ppm) ; (DMSO-d6)) : 1,32 (d, J=7,1 Hz, 3H); 2,59 (d, J= 4,7 Hz, 3H); 2,73 (t, J= 7,1 Hz, 2H); 3,01 (s, 3H); 3,33 (m, 2H); 4,37 - 4,46 (m, 2H); 4,73 (m, 1H); 7,32 (d, J= 8,6 Hz, 4H); 7,34 (d, J=8,6 Hz, 4H); 7,62 (t, J= 1,9 Hz, 1H); 7,77 (t, J= 1,9 Hz, 1H); 7,85 (q, J= 4,7 Hz, 1H); 8,00 (t, J= 1,9 Hz, 1H); 8,71 (d, J= 7,5 Hz, 1H); Spectre de masse : ES m/z = 625 [M+H]⁺; m/z = 623 [M-H]⁻; Pouvoir rotatoire : α_{D} = + 28,1 (c=0,557, DMSO) |
| 47 | Spectre RMN 1H (400 MHz ; (δ en ppm) ; (DMSO-d6)) : 2,73 (t, J=7,1 Hz, 2H); 3,01 (s, 3H); 3,34 (t, J=7,1 Hz, 2H); 3,65- 3,76 (m, 2H); 4,39 - 4,46 (m, 2H); 4,74 (m, 1H); 4,89 (t, J=5,9 Hz, 1H); 7,09 (s large, 1H); 7,32 (d, J=8,6 Hz, 4H); 7,35 (d, J=8,6 Hz, 4H); 7,41 (s large, 1H); 7,62 (t, J=1,9 Hz, 1H); 7,77 (t, J=1,9 Hz, 1H); 7,99 (t, J=1,9 Hz, 1H); 8,47 (d, J=8,0 Hz, 1H); Spectre de masse : ES m/z = 627 [M+H]⁺, m/z = 625 [M-H]⁻ ; Pouvoir rotatoire : α_{D} = + 24,4 (c=0,534, DMSO) |
| 48 | Spectre RMN 1H (400 MHz ; (δ en ppm) ; (DMSO-d6)) : 1,33 (d, J=7,2 Hz, 3H); 2,74 (t, J=7,1 Hz, 2H); 3,00 (s, 3H); 3,33 (t, J=7,1 Hz, 2H); 4,35 - 4,44 (m, 2H); 4,72 (m, 1H); 6,97 (s large, 1H); 7,32 (d, J=8,5 Hz, 4H); 7,34 (d, J=8,5 Hz, 4H); 7,40 (s large, 1H); 7,74 (t, J=1,8 Hz, 1H); 7,80 (t, J=1,8 Hz, 1H); 8,12 (t, J=1,8 Hz, 1H); 8,65 (d, J=7,5 Hz, 1H); Spectre de masse : ES m/z = 655 [M+H]⁺; m/z = 653 [M-H]⁻; Pouvoir rotatoire : α_{D} = + 27,7 (c=0,5826, DMSO) |
| 49 | Spectre RMN 1H (400 MHz ; (δ en ppm) ; (DMSO-d6)) : 0,90 (t, J=7,6 Hz, 3H); 1,68 (m, 1H); 1,82 (m, 1H); 2,74 (t, J=7,3 Hz, 2H); 3,00 (s, 3H); 3,33 (t, J=7,3 Hz, 2H); 4,30 (m, 1H); 4,41 (s, 1H); 4,74 (m, 1H); 7,00 (s large, 1H); 7,31 (d, J=8,6 Hz, 4H); 7,34 (d, J=8,6 Hz, 4H); 7,43 (s large, 1H); 7,74 (t, J=1,2 Hz, 1H); 7,81 (t, J=1,2 Hz, 1H) 8,14 (t, J=1,2 Hz, 1H); 8,55 (d, J=8,2 Hz, 1H); Spectre de masse : ES m/z = 667 [M+H]⁺, m/z = 665 [M-H]⁻; Pouvoir rotatoire : α_{D} = + 21,3 (c=0,559, DMSO) |
| 50 | Spectre RMN 1H (400 MHz ; (δ en ppm) ; (DMSO-d6)) : 0,92 (t, J=6,8 Hz, 6H); 2,10 (m, 1H); 2,75 (t, J=7,1 Hz, 2H); 3,00 (s, 3H); 3,32 (m partiellement masqué, 2H); 4,27 (t, J=8,2 Hz, 1H); 4,41 (s, 1H); 4,74 (m, 1H); 7,05 (s large, 1H); 7,30 (d, J=8,6 Hz, 4H); 7,34 (d, J=8,6 Hz, 4H); 7,48 (s large, 1H); 7,74 (t, J=1,2 Hz, 1H); 7,80 (t, J=1,2 Hz, 1H); 8,13 (t, J=1,2 Hz, 1H); 8,45 (d, J=8,2 Hz, 1H); Spectre de masse : ES m/z = 681 [M+H]⁺, m/z = 679 [M-H]⁻; Pouvoir rotatoire : α_{D} = + 22,3 (c=0,522, DMSO) |
| 51 | Spectre RMN 1H (400 MHz; (δ en ppm) ; (DMSO-d6)) : 1,32 (d, J=7,3 Hz, 3H); 2,59 (d, J=4,9 Hz, 3H); 2,74 (t, J=7,1 Hz, 2H); 3,00 (s, 3H); 3,33 (m partiellement masqué, 2H); 4,37 - 4,46 (m, 2H); 4,73 (m, 1H); 7,31 (d, J=8,7 Hz, 4H); 7,34 (d, J=8,7 Hz, 4H); 7,75 (t, J=1,0 Hz, 1H); 7,80 (t, J=1,0 Hz, 1H); 7,85 (q, J=4,9 Hz, 1H); 8,14 (t, J=1,0 Hz, 1H); 8,72 (d, J=7,7 Hz, 1H); Spectre de masse : ES m/z = 667 [M+H]⁺, m/z = 665 [M-H]⁻; Pouvoir rotatoire : α_{D} = + 27,6 (c=0,504, DMSO) |
| 52 | Spectre RMN 1H (400 MHz ; (δ en ppm) ; (DMSO-d6)) : 2,74 (t, J=7,2 Hz, 2H); 3,01 (s, 3H); 3,33 (t, J=7,2 Hz, 2H); 3,71 (m, 2H); 4,41 - 4,47 (m, 2H); 4,74 (m, 1H); 4,89 (t, J=6,1 Hz, 1H); 7,09 (s large, 1H); 7,31 (d, J=8,6 Hz, 4H); 7,35 (d, J=8,6 Hz, 4H); 7,41 (s large, 1H); 7,74 (t, J=1,3 Hz, 1H); 7,80 (t, J=1,3 Hz, 1H); 8,13 (t, J=1,3Hz, 1H); 8,48 (d, J=8,2 Hz, 1H); Spectre de masse : ES m/z = 669 [M+H]⁺, m/z = 667 [M-H]⁻; Pouvoir rotatoire : α_{D} = + 23,3 (c=0,553, DMSO) |
| 53 | Spectre RMN 1H (400 MHz ; (δ en ppm) ; (DMSO-d6)) : 2,76 (t, J=7,3 Hz, 2H); 3,03 (s, 3H); 3,35 (m, 2H); 3,84 (d, J=5,9 Hz, 2H); 4,41 (s, 1H); 4,80 (m, 1H); 7,06 (s large, 1H); 7,30 (d, J=8,6 Hz, 4H); 7,33 (d, J=8,6 Hz, 4H); 7,43 (s large, 1H); 7,86 (s large, 1H); 8,08 (s large, 1H); 8,22 (s large, 1H); 9,04 (t, J=5,9 Hz, 1H); Spectre de masse : ES m/z = 629 [M+H]⁺, m/z = 627 [M-H]⁻ |
| 54 | Spectre RMN 1H (400 MHz ; (δ en ppm) ; (DMSO-d6)) : 1,35 (d, J=7,3 Hz, 3H); 2,76 (t, J=7,2 Hz, 2H); 3,02 (s, 3H); 3,34 (t, J=7,2 Hz, 2H); 4,33 - 4,50 (m, 2H); 4,81 (m, 1H); 6,99 (s large, 1H); 7,30 (d, J=8,7 Hz, 4H); 7,33 (d, J=8,7 Hz, 4H); 7,43 (s large, 1H); 7,86 (s large, 1H); 8,09 (s large, 1H); 8,28 (s large, 1H); 8,83 (d, J=7,7 Hz, 1H); Spectre de masse : ES m/z = 643 [M+H]⁺, m/z = 641 [M-H]⁻; Pouvoir rotatoire : α_{D} = + 22,5 (c=0,516, DMSO) |
| 55 | Spectre RMN 1H (400 MHz, ; (δ en ppm) ; (DMSO-d6)) : 0,91 (t, J=7,3 Hz, 3H); 1,71 (m, 1H); 1,84 (m, 1H); 2,77 (t, J=7,1 Hz, 2H); 3,02 (s, 3H); 3,34 (t, J=7,1 Hz, 2H); 4,34 (m, 1H); 4,41 (s, 1H); 4,81 (m, 1H); 7,02 (s large, 1H); 7,28 - 7,34 (m, 8H); 7,46 (s large, 1H); 7,86 (s large, 1H); 8,10 (s large, 1H); 8,30 (s large, 1H); 8,74(d, J=8,3 Hz, 1H); Spectre de masse : ES m/z = 657 [M+H]⁺, m/z = 655 [M-H]⁻; Pouvoir rotatoire : α_{D} = + 18,3 (c=0,529, DMSO) |
| 56 | Spectre RMN 1H (400 MHz ; (δ en ppm) ; (DMSO-d6)) : 0,92 (t, J=6,8 Hz, 3H); 0,95 (t, J=6,8 Hz, 3H); 2,12 (m, 1H); 2,77 (t, J=7,1 Hz, 2H); 3,02 (s, 3H); 3,33 (t, J=7,1 Hz, 2H); 4,31 (m, 1H); 4,41 (s, 1H); 4,81 (m, 1H); 7,07 (s large, 1H); 7,25 - 7,36 (m, 8H); 7,52 (s large, 1H); 7,86 (s large, 1H); 8,10 (s large, 1H); 8,28 (s large, 1H); 8,66 (d, J=8,5 Hz, 1H); Spectre de masse : ES m/z = 671 [M+H]⁺, m/z = 669 [M-H]⁻; Pouvoir rotatoire : α_{D} = + 17,6 (c=0,548, DMSO) |
| 57 | Spectre RMN 1H (400 MHz ; (δ en ppm) ; (DMSO-d6)) : 1,34 (d, J=7,3 Hz, 3H); 2,60 (d, J=4,6 Hz, 3H); 2,77 (t, J=7,2 Hz, 2H); 3,03 (s, 3H); 3,34 (t, J=7,2 Hz, 2H); 4,41 (s, 1H); 4,46 (m, 1H); 4,81 (m, 1H); 7,26 - 7,37 (m, 8H); 7,83 - 7,92 (m, 2H); 8,10 (s large, 1H); 8,29 (s large, 1H); 8,89 (d, J=7,6 Hz, 1H); Spectre de masse : ES m/z = 657 [M+H]⁺ ; m/z = 655 [M-H]⁻; Pouvoir rotatoire : α_{D} = + 22,1 (c=0,523, DMSO) |
| 58 | Spectre RMN 1H (400 MHz ; (δ en ppm) ; (DMSO-d6)) : 2,77 (t, J=7,3 Hz, 2H); 3,03 (s, 3H); 3,34 (t, J=7,3 Hz, 2H); 3,63- 3,86 (m, 2H); 4,42 (s, 1H); 4,47 (m, 1H); 4,82 (m, 1H); 4,91 (t, J=6,0 Hz, 1H); 7,11 (s large, 1H); 7,30 (d, J=8,6 Hz, 4H); 7,34 (d, J=8,6 Hz, 4H); 7,44 (s large, 1H); 7,87 (s large, 1H); 8,10 (s large, 1H); 8,29 (s large, 1H); 8,68 (d, J=8,0 Hz, 1H); Spectre de masse : ES m/z = 659 [M+H]⁺; m/z = 657 [M-H]⁻ ; Pouvoir rotatoire : α_{D} = + 19,5 (c=0,546, DMSO) |
| 59 | Spectre RMN 1H (400 MHz ; (δ en ppm) ; (DMSO-d6)) : 1,34 (d, J=7,3 Hz, 3H); 2,74 (t, J=7,3 Hz, 2H); 3,03 (s, 3H); 3,36 (t, J=7,3 Hz, 2H); 4,36 - 4,46 (m, 2H); 4,75 (m, 1H); 7,00 (s large, 1H); 7,31 (d, J=8,8 Hz, 4H); 7,34 (d, J=8,8 Hz, 4H); 7,42 (s large, 1H); 8,02 (s large, 1H); 8,09 (s large, 1H); 8,36 (s large, 1H); 8,75 (d, J=7,5Hz, 1H); Spectre de masse : ES m/z = 600 [M+H]⁺, m/z = 598 [M-H]⁻; Pouvoir rotatoire : α_{D} = + 28,9 (c=0,568, DMSO) |
| 60 | Spectre RMN 1H (400 MHz ; (δ en ppm) ; (DMSO-d6)) : 0,91 (t, J=7,6 Hz, 3H); 1,69 (m, 1H); 1,80 (m, 1H); 2,75 (t, J=7,3 Hz, 2H); 3,03 (s, 3H); 3,35 (t, J=7,3 Hz, 2H); 4,32 (m, 1H); 4,40 (s, 1H); 4,75 (m, 1H); 7,02 (s large, 1H); 7,26 - 7,37 (m, 8H); 7,45 (s large, 1H); 8,03 (s large, 1H); 8,10 (s large, 1H); 8,39 (s large, 1H); 8,64(d, J=7,9 Hz, 1H); Spectre de masse : ES m/z = 614 [M+H]⁺, m/z = 612 [M-H]⁻; Pouvoir rotatoire : α_{D} = + 24,4 (c=0,516, DMSO) |
| 61 | Spectre RMN 1H (400 MHz ; (δ en ppm) ; (DMSO-d6)) : 0,92 (d, J=6,8 Hz, 3H); 0,94 (d, J=6,8 Hz, 3H); 2,11 (m, 1H); 2,75 (t, J=7,1 Hz, 2H); 3,03 (s, 3H); 3,35 (t, J=7,1 Hz, 2H); 4,30 (m, 1H); 4,40 (s, 1H); 4,75 (m, 1H); 7,07 (s large, 1H); 7,28 - 7,35 (m, 8H); 7,50 (s large, 1H); 8,02 (s large, 1H); 8,08 (s large, 1H); 8,40 (s large, 1H); 8,52 (d, J=8,8 Hz, 1H); Spectre de masse : ES m/z = 628 [M+H]⁺, m/z = 626 [M-H]⁻; Pouvoir rotatoire : α_{D} = + 25,2 (c=0,562, DMSO) |
| 62 | spectre RMN 1H (400 MHz ; (δ en ppm) ; (DMSO-d6)) : 1,33 (d, J=7,3 Hz, 3H); 2,59 (d, J=4,6 Hz, 3H); 2,75 (t, J=7,1 Hz, 2H); 3,03 (s, 3H); 3,35 (t, J=7,1 Hz, 2H); 4,40 (s, 1H); 4,43 (m, 1H); 4,74 (m, 1H); 7,24 - 7,39 (m, 8H); 7,88 (q, J=4,6 Hz, 1H); 8,03 (s large, 1H); 8,10 (s large, 1H); 8,37 (s large, 1H); 8,81 (d, J=7,6 Hz, 1H); Spectre de masse : ES m/z = 614 [M+H]⁺, m/z = 612 [M-H]⁻; Pouvoir rotatoire : α_{D} = + 28,1 (c=0,578, DMSO) |
| 63 | Spectre RMN 1H (400 MHz ; (δ en ppm) ; (DMSO-d6)) : 2,75 (t, J=7,3 Hz, 2H); 3,03 (s, 3H); 3,36 (m, 2H); 3,73 (m, 2H); 4,41 (s, 1H); 4,44 (m, 1H); 4,75 (m, 1H); 4,91 (t, J=5,9 Hz, 1H); 7,11 (s large, 1H); 7,31 (d, J=8,8 Hz, 4H); 7,35 (d, J=8,8 Hz, 4H); 7,43 (s large, 1H); 8,03 (s large, 1H); 8,09 (s large, 1H); 8,38 (s large, 1H); 8,60 (d, J=8,1 Hz, 1H); Spectre de masse : ES m/z = 616 [M+H]⁺; m/z = 614 [M-H]⁻; Pouvoir rotatoire : α_{D} = + 24,4 (c=0,528, DMSO) |
| 64 | Spectre RMN 1H (400 MHz ; (δ en ppm) ; (DMSO-d6)) : 1,37 (d, J=7,5 Hz, 3H); 3,00 (s large, 3H); 2,50 - 3,60 (m très étalé, 4H); 4,39 (m, 2H); 4,80 (m étalé, 1H); 7,32 - 7,46 (m, 9H); 7,48 - 7,60 (m, 2H); 8,63 (d large, J=7,0 Hz, 1H); 12,70 (m étalé, 1H); Spectre de masse : ES m/z = 594 [M+H]⁺, m/z = 592 [M-H]⁻; Pouvoir rotatoire : α_{D} = + 20,7 (c=0,326, DMSO) |
| 65 | Spectre RMN 1H (500 MHz ; (δ en ppm) ; (DMSO-d6)) : 1,40 (d, J=7,3 Hz, 3H); 2,38 (s, 3H); 3,00 (s large, 3H); 2,70 - 4,30 (m très étalé, 5H); 4,41 (m, 1H); 4,83 (m étalé, 1H); 7,33 (s large, 1H); 7,33-7,47 (m, 8H); 7,61 (s large, 1H); 7,74 (s large, 1H); 8,71 (d, J=7,5 Hz, 1H); 12,61 (m étalé, 1H); Spectre de masse : ES m/z = 590 [M+H]⁺, m/z = 588 [M-H]⁻; Pouvoir rotatoire : α_{D} = + 13 (c=0,313, DMSO) |
| 66 | Spectre RMN 1H (500 MHz ; (δ en ppm) ; (DMSO-d6)) : 1,40 (d, J=7,3 Hz, 3H); 2,70 - 4,30 (m très étalé, 5H); 3,04 (s large, 3H); 4,42 (m, 1H); 4,85 (m étalé, 1H); 7,38 (m, 8H); 7,66 (s large, 1H); 7,79 (s large, 1H); 7,99 (s large, 1H); 8,89 (d, J=7,4 Hz, 1H); 12,67 (m étalé, 1H); Spectre de masse : ES m/z = 610 [M+H]⁺, m/z = 608 [M-H]⁻; Pouvoir rotatoire : α_{D} = + 15,8 (c=0,310, DMSO) |
| 67 | Spectre RMN 1H (500 MHz ; (δ en ppm) ; (DMSO-d6)) : 1,40 (d, J=7,3 Hz, 3H); 2,70 - 4,30 (m très étalé, 5H); 3,03 (s large, 1H); 4,41 (m, 1H); 4,84 (m étalé, 1H); 7,23 (m, 8H); 7,78 (s large, 1H); 7,82 (s large, 1H); 8,12 (s large, 1H); 8,90 (d, J=7,3 Hz, 1H); 12,65 (m étalé, 1H); Spectre de masse : ES m/z = 654 [M+H]⁺; m/z = 652 [M-H]⁻; Pouvoir rotatoire : α_{D} = + 20,9 (c=0,5236, DMSO) |
| 68 | Spectre RMN 1H (500 MHz ; (δ en ppm) ; (DMSO-d6)) : 1,42 (d, J=7,3 Hz, 3H); 2,70 - 4,30 (m très étalé, 5H); 3,05 (s large, 3H); 4,45 (m, 1H); 4,93 (m étalé, 1H); 7,37 (m, 8H); 7,91 (s large, 1H); 8,12 (s large, 1H); 8,29 (s large, 1H); 9,06 (d, J=7,3 Hz, 1H); 12,70 (m étalé, 1H); Spectre de masse : ES m/z = 644 [M+H]⁺, m/z = 642 [M-H]⁻; Pouvoir rotatoire : α_{D} = + 14,3 (c=0,349, DMSO) |
| 69 | Spectre RMN 1H (500 MHz ; (δ en ppm) ; (DMSO-d6)) : 1,41 (d, J=7,3 Hz, 3H); 2,70 - 4,30 (m très étalé, 5H); 3,05 (s large, 3H); 4,43 (m, 1H); 4,83 (m étalé, 1H); 7,37 (m, δH); 8,05 (s large, 1H); 8,12 (s large, 1H); 8,36 (s large, 1H); 8,97 (d, J=7,2 Hz, 1H); 12,72 (m étalé, 1H); Spectre de masse : ES m/z = 601 [M+H]⁺, m/z = 599 [M-H]⁻; Pouvoir rotatoire : α_{D} = + 8 (c=0,226, DMSO) |
| 70 | Spectre RMN 1H (400 MHz ; (δ en ppm) ; (DMSO-d6)) : 1,33 (d, J=7,1 Hz, 3H); 2,73 (m, 2H); 3,00 (s, 3H); 3,35 (m, 2H); 4,38 (s, 1H); 4,41 (m, 1H); 4,72 (m, 1H); 6,98 (s large, 1H); 7,30 (d, J=8,6 Hz, 4H); 7,39 (s large, 1H); 7,42 (m partiellement masqué, 1H); 7,45 (d, J=8,6 Hz, 4H); 7,67 (t, J=1,8 Hz, 1H); 7,74 (dt, J=9,2; 1,8 Hz, 1H); 8,60 (d, J=7,5 Hz, 1H); Spectre de masse : ES m/z = 681 [M+H]⁺, m/z = 679 [M-H]⁻; Pouvoir rotatoire : α_{D} = + 21,4 (c=0,352, DMSO) |
| 71 | Spectre RMN 1H (400 MHz ; (δ en ppm) ; (DMSO-d6)) : 1,33 (d, J=7,1 Hz, 3H); 2,81 (t, J=7,6 Hz, 2H); 3,01 (s, 3H); 3,41(t, J=7,6 Hz, 2H); 4,41 (m, 1H); 4,66 (s, 1H); 4,77 (m, 1H); 6,98 (s large, 1H); 7,39 (s large, 1H); 7,43 (dt, J=9,5; 2,0 Hz, 1H); 7,61 (d, J=8,7 Hz, 4H); 7,64 (d, J=8,7 Hz, 4H); 7,69 (t, J=2,0 Hz, 1H); 7,75 (dt, J=9,5; 2,0 Hz, 1H); 8,61 (d, J=7,6 Hz, 1H); Spectre de masse : ES m/z = 661_[M+H]⁺, m/z = 659 [M-H]⁻; Pouvoir rotatoire : α_{D} = + 21,8 (c=0,361, DMSO) |
| 72 | Spectre RMN 1H (400 MHz ; (δ en ppm) ; (DMSO-d6)) : 1,34 (d, J=7,3 Hz, 3H); 2,79 (t, J=7,5 Hz, 2H); 3,01 (s, 3H); 3,39(m, 2H); 4,41 (m, 1H); 4,65 (s, 1H); 4,76 (m, 1H); 6,98 (s large, 1H); 7,40 (s large, 1H); 7,42 (dt, J=9,6; 1,9 Hz, 1H); 7,58 (d, J=8,6 Hz, 4H); 7,68 (t, J=1,9 Hz, 1H); 7,74 (m, 5H); 8,60 (d, J=7,6 Hz, 1H); Spectre de masse ES m/z = 575 [M+H]⁺, m/z = 573 [M-H]⁻; Pouvoir rotatoire : α_{D} = + 16 (c=0,289, DMSO) |
| 73 | Spectre RMN 1H (400 MHz ; (δ en ppm) ; (DMSO-d6)) : 1,33 (d, J=7,3 Hz, 3H); 2,67 (t, J=7,2 Hz, 2H); 2,99 (s, 3H); 3,31 (m partiellement masqué, 2H); 3,67 (s, 6H); 4,19 (s, 1H); 4,40 (m, 1H); 4,69 (m, 1H); 6,79 (d, J=8,8 Hz, 4H); 6,98 (s large, 1H); 7,22 (d, J=8,8 Hz, 4H); 7,39 (s large, 1H); 7,42 (dt, J=9,5; 1,8 Hz, 1H); 7,67 (t, J=1,8 Hz, 1H); 7,73 (d large, J=9,5 Hz, 1H); 8,60 (d, J=7,7 Hz, 1H); Spectre de masse : ES m/z = 585 [M+H]⁺, m/z = 583 [M-H]⁻; Pouvoir rotatoire α_{D} = + 18,9 (c=0,398, DMSO) |
| 74 | Spectre RMN 1H (400 MHz ; (δ en ppm) ; (DMSO-d6)) : 1,33 (d, J=7,3 Hz, 3H); 2,72 (t, J=7,1 Hz, 2H); 2,99 (s, 3H); 3,33 (m partiellement masqué, 2H); 4,39 (s, 1H); 4,40 (m, 1H); 4,71 (m, 1H); 6,98 (s large, 1H); 7,07 (t, J=8,8 Hz, 4H); 7,33 - 7,45 (m, 6H); 7,67 (s large, 1H); 7,74 (d large, J=9,5 Hz, 1H); 8,60 (d, J=7,7 Hz, 1H); Spectre de masse : ES m/z = 561 [M+H]⁺, m/z = 559 [M-H]⁻; Pouvoir rotatoire : α_{D} = + 18,1 (c=0,309, DMSO) |
| 75 | Spectre RMN 1H (400 MHz ; (δ en ppm) ; (DMSO-d6)) : 1,41 (d, J=7,3 Hz, 3H); 2,74 (m, 2H); 3,00 (s, 3H); 3,36 (m, 2H); 3,64 (s, 3H); 4,40 (s, 1H); 4,48 (m, 1H); 4,72 (m, 1H); 7,31 (d, J=8,6 Hz, 4H); 7,36 (d, J=8,6 Hz, 4H); 7,46(dm, J=9,5 Hz, 1H); 7,68 (s large, 1H); 7,71 (d large, J=9,5 Hz, 1H); 8,93 (d, J=6,8 Hz, 1H); Spectre de masse : ES m/z = 608 [M+H]⁺, m/z = 606 [M-H]⁻; Pouvoir rotatoire : α_{D} = + 6,7 (c=0,347, DMSO) |
| 76 | Spectre RMN 1H (400 MHz ; (δ en ppm) ; (DMSO-d6)) : 0,90 (t, J=7,3 Hz, 3H); 1,70 (m, 1H); 1,81 (m, 1H); 2,20 (s, 6H); 2,69 (m, 2H); 2,99 (s, 3H); 3,33 (m partiellement masqué, 2H); 4,22 (s, 1H); 4,30 (m, 1H); 4,70 (m, 1H); 7,01 (s large, 1H); 7,03 (d, J=8,1 Hz, 4H); 7,20 (d, J=8,1 Hz, 4H); 7,39 - 7,44 (m, 2H); 7,67 (s large, 1H); 7,75 (dm, J=9,5 Hz, 1H); 8,50 (d, J=7,8 Hz, 1H); Spectre de masse : ES m/z = 567 [M+H]⁺; m/z = 565 [M-H]⁻; Pouvoir rotatoire : α_{D} = + 17,7 (c=0,474, DMSO) |
| 77 | Spectre RMN 1H (400 MHz ; (δ en ppm) ; (DMSO-d6)) : 1,32 (d, J=7,3 Hz, 3H); 2,59 (d, J=4,7 Hz, 3H); 2,74 (m, 2H); 3,00 (s, 3H); 3,35 (m, 2H); 4,37 (s, 1H); 4,42 (m, 1H); 4,72 (m, 1H); 7,29 (d, J=8,7 Hz, 4H); 7,42 (m partiellement masqué, 1H); 7,45 (d, J=8,7 Hz, 4H); 7,68 (t, J=1,9 Hz, 1H); 7,76 (ddd, J=9,4; 1,9 Hz, 1H); 7,85 (q, J=4,7 Hz, 1H); 8,67 (d, J=7,6 Hz, 1H); Spectre de masse : ES m/z = 695 [M+H]⁺, m/z = 693 [M-H]⁻; Pouvoir rotatoire : α_{D} = + 20,6 (c=0,399, DMSO) |
| 78 | Spectre RMN 1H (400 MHz ; (δ en ppm) ; (DMSO-d6)) : 1,34 (d, J=7,3 Hz, 3H); 2,74 (m, 2H); 2,99 (s, 3H); 3,34 (m, 2H); 4,19 (m, 1H); 4,39 (s, 1H); 4,73 (m, 1H); 7,30 (d, J=8,7 Hz, 4H); 7,42 (m partiellement masqué, 1H); 7,45 (d, J=8,7 Hz, 4H); 7,60 - 7,68 (m, 2H); 8,53 (d large, J=6,6 Hz, 1H); 12,70 (m très étalé, 1H); Spectre de masse : ES m/z = 682 [M+H]⁺, m/z = 680 [M-H]⁻; Pouvoir rotatoire : α_{D} = - 9,1 (c=0,351, DMSO) |
| 79 | Spectre RMN 1H (400 MHz ; (δ en ppm) ; (DMSO-d6)) : 1,18 (t, J=7,1 Hz, 3H); 1,41 (d, J=7,3 Hz, 3H); 2,74 (m, 2H); 3,01 (s, 3H); 3,36 (m, 2H); 4,11 (m, 2H); 4,40 (s, 1H); 4,45 (m, 1H); 4,73 (m, 1H); 7,31 (d, J=8,6 Hz, 4H); 7,36 (d, J=8,6 Hz, 4H); 7,46 (dt, J=9,5; 2,0 Hz, 1H); 7,68 (t, J=2,0 Hz, 1H); 7,71 (dt, J=9,7; 2,0 Hz, 1H); 8,91 (d, J=7,0 Hz, 1H); Spectre de masse : ES m/z = 622 [M+H]⁺; m/z = 620 [M-H]⁻; Analyse élémentaire: Calculée: C: 55,95%- H: 4,86%- N: 6,75%- S: 5,15% Mesurée: C: 55,61%-H: 5,01%-N: 6,70%-S: 4,85% Pouvoir rotatoire : α_{D} = + 5 (c=0,518, DMSO) |
| 80 | Spectre RMN 1H (400 MHz ; (δ en ppm) ; (DMSO-d6)) : 0,90 (t, J=7,3 Hz, 3H); 1,69 (m, 1H); 1,80 (m, 1H); 2,77 (t, J=7,3 Hz, 2H); 3,00 (s, 3H); 3,37 (t, J=7,3 Hz, 2H); 4,30 (m, 1H); 4,51 (s, 1H); 4,74 (m, 1H); 7,00 (s large,1H); 7,26 (d, J=8,8 Hz, 4H); 7,43 (m, 2H); 7,49 (d, J=8,8 Hz, 4H); 7,69 (t, J=1,7 Hz, 1H); 7,76 (dt, J=9,4; 1,7 Hz, 1H); 8,50 (d, J=8,1 Hz, 1H); Spectre de masse : ES m/z = 707 [M+H]⁺; m/z = 705 [M-H]⁻; Pouvoir rotatoire : α_{D} = + 18,2 (c=0,5976, DMSO) ; Analyse élémentaire: Calculée: C: 50,99%- H: 4,14%- N: 7,93%- S: 4,54% Mesurée: C: 50,75%- H: 4,48%- N: 7,92%- S: 4,39% |

Les composés selon l'invention ont fait l'objet d'essais pharmacologiques permettant de déterminer l'activité vis-à-vis des récepteurs humains aux cannabinoïdes de type CB1. L'efficacité des composés de formule (I) a été déterminée dans un test fonctionnel mesurant l'activité des récepteurs aux cannabinoïdes CB1 1 (test de l'AMP cyclique intracellulaire). Le test de détection de l'AMP cyclique intracellulaire dans les cellules U373MG exprimant naturellement le récepteur CB1 humain, a été effectué comme décrit dans la référence: Bouaboula et al., 1995, J. Biol. Chem. 270:13973-13980. Le kit *HTRF cAMP Dynamic Kit* de CisBio a été utilisé pour la quantification de l'AMP cyclique intracellulaire. Dans ce test, les CI₅₀ sont comprises entre 0,001 µM et 2 µM.

Par exemple, les composés n° 1, 3, 4, 6, 12, 36, 56, 68, 70 et 79 ont montré des CI₅₀ respectivement de 0,043 ; 0,009 ; 0,284 ; 0,020 ; 0,009 ; 0,952 ; 0,041 ; 0,024 ; 0,031 et 0,070 µM.

D'autres essais consistant à mesurer l'activité *in vivo* des composés de l'invention ont été effectués. Leur activité antagoniste a été montrée au moyen du modèle d'hypothermie induite par un agoniste des récepteurs aux cannabinoïdes CB (CP55,940 racémique (1RS, 3RS, 4RS)- 3-[hydroxy-2-(1,1-diméthylheptyl)phényl]-4-(3-hydroxypropyl)cyclohexane-1-ol) à une dose de 1,25mg/kg) chez la souris, selon la méthode décrite par Pertwee R.G. dans Marijuana 84, Harvey D.J. eds, Oxford IRL Press, 263-277 (1985). Au temps 0 min, la température rectale des souris mâles CD1 est mesurée avant l'injection du produit à tester. A 30 minutes, une nouvelle mesure de la température rectale des souris est effectuée et l'agoniste CP55,940 racémique (1RS, 3RS, 4RS-3-[hydroxy-2-(1,1-diméthylheptyl)phényl]-4-(3-hydroxypropyl)cyclohexane-1-ol) (1,25 mg/kg i.p. en crémophor 10%) est administré. A 90 minutes, la température rectale est de nouveau mesurée. Les résultats sont exprimés en % par rapport au lot témoin injecté avec le CP 55 940. (température minimale) et au lot véhicule sans traitement avec le CP55940 (température maximale). Par exemple, les composés n° 1, 38, 55 et 57 ont montré des pourcentages d'inhibition de la baisse de température induite par l'agoniste respectivement de 5%, 17%, 28% et 21% à 3mg/kg po.

Leur activité antagoniste a également été montrée au moyen du modèle de l'inhibition du transit gastrointestinal induit par le CP55,940 racémique (1RS, 3RS, 4RS-3-[hydroxy-2-(1,1-diméthylheptyl)phényl]-4-(3-hydroxypropyl)cyclohexane-1-ol) chez la souris, selon la méthode décrite par Rinaldi-Carmona et al., J.Pharmacol.Exp.Ther. 2004, 310, 905-914. En bref, des souris mâles CD1 reçoivent le produit à tester *per os* 30 minutes ou 2 heures avant l'administration de l'agoniste CP55,940 racémique (1RS, 3RS, 4RS-3-[hydroxy-2-(1,1-diméthylheptyl)hényl]-4-(3-hydroxypropyl)cyclohexane-1-ol) (0,15 mg/kg ip en crémophore 10%). Après encore 30 minutes, les animaux reçoivent un bolus de charbon *po.* Trente minutes plus tard, les animaux sont euthanasiés (CO₂/O₂) et l'intestin est disséqué. La progression du bolus de charbon dans l'intestin est exprimée en pourcentage de la longueur totale de l'intestin.

Par exemple, les composés n° 1, 3, 16, 55, 57 et 68 ont montré des pourcentages d'inhibition à 1mg/kg respectivement de 39,2%, 49%, 18%, 74%, 78% et 7% à 3 heures après l'administration du produit.

En conséquence, les composés de l'invention de formule (I) sont des antagonistes des récepteurs aux cannabinoïdes de type CB1 *in vitro* et *in vivo.* Certains composés sont actifs *in vivo* à la fois sur le test d'hypothermie et de transit, et certains composés montrent des activités dissociées entre le test d'hypothermie et de transit.

Ainsi les composés selon l'invention peuvent être utilisés dans le traitement ou la prévention de maladies impliquant les récepteurs aux cannabinoïdes CB 1.

Par exemple et de manière non limitative, les composés de formule (I) sont utiles comme médicaments psychotropes, notamment pour le traitement des désordres psychiatriques incluant l'anxiété, la dépression, les troubles de l'humeur, l'insomnie, les troubles délirants, les troubles obsessionnels, les psychoses en général, la schizophrénie, les troubles déficit de l'attention et de l'hyperactivité (TDAH) chez les enfants hyperkinétiques (MBD) ainsi que pour le traitement des troubles liés à l'utilisation de substances psychotropes, notamment dans le cas d'un abus d'une substance et/ou de dépendance à une substance, y compris la dépendance alcoolique et la dépendance nicotinique et les troubles de sevrage. Les composés de formule (I) selon l'invention peuvent être utilisés comme médicaments pour le traitement de la migraine, du stress, des maladies d'origine psychosomatique, des crises d'attaques de panique, de l'épilepsie, des troubles du mouvement, en particulier des dyskinésies ou de la maladie de Parkinson, des tremblements et de la dystonie.

Les composés de formule (I) selon l'invention peuvent être utilisés comme médicaments pour le cancer de la peau et la protection de la peau.

Les composés de formule (I) selon l'invention peuvent également être utilisés comme médicaments dans le traitement des troubles mnésiques, des troubles cognitifs, en particulier dans le traitement des troubles cognitifs liés aux démences séniles, à la maladie d'Alzheimer, à la schizophrénie et aux maladies neurodégénératives, ainsi que dans le traitement des troubles de l'attention ou de la vigilance.

De plus, les composés de formule (I) peuvent être utiles comme neuroprotecteurs, dans le traitement de l'ischémie, des traumatismes crâniens et le traitement des maladies neurodégénératives : incluant la chorée de Huntington, le syndrome de Tourrette.

Les composés de formule (I) selon l'invention peuvent être utilisés comme médicaments dans le traitement de la douleur : les douleurs neuropathiques, les douleurs aiguës périphériques, les douleurs chroniques et les douleurs d'origine inflammatoire.

Les composés de formule (I) selon l'invention peuvent être utilisés comme médicaments dans le traitement des troubles de l'appétit, de l'appétence (pour les sucres, carbohydrates, drogues, alcools ou toute substance appétissante) et/ou des conduites alimentaires, notamment pour le traitement de la boulimie ainsi que pour le traitement du diabète de type II ou diabète non insulinodépendant et pour le traitement des dyslipidémies, du syndrome métabolique. Ainsi les composés de formule (I) selon l'invention sont utiles dans le traitement de l'obésité et des risques associés à l'obésité, notamment les risques cardio-vasculaires.

De plus, les composés de formule (I) selon l'invention peuvent être utilisés en tant que médicaments dans le traitement des troubles gastro-intestinaux, des troubles diarrhéiques, des ulcères, des vomissements, des troubles vésicaux et urinaires, des troubles d'origine endocrinienne, des troubles cardio-vasculaires, de l'hypotension, du choc hémorragique, du choc septique, de la cirrhose, de la fibrose hépatique, de la stéatohépatite et de la stéatose hépatique, quelle que soit l'étiologie de ces affections : en particulier virus, alcool, médicament, produit chimique, maladie auto-immune, obésité, diabète, maladie métabolique congénitale, (hémochromatose, déficit en alpha-1 antitrypsine, maladie de Wilson, etc...), de la cirrhose chronique du foie, des fibroses, de la stéatohépatite non alcoolique (NASH), de l'asthme, des maladies pulmonaires chronique obstructive, du syndrome de Raynaud, du glaucome, des troubles de la fertilité, des phénomènes inflammatoires, des maladies inflammatoires, des maladies du système immunitaire, en particulier auto-immunes et neuroinflammatoires telles que l'arthrite rhumatoïde, l'arthrite réactionnelle, les maladies entraînant une démyélinisation, la sclérose en plaque, des maladies infectieuses et virales telles que les encéphalites, des accidents vasculaires cérébraux ainsi qu'en tant que médicaments pour la chimiothérapie anticancéreuse, pour le traitement du syndrome de Guillain-Barré, pour le traitement de l'ostéoporose et l'apnée du sommeil.

Selon un de ses aspects, la présente invention est relative à l'utilisation d'un composé de formule (I), de ses sels pharmaceutiquement acceptables et de leurs solvats ou hydrates pour le traitement des troubles et maladies indiqués ci-dessus.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques pour le traitement des troubles ou des maladies cités ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

Est également décrite une méthode de traitement des pathologies ci-dessus indiquées qui comprend l'administration, à un patient, d'une dose efficace d'un composé selon l'invention, ou d'un de ses sels pharmaceutiquement acceptables.

## Revendications

1. Composé de formule (I) 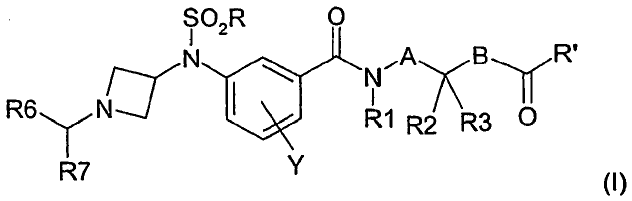 dans laquelle :
R représente un groupe (C₁-C₆)alkyle, halo(C₁-C₆)alkyle;
R' représente un groupe NR4R5, OR8 ;
A et B, s'ils sont présents, représentent indépendamment l'un de l'autre, un ou deux atomes de carbone, ces atomes de carbones étant substitués par un ou plusieurs hydrogènes, groupes (C₁-C₆)alkyle ; le ou les groupes (C₁-C₆)alkyle étant éventuellement substitués par un ou plusieurs groupes hydroxy, (C₁-C₆)alcoxy, halo(C₁-C₆)alkyle, (C₁-C₆)alkylS(O)ₚ, NR4R5, CONR4R5 ;
A+B représentent au maximum deux carbones ;
R1 représente un atome d'hydrogène ou un groupe (C₁-C₆)alkyle ;
R2 et R3 représentent indépendamment l'un de l'autre, un atome d'hydrogène, un groupe (C₁-C₆)alkyle ; le groupe (C₁-C₆)alkyle étant éventuellement substitué par un ou plusieurs groupes hydroxy, (C₁-C₆)alcoxy, halo(C₁-C₆)alkyle, (C₁-C₆)alkylS(O)ₚ, NR4R5, CONR4R5 ;
R4 et R5 représentent indépendamment l'un de l'autre, un atome d'hydrogène, un groupe (C₁-C₆)alkyle, ou forment ensemble, avec l'atome d'azote qui les porte, un hétérocycle du type azétidine, pyrrolidine, pipéridine, azepane, pipérazine, homopipérazine, morpholine, thiomorpholine, thiomorpholine S-oxyde, thiomorpholine S-dioxyde, cet hétérocycle étant éventuellement substitué par un groupe (C₁-C₆)alkyle ;
R6 et R7 représentent chacun un groupe phényle, éventuellement substitué par un ou plusieurs atomes ou groupes choisis parmi un atome d'hydrogène, un halogène, un groupe (C₁-C₆)alkyle, halo(C₁-C₆)alkyle, (C₁-C₆)alcoxy, halo(C₁-C₆)alcoxy ou cyano; ,
Y représente un atome d'hydrogène, un halogène, un groupe (C₁-C₆)alkyle, halo(C₁-C₆)alkyle, (C₁-C₆)alcoxy, halo(C₁-C₆)alcoxy, (C₁-C₆)alkylS(O)ₚ ou cyano ;
R8 représente un atome d'hydrogène, un groupe (C₁-C₆)alkyle, un groupe halo(C₁-C₆)alkyle, un groupe allyle, un groupe phényle(C₁-C₆)alkyle, le groupe phényle étant éventuellement substitué par 1 ou 2 groupes O-méthyle ;
p représente un nombre entier choisi parmi 0, 1 ou 2 ;
à l'état de base ou de sel d'addition à un acide ou à une base.

2. Composé de formule (I) selon la revendication 1 **caractérisé en ce que** :
R' est un NR4R5, OR8 ;
R représente un méthyle ;
A et B, s'ils sont présents, représentent indépendamment l'un de l'autre, un -CH₂- ;
R1 représente un atome d'hydrogène ;
R2 et R3 représentent indépendamment l'un de l'autre, un atome d'hydrogène, un groupe (C₁-C₆)alkyle ; le groupe (C₁-C₆)alkyle étant éventuellement substitué par un groupe hydroxy ;
R4 et R5 représentent indépendamment l'un de l'autre un atome d'hydrogène ou un méthyle ou forment ensemble, avec l'atome d'azote qui les porte, une morpholine ;
R6 et R7 représentent chacun un groupe phényle, éventuellement substitué par un ou plusieurs atomes ou groupes choisis parmi un halogène, un groupe (C₁-C₆)alkyle, halo(C₁-C₆)alkyle, (C₁-C₆)alcoxy, halo(C₁-C₆)alcoxy ou cyano en position para ;
R8 représente un atome d'hydrogène, un groupe (C₁-C₆)alkyle ;
Y représente un hydrogène, un halogène, un groupe (C₁-C₆)alcoxy ; un groupe (C₁-C₆)alkyle ;un cyano ; un groupe halo(C₁-C₆)alkyle ;
sous forme d'énantiomères ou de diastéréoisomères ou de mélanges de ces formes ;
à l'état de base ou de sel d'addition à un acide ou à une base.

3. Composé de formule (I) selon la revendication 1 **caractérisé en ce que** :
R' est un NR4R5, OR8 ;
R représente un méthyle ;
A et B, s'ils sont présents, représentent indépendamment l'un de l'autre, un -CH₂- ;
R1 représente un atome d'hydrogène ;
R2 et R3 représentent indépendamment l'un de l'autre, un atome d'hydrogène, un groupe (C₁-C₆)alkyle ; le groupe (C₁-C₆)alkyle étant éventuellement substitué par un groupe hydroxy ;
R4 et R5 représentent indépendamment l'un de l'autre un atome d'hydrogène ou un méthyle ou forment ensemble, avec l'atome d'azote qui les porte, une morpholine ;
R6 et R7 représentent chacun un groupe phényle substitué par un atome de chlore, fluor, brome, un groupe Me, OMe, CN, CF₃, OCF₃ en position para ;
R8 représente un atome d'hydrogène, un groupe (C₁-C₆)alkyle ;
Y représente un hydrogène, un halogène, un groupe (C₁-C₆)alcoxy ; un groupe (C₁-C₆)alkyle ;un cyano ; un groupe halo(C₁-C₆)alkyle ;
sous forme d'énantiomères ou de diastéréoisomères ou de mélanges de ces formes ;
à l'état de base ou de sel d'addition à un acide ou à une base.

4. Composé de formule (I) selon la revendication 1 **caractérisé en ce que** :
R' est un NR4R5, OR8 ;
R représente un méthyle ;
A et B, s'ils sont présents, représentent indépendamment l'un de l'autre, un -CH₂-R1 représente un atome d'hydrogène ;
R2 et R3 représentent indépendamment l'un de l'autre, un atome d'hydrogène, un groupe (C₁-C₆)alkyle ; le groupe (C₁-C₆)alkyle étant éventuellement substitué par un groupe hydroxy ;
R4 et R5 représentent indépendamment l'un de l'autre un atome d'hydrogène ou un méthyle ou forment ensemble, avec l'atome d'azote qui les porte, une morpholine ;
R6 et R7 représentent chacun un groupe phényle, éventuellement substitué par un ou plusieurs atomes ou groupes choisis parmi un halogène, un groupe (C₁-C₆)alkyle, halo(C₁-C₆)alkyle, (C₁-C₆)alcoxy, halo(C₁-C₆)alcoxy ou cyano en position para ;
R8 représente un atome d'hydrogène, un groupe (C₁-C₆)alkyle ;
Y représente un atome d'hydrogène, de chlore, fluor, brome, un groupe Me, OMe, CN, CF₃,
sous forme d'énantiomères ou de diastéréoisomères ou de mélanges de ces formes ;
à l'état de base ou de sel d'addition à un acide ou à une base.

5. Composé de formule (I) selon la revendication 1 **caractérisé en ce que** :
R' est un NR4R5, OR8 ;
R représente un méthyle ;
A et B, s'ils sont présents, représentent indépendamment l'un de l'autre, un -CH₂- ;
R1 représente un atome d'hydrogène ;
R2 et R3 représentent indépendamment l'un de l'autre, un atome d'hydrogène, un groupe (C₁-C₆)alkyle ; le groupe (C₁-C₆)alkyle étant éventuellement substitué par un groupe hydroxy ;
R4 et R5 représentent indépendamment l'un de l'autre un atome d'hydrogène ou un méthyle ou forment ensemble, avec l'atome d'azote qui les porte, une morpholine ;
R6 et R7 représentent chacun un groupe phényle substitué par un atome de chlore, fluor, brome, un groupe Me, OMe, CN, CF₃, OCF₃ en position para ;
R8 représente un atome d'hydrogène, un groupe (C₁-C₆)alkyle ;
Y représente un atome d'hydrogène, de chlore, fluor, brome, un groupe Me, OMe, CN, CF₃,
sous forme d'énantiomères ou de diastéréoisomères ou de mélanges de ces formes ;
à l'état de base ou de sel d'addition à un acide ou à une base.

6. Composé de formule (I) selon la revendication 1 choisi parmi :
3-({1-[bis-(4-chloro-phényl)-méthyl]-azétidin-3-yl}-méthanesulfonyl-amino)-*N*-((S)-1-carbamoyl-éthyl)-benzamide
3-({1-[bis-(4-chloro-phényl)-méthyl]-azétidin-3-yl}-méthanesulfonyl-amino)-*N-*carbamoylméthyl-5-fluoro-benzamide
3-({1-[bis-(4-chloro-phényl)-méthyl]-azétidin-3-yl}-méthanesulfonyl-amino)-*N*-((S)-1-carbamoyl-éthyl)-5-fluoro-benzamide,
3-({1-[bis-(4-chloro-phényl)-méthyl]-azétidin-3-yl}-méthanesulfonyl-amino)-*N*-((R)-1-carbamoyl-éthyl)-5-fluoro-benzainide
3-({1-[bis-(4-chloro-phényl)-méthyl]-azétidin-3-yl}-méthanesulfonyl-amino-*N*-((R)-1-carbamoyl-éthyl)-benzamide
3-({1-[bis-(4-chloro-phényl)-méthyl]-azétidin-3-yl}-méthanesulfonyl-amino)-*N*-((S)-1-carbamoyl-2-hydroxy-éthyl)-benzamide
3-({1-[bis-(4-chloro-phényl)-méthyl]-azétidin-3-yl}-méthanesulfonyl-amino)-*N*-(2-carbamoyl-éthyl)-benzamide
3-({1-[bis-(4-chloro-phényl)-méthyl]-azétidin-3-yl}-méthanesulfonyl-amino)-*N*-(2-morpholin-4-yl-2-oxo-éthyl)-benzamide
3-({1-[bis-(4-chloro-phényl)-méthyl]-azétidin-3-yl}-méthanesulfonyl-amino)-*N*-((S)-1-méthylcarbamoyl-éthyl)-benzamide
3-({1-[bis-(4-chloro-phényl)-méthyl]-azétidin-3-yl}méthanesulfonyl-amino)-*N*-((S)-1-carbamoyl-propyl)-benzamide
3-({1-[bis-(4-chloro-phényl)-méthyl]-azétidin-3yl}-méthanesulfonyl-amino)-*N*-((S)-1-carbamoyl-3-méthyl-butyl)-benzamide
3-({1-[bis-(4-chloro-phényl)-méthyl]-azétidin-3-yl}-méthanesulfonyl-amino)-*N*-((S)-1-carbamoyl-2-méthyl-propyl)-5-fluoro-benzamide
3-({1-[bis-(4-chloro-phényl)-méthyl]-azétidin-3-yl}-méthanesulfonyl-amino)-*N*-((S)-1-carbamoyl-2-hydroxy-éthyl)-5-fluoro-benzamide
3-({1-[bis-(4-chloro-phényl)-méthyl]-azétidin-3-yl}-méthanesulfonyl-amino)-*N*-((S)-1-carbamoyl-propyl)-5-fluoro-benzamide
3-({1-[bis-(4-chloro-phényl)-méthyl]-azétidin-3-yl)-méthanesulfonyl-amino)-*N-*(méthylcarbamoyl-méthyl)-benzamide
3-({1-[bis-(4-chloro-phényl)-méthyl]-azétidin-3-yl}-méthanesulfonyl-amino)-*N*-(carbamoyl-méthyl)-benzamide
Ester tert-butylique de l'acide (S)-2-[3-({1-[bis-(4-chloro-phényl)-méthyl]-azétidin-3-yl}-méthanesulfonyl-amino)-benzoylamino]-propionique
Acide (S)-2-[3-({1-[bis-(4-chloro-phényl)-méthyl]-azétidin-3-yl}-méthanesulfonyl-amino)-benzoylamino]-propionique
Ester tert-butylique de l'acide 3-({1-[bis-(4-chloro-phényl)-méthyl]-azétidin-3-yl}-méthanesulfonyl-amino)-benzoylamino-acétique
Ester tert-butylique de l'acide 4-[3-({1-[bis-(4-chloro-phényl)-méthyl]-azétidin-3-yl}-méthanesulfonyl-amino)-5-fluoro-benzoylamino]-butyrique
Ester tert-butylique de l'acide (S)-2-[3-({1-[bis-(4-chloro-phényl)-méthyl]-azétidin-3-yl}-méthanesulfonyl-amino)-5-fluoro-benzoylamino]-propionique
3-({1-[bis-(4-chloro-phényl)-méthyl]-azétidin-3-yl}-méthanesulfonyl-amino)-*N*-((S)-1-carbamoyl-éthyl)-5-méthoxy-benzamide
3-({1-[bis-(4-chloro-phényl)-méthyl]-azétidin-3-yl}-méthanesulfonyl-amino)-*N*-((S)-1-diméthylcarbamoyl-éthyl)-benzamide
Ester tert-butylique de l'acide 4-[3-({1-[bis-(4-chloro-phényl)-méthyl]-azétidin-3-yl}-méthanesulfonyl-amino)-benzoylamino]-butyrique
Acide (S)-2-[3-({1-[bis-(4-chloro-phényl)-méthyl]-azétidin-3-yl}-méthanesulfonyl-amino)-5-fluoro-benzoylamino]-propionique et son sel de sodium
3-({1-[bis-(4-chloro-phényl)-méthyl]-azétidin-3-yl}-méthanesulfonyl-amino)-5-fluoro-*N*-((S)-1-méthylcarbamoyl-éthyl)-benzamide
3-({1-[bis-(4-chloro-phényl)-méthyl]-azétidin-3-yl}-méthanesulfonyl-amino)-N ((1S,2R)-1-carbamoyl-2-hydroxy-propyl)-benzamide
3-({1-[bis-(4-chloro-phényl)-méthyl]-azétidin-3-yl}-méthanesulfonyl-amino)-*N*-(3-carbamoyl-propyl)-5-fluoro-benzamide
Acide [3-({1-[bis-(4-chloro-phényl)-méthyl]-azétidin-3-yl}-méthanesulfonyl-amino)-benzoylamino]-acétique
3-({1-[Bis-(4-chloro-phenyl)-methyl]-azetidin-3-yl}-methanesulfonyl-amino)-*N*-((S)-1-carbamoyl-ethyl)-2-fluoro-benzamide
3-({1-[Bis-(4-chloro-phenyl)-methyl]-azetidin-3-yl}-methanesulfonyl-amino)-*N*-((S)-1-methylcarbamoyl-ethyl)-2-fluoro-benzamide
3-({1-[Bis-(4-chloro-phenyl)-methyl]-azetidin-3-yl}-methanesulfonyl-amino)-*N*-((S)-1-carbamoyl-ethyl)-6-fluoro-benzamide
3-({1-[Bis-(4-chloro-phenyl)-methyl]-azetidin-3-yl}-methanesulfonyl-amino)-*N*-((S)-1-carbamoyl-propyl)-6-fluoro-benzamide
3-({1-[Bis-(4-chloro-phenyl)-methyl]-azetidin-3-yl}-methanesulfonyl-amino)-N-((S)-1-carbamoyl-2-methyl-propyl)-6-fluoro-benzamide
3-({1-[Bis-(4-chloro-phenyl)-methyl]-azetidin-3-yl}-methanesulfonyl-amino)-*N*-((S)-1-methylcarbamoyl-ethyl)-6-fluoro-benzamide
3-({1-[Bis-(4-chloro-phenyl)-methyl]-azetidin-3-yl}-methanesulfonyl-amino)-*N*-((S)-1-carbamoyl-propyl)-4-fluoro-benzamide
3-({1-[Bis-(4-chloro-phenyl)-methyl]-azetidin-3-yl}-methanesulfonyl-amino)-*N-*carbamoylmethyl-5-méthyl-benzamide
3-({1-[Bis-(4-chloro-phenyl)-methyl]-azetidin-3-yl}-methanesulfonyl-amino)-*N*-((S)-1-carbamoyl-ethyl)-5-méthyl-benzamide
3-({1-[Bis-(4-chloro-phenyl)-methyl]-azetidin-3-yl}-methanesulfonyl-amino)-*N*-((S)-1-carbamoyl-propyl)-5-méthyl-benzamide
3-({1-[Bis-(4-chloro-phenyl)-methyl]-azetidin-3-yl}-methanesulfonyl-amino)-*N*-((S)-1-carbamoyl-2-methyl-propyl)-5-méthyl-benzamide
3-({1-[Bis-(4-chloro-phenyl)-methyl]-azetidin-3-yl}-methanesulfonyl-amino)-*N*-((S)-1-methylcarbamoyl-ethyl)-5-méthyl-benzamide
3-({1-[Bis-(4-chloro-phenyl)-methyl]-azetidin-3-yl}-methanesulfonyl-amino)-*N*-((S)-1-carbamoyl-2-hydroxy-ethyl)-5-méthyl-benzamide
3-({1-[Bis-(4-chloro-phenyl)-methyl]-azetidin-3-yl}-methanesulfonyl-amino)-*N*-((S)-1-carbamoyl-ethyl)-5-chloro-benzamide
3-({1-[Bis-(4-chloro-phenyl)-methyl]-azetidin-3-yl}-methanesulfonyl-amino)-*N*-((S)-1-carbamoyl-propyl)-5-chloro-benzamide
3-({1-[Bis-(4-chloro-phenyl)-methyl]-azetidin-3-yl}-methanesulfonyl-amino)-*N*-((S)-1-carbamoyl-2-methyl-propyl)-5-chloro-benzamide
3-({1-[Bis-(4-chloro-phenyl)-methyl]-azetidin-3-yl}-methanesulfonyl-amino)-*N*-((S)-1-methylcarbamoyl-ethyl)-5-chloro-benzamide
3-({1-[Bis-(4-chloro-phenyl)-methyl]-azetidin-3-yl}-methanesulfonyl-amino)-*N*-((S)-1-carbamoyl-2-hydroxy-ethyl)-5-chloro-benzamide
3-({1-[Bis-(4-chloro-phenyl)-methyl]-azetidin-3-yl}-methanesulfonyl-amino)-*N*-((S)-1-carbamoyl-ethyl)-5-bromo-benzamide
3-({1-[Bis-(4-chloro-phenyl)-methyl]-azetidin-3-yl}-methanesulfonyl-amino)-*N*-((S)-1-carbamoyl-propyl)-5-bromo-benzamide
3-({1-[Bis-(4-chloro-phenyl)-methyl]-azetidin-3-yl}-methanesulfonyl-amino)-*N*-((S)-1-carbamoyl-2-methyl-propyl)-5-bromo-benzamide
3-({1-[Bis-(4-chloro-phenyl)-methyl]-azetidin-3-yl}-methanesulfonyl-amino)-*N*-((S)-1-methylcarbamoyl-ethyl)-5-bromo-benzamide
3-({1-[Bis-(4-chloro-phenyl)-methyl]-azetidin-3-yl}-methanesulfonyl-amino)-*N*-((S)-1-carbamoyl-2-hydroxy-ethyl)-5-bromo-benzamide
3-({1-[Bis-(4-chloro-phenyl)-methyl]-azetidin-3-yl}-methanesulfonyl-amino)-*N-*carbamoylmethyl-5-trifluorométhyl-benzamide
3-({1-[Bis-(4-chloro-phenyl)-methyl]-azetidin-3-yl}-methanesulfonyl-amino)-*N*-((S)-1-carbamoyl-ethyl)-5-trifluorométhyl-benzamide
3-({1-[Bis-(4-chloro-phenyl)-methyl]-azetidin-3-yl}-methanesulfonyl-amino)-*N*-((S)-1-carbamoyl-propyl)-5-trifluorométhyl-benzamide
3-({1-[Bis-(4-chloro-phenyl)-methyl]-azetidin-3-yl}-methanesulfonyl-amino)-N-((S)-1-carbamoyl-2-methyl-propyl)-5-trifluorométhyl-benzamide
3-({1-[Bis-(4-chloro-phenyl)-methyl]-azetidin-3-yl}-methanesulfonyl-amino)-*N*-((S)-1-methylcarbamoyl-ethyl)-5-trifluorométhyl-benzamide
3-({1-[Bis-(4-chloro-phenyl)-methyl]-azetidin-3-yl}-methanesulfonyl-amino)-*N*-((S)-1-carbamoyl-2-hydroxy-ethyl)-5-trifluorométhyl-benzamide
3-({1-[Bis-(4-chloro-phenyl)-methyl]-azetidin-3-yl}-methanesulfonyl-amino)-*N*-((S)-1-carbamoyl-ethyl)-5-cyano-benzamide
3-({1-[Bis-(4-chloro-phenyl)-methyl]-azetidin-3-yl}-methanesulfonyl-amino)-*N*-((S)-1-carbamoyl-propyl)-5-cyano-benzamide
3-({1-[Bis-(4-chloro-phenyl)-methyl]-azetidin-3-yl}-methanesulfonyl-amino)-*N*-((S)-1-carbamoyl-2-methyl-propyl)-5-cyano-benzamide
3-({1-[Bis-(4-chloro-phenyl)-methyl]-azetidin-3-yl}-methanesulfonyl-amino)-*N*-((S)-1-methylcarbamoyl-ethyl)-5-cyano-benzamide
3-({1-[Bis-(4-ehloro-phenyl)-methyl]-azetidin-3-yl}-methanesulfonyl-amino)-N-((S)-1-carbamoyl-2-hydroxy-ethyl)-5-cyano-benzamide
Acide (S)-2-[3-({1-[bis-(4-chloro-phényl)-méthyl]-azétidin-3-yl}-méthanesulfonyl-amino)-6-fluoro-benzoylamino]-propionique et son sel de l'acide trifluoroacétique.
Acide (S)-2-[3-({1-[bis-(4-chloro-phényl)-méthyl]-azétidin-3-yl}-méthanesulfonyl-amino)-5-méthyl-benzoylamino]-propiomque et son sel de l'acide trifluoroacétique.
Acide (S)-2-[3-({1-[bis-(4-chloro-phényl)-méthyl]-azétidin-3-yl}-méthanesulfonyl-amino)-5-chloro-benzoylamino]-propionique et son sel de l'acide trifluoroacétique.
Acide (S)-2-[3-({1-[bis-(4-chloro-phényl)-méthyl]-azétidin-3-yl}-méthanesulfonyl-amino)-5-bromo-benzoylamino]-propionique et son sel de l'acide trifluoroacétique
Acide (S)-2-[3-({1-[bis-(4-chloro-phényl)méthyl]-azétidin-3-yl}-méthanesulfonyl-amino)-3-trifluorométhyl-benzoylamino]-propionique et son sel de l'acide trifluoroacétique
Acide (S)-2-[3-({1-[bis-(4-chloro-phényl)-méthyl]-azétidin-3-yl}-méthanesulfonyl-amino)-5-cyano-benzoylamino]-propionique et son sel de l'acide trifluoroacétique
3-({1-[Bis-(4-bromo-phenyl)-methyl]-azetidin-3-yl}-methanesulfonyl-amino)-*N*-((S)-1-carbamoyl-ethyl)-5-fluoro-benzamide
3-({1-[Bis-(4-trifluoromethyl-phenyl)-methyl]-azetidin-3-yl}-methanesulfonyl-amino)-*N*-((S)-1-carbamoyl-ethyl)-5-fluoro-benzamide
3-({1-[Bis-(4-cyano-phenyl)-methyl]-azetidin-3-yl}-methanesulfonyl-amino)-*N*-((S)-1-carbamoyl-ethyl)-5-fluoro-benzamide
3-({1-[Bis-(4-methoxy-phenyl)-methyl]-azetidin-3-yl}-methanesulfonyl-amino)-*N*-((S)-1-carbamoyl-ethyl)-5-fluoro-benzamide
3-({1-[Bis-(4-fluoro-phenyl)-methyl]-azetidin-3-yl}-methanesulfonyl-amino)-*N*-((S)-1-carbamoyl-ethyl)-5-fluoro-benzamide
Ester methylique de l'acide (S)-2-[3-({1-[Bis-(4-chloro-phenyl)-methyl]-azetidin-3-yl}-methanesulfonyl-amino)-5-fluoro-benzoylamino]-propionique
3-({1-[Bis-(4-methyl-phenyl)methyl]-azetidin-3-yl}-methanesulfonyl-amino)-*N*-((S)-1-carbamoyl-propyl)-5-fluoro-benzamide
3-({1-[Bis-(4-bromo-phenyl)-methyl]-azetidin-3-yl}-methanesulfonyl-amino)-*N*-((S)-1-methylcarbamoyl-ethyl)-5-fluoro-benzamide
Acide (S)-2-[3-({1-[Bis-(4-bromo-phenyl)-methyl]-azetidin-3-yl}-methanesulfonyl-amino)-5-fluoro-benzoylamino]-propionique
Ester éthylique de l'acide (S)-2-[3-({1-[Bis-(4-chloro-phenyl)-methyl]-azetidin-3-yl}-methanesulfonyl-amino)-5-fluoro-benzoylamino]-propionique 3-({1-[Bis-(4-trifluoromethoxy-phenyl)-methyl]-azetidin-3-yl}-methanesulfonyl-amino)-*N*-((S)-1-carbamoyl-propyl)-5-fluoro-benzamide
leurs isomères optiques et leurs sels pharmaceutiquement acceptables.

7. Médicament **caractérisé en ce qu'**il comprend un composé de formule (I) tel que défini dans les revendications 1 à 6.

8. Composition pharmaceutique **caractérisée en ce qu'**elle comprend un composé de formule (I) tel que défini dans les revendications 1 à 6.

9. Utilisation d'un composé de formule (I) tel que défini dans les revendications 1 à 6 pour la préparation d'un médicament pour le traitement ou la prévention des désordres psychiatriques, de la dépendance et du sevrage à une substance, du sevrage tabagique, des troubles cognitifs et de l'attention et des maladies neurodégénératives aiguës et chroniques.

10. Utilisation d'un composé de formule (I) tel que défini dans les revendications 1 à 6 pour la préparation d'un médicament pour le traitement ou la prévention des troubles du métabolisme, des troubles de l'appétence, des troubles de l'appétit, de l'obésité, du diabète, du syndrome métabolique, de la dyslipidémie, de l'apnée du sommeil.

11. Utilisation d'un composé de formule (I) tel que défini dans les revendications 1 à 6 pour la préparation d'un médicament pour le traitement ou la prévention de la douleur, de la douleur neuropathique, des douleurs neuropathiques induites par les anticancéreux.

12. Utilisation d'un composé de formule (I) tel que défini dans les revendications 1 à 6 pour la préparation d'un médicament pour le traitement ou la prévention des troubles gastro-intestinaux, des vomissements, de l'ulcère, des troubles diarrhéiques, des troubles vésicaux et urinaires, des troubles d'origine endocrinienne, des troubles cardio-vasculaires, de l'hypotension, du choc hémorragique, du choc septique, des maladies du foie, de la cirrhose chronique du foie, des fibroses, de la stéatohépatite non alcoolique (NASH), de la stéatohépatite et de la stéatose hépatique, quelle que soit l'étiologie de ces affections (alcool, médicament, produit chimique, maladie auto-immune, obésité, diabète, maladie métabolique congénitale).

13. Utilisation d'un composé de formule (I) tel que défini dans les revendications 1 à 6 pour la préparation d'un médicament pour le traitement ou la prévention des maladies du système immunitaire, de l'arthrite rhumatoïde, de la démyélinisation, de la sclérose en plaques, des maladies inflammatoires.

14. Utilisation d'un composé de formule (I) tel que défini dans les revendications 1 à 6 pour la préparation d'un médicament pour le traitement ou la prévention de la maladie d'Alzheimer, de Parkinson, de la schizophrénie, des troubles cognitifs associés à la schizophrénie, au diabète, à l'obésité, au syndrome métabolique.

15. Utilisation d'un composé de formule (I) tel que défini dans les revendications 1 à 6 pour la préparation d'un médicament pour le traitement ou la prévention de l'asthme, des maladies pulmonaires chronique obstructive, du syndrome de Raynaud, du glaucome, des troubles de la fertilité.

16. Utilisation d'un composé de formule (I) tel que défini dans les revendications 1 à 6 pour la préparation d'un médicament pour le traitement ou la prévention des maladies infectieuses et virales telles que les encéphalites, des accidents vasculaires cérébraux, du syndrome de Guillain-Barré, de l'ostéoporose et l'apnée du sommeil et pour la chimiothérapie anticancéreuse.

17. Procédé de préparation de composés de formule (I) pour lesquels R' est NR4R5 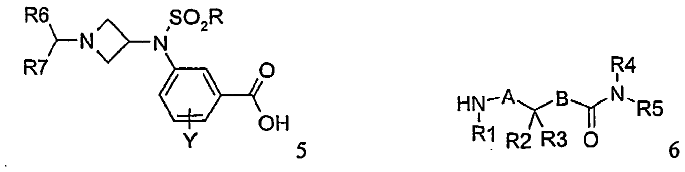 un dérivé acide 5 et un dérivé aminé 6 sont mis en réaction dans un solvant inerte ; en présence d'un agent de couplage et éventuellement d'un additif évitant la racémisation, on isole le produit et le transforme éventuellement en sel d'addition à un acide.

18. Procédé de préparation de composés de formule (I) pour lesquels R' est OR8 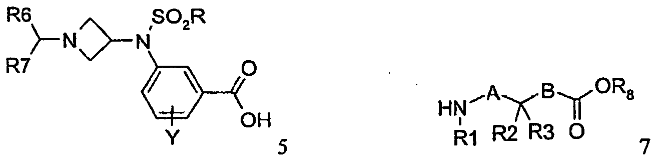 un dérivé acide 5 et un dérivé aminé 7 sont mis en réaction dans un solvant inerte ; en présence d'un agent de couplage et éventuellement d'un additif évitant la racémisation, on isole le produit et le transforme éventuellement en sel d'addition à un acide.

19. Procédé de préparation de composés de formule (I) pour lesquels R' est OR8 et R8 est H, par hydrolyse de la fonction OR8 où R8 est un groupe (C₁-C₆)alkyle, un groupe halo(C₁-C₆)alkyle, un groupe allyle, un groupe phényle(C₁-C₆)alkyle, le groupe phényle étant éventuellement substitué par 1 ou 2 groupes O-méthyle ; on isole le produit ainsi hydrolysé et on le transforme éventuellement en sel d'addition à une base.

20. Procédé de préparation de composés de formule (I) pour lesquels R' est NR4R5, à partir d'un composé de formule (I) pour lesquels R' est OR8 et R8 est H et un dérivé aminé HNR4R5 sont mis en réaction dans un solvant inerte ; en présence d'un agent de couplage et éventuellement d'un additif évitant la racémisation, on isole le produit et le transforme éventuellement en sel d'addition à un acide.

21. Procédé de préparation de composés de formule 5 pour lesquels Y, R, R6, R7 sont tels que définis dans la revendication 1 par hydrolyse de la fonction ester des composés de formule 4 pour lesquels Y, R, R6, R7 sont tels que définis dans la revendication 1 et R" est un groupe (C₁-C₆)alkyle, un groupe halo(C₁-C₆)alkyle, un groupe allyle, un groupe phényle(C₁-C₆)alkyle, le groupe phényle étant éventuellement substitué par 1 ou 2 groupes O-méthyle

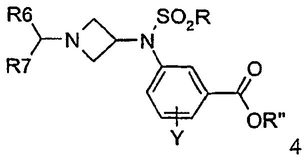

22. Intermédiaires de synthèse de composés de formule (I)
Ester méthylique de l'acide 3-({1-[bis-(4-chloro-phényl)-méthyl]-azétidin-3-yl}-méthanesulfonyl-amino)-5-fluoro-benzoïque
Ester méthylique de l'acide 3-({1-[bis-(4-fluoro-phényl)-méthyl]-azétidin-3-yl}-méthanesulfonyl-amino)-5-fluoro-benzoique
Acide 3-({1-[bis-(4-fluoro-phényl)-méthyl]-azétidin-3-yl}-méthanesulfonyl-amino)-5-fluoro-benzoïque
Ester éthylique de l'acide 3-({1-[bis-(4-bromo-phényl)-méthyl]-azétidin-3-yl}-méthanesulfonyl-amino)-5-fluoro-benzoïque
Acide 3-({1-[bis-(4-bromo-phényl)méthyl]-azétidin-3-yl}-méthanesulfonyl-amino)-5-fluoro-benzoïque
Ester éthylique de l'acide 3-({1-[bis-(4-trifluorométhyl-phényl)-méthyl]-azétidin-3-yl}-méthanesulfonyl-amino)-5-fluoro-benzoïque
Acide 3-({1-[bis-(4-trifluorométhyl-phényl)-méthyl]-azétidin-3-yl}-méthanesulfonyl-amino)-5-fluoro-benzoïque
Ester éthylique de l'acide 3-({1-[bis-(4-méthoxy-phényl)-méthyl]-azétidin-3-yl}-méthanesulfonyl-amino)-5-fluoro-benzoïque
Acide 3-({1-[bis-(4-méthoxy-phényl)-méthyl]-azétidin-3-yl}-méthanesulfonyl-amino)-5-fluoro-benzoïque
Ester méthylique de l'acide 3-({1-[bis-(4-méthyl-phényl)-méthyl]-azétidin-3-yl}-méthanesulfonyl-amino)-5-fluoro-benzoïque
Acide 3-({1-[bis-(4-méthyl-phényl)-méthyl]-azétidin-3-yl}-méthanesulfonyl-amino)-5-fluoro-benzoïque
Ester éthylique de l'acide 3-({1-[bis-(4-cyano-phényl-méthyl]-azétidin-3-yl}-méthanesulfonyl-amino)-5-fluoro-benzoïque
Acide 3-({1-[bis-(4-cyano-phényl)-méthyl]-azétidin-3-yl}-méthanesulfonyl-amino)-5-fluoro-benzoïque
Ester méthylique de l'acide 3-({1-[bis-(4-trifluorométhoxy-phényl)-méthyl]-azétidin-3-yl}-méthanesulfonyl-amino)-5-fluoro-benzoïque
Acide 3-({1-[bis-(4-trifluorométhoxy-phényl)-méthyl]-azétidin-3-yl}-méthanesulfonyl-amino)-5-fluoro-benzoïque
Ester méthylique de l'acide 3-({1-[bis-(4-chloro-phényl)-méthyl]-azétidin-3-yl}-méthanesulfonyl-amino)-2-fluoro-benzoïque
Acide 3-({1-[bis-(4-chloro-phényl)-méthyl]-azétidin-3-yl}-méthanesulfonyl-amino)-2-fluoro-benzoïque
Ester éthylique de l'acide 3-({1-[bis-(4-chloro-phényl)-méthyl]-azétidin-3-yl}-méthanesulfonyl-amino)-4-fluoro-benzoïque
Acide 3-({1-[bis-(4-chloro-phényl)-méthyl]-azétidin-3-yl}-méthanesulfonyl-amino)-4-fluoro-benzoïque
Ester méthylique de l'acide 3-({1-[bis-(4-chloro-phényl)-méthyl]-azétidin-3-yl}-méthanesulfonyl-amino)-6-fluoro-benzoïque
Acide 3-({1-[bis-(4-chloro-phényl)-méthyl]-azétidin-3-yl}-méthanesulfonyl-amino)-6-fluoro-benzoïque
Ester méthylique de l'acide 3-({1-[bis-(4-chloro-phényl)-méthyl]-azétidin-3-yl}-méthanesulfonyl-amino)-5-méthoxy-benzoïque
Acide 3-({1-[bis-(4-ehloro-phényl)-méthyl]-azétidin-3-yl}-méthanesulfonyl-amino)-5-méthoxy-benzoïque
Ester Allylique de l'acide 3-({1-[bis-(4-chloro-phényl)-méthyl]-azétidin-3-yl}-méthanesulfonyl-amino)-5-méthyl-benzoïque
Acide 3-({1-[bis-(4-chloro-phényl)méthyl]-azétidin-3-yl}-méthanesulfonyl-amino)-5-méthyl-benzoïque
Ester méthylique de l'acide 3-({1-(bis-(4-chloro-phényl)-méthyl]-azétidin-3-yl}-méthanesulfonyl-amino)-5-chloro-benzoïque
Acide 3-({1-[bis-(4-chloro-phényl)-méthyl]-azétidin-3-yl}-méthanesulfonyl-amino)-5-chloro-benzoïque
Ester méthylique de l'acide 3-({1-[bis-(4-chloro-phényl)-méthyl]-azétidin-3-yl}-méthanesulfonyl-amino)-5-bromo-benzoïque
Acide 3-({1-[bis-(4-chloro-phényl)-méthyl]-azétidin-3-yl}-méthanesulfonyl-amino)-5-bromo-benzoïque
Ester méthylique de l'acide 3-({1-[bis-(4-chloro-phényl)-méthyl]-azétidin-3-yl}-méthanesulfonyl-amino)-5-trifluorométhyl-benzoïque
Acide 3-({1-[bis-(4-chloro-phényl)-méthyl]-azétidin-3-yl}-méthanesulfonyl-amino)-5-trifluorométhyl-benzoïque
Ester méthylique de l'acide 3-({1-[bis-(4-chloro-phényl)-méthyl]-azétidin-3-yl}-méthanesulfonyl-amino)-5-cyano-benzoïque
Acide 3-({1-[bis-(4-chloro-phényl)-méthyl]-azétidin-3-yl}-méthanesulfonyl-amino)-5-cyano-benzoïque

## Patentansprüche

1. Verbindung der Formel (I) 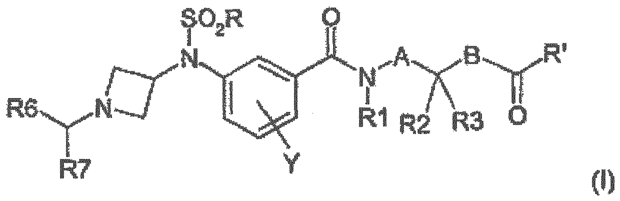 worin:
R für eine (C₁-C₆) Alkyl- oder Halogen(C₁-C₆)alkyl gruppe steht;
R' für eine NR4R5- oder OR8-Gruppe steht;
A und B, wenn sie vorhanden sind, unabhängig voneinander für ein oder zwei Kohlenstoffatome stehen, wobei diese Kohlenstoffatome durch ein oder mehrere wasserstoffatome oder (C₁-C₆)-Alkylgruppen substituiert sind; wobei die (C₁-C₆)-Alkylgruppe bzw. die (C₁-C₆)-Alkylgruppen gegebenenfalls durch eine oder mehrere Hydroxy-, (C₁-C₆)Alkoxy-, Halogen (C₁-C₆)alkyl-, (C₁-C₆) AlkylS(O)ₚ-, NR4R5- oder CONR4R5-Gruppen substituiert ist bzw. sind;
A + B für maximal zwei Kohlenstoffatome stehen;
R1 für ein Wasserstoffatom oder eine (C₁-C₆)-Alkylgruppe steht;
R2 und R3 unabhängig voneinander für ein Wasserstoffatom oder eine (C₁-C₆)-Alkylgruppe stehen;
wobei die (C₁-C₆)-Alkylgruppe gegebenenfalls durch eine oder mehrere Hydroxy-, (C₁-C₆)Alkoxy-, Halogen(C₁-C₆)alkyl-, (C₁-C₆)AlkylS(O)ₚ-, NR4R5- oder CONR4R5-Gruppen substituiert ist;
R4 und R5 unabhängig voneinander für ein Wasserstoffatom oder eine (C₁-C₆)-Alkylgruppe stehen oder zusammen mit dem Stickstoffatom, an das sie gebunden sind, einen Heterocyclus vom Azetidin-, Pyrrolidin-, Piperidin-, Azepan-, Piperazin-, Homopiperazin-, Morpholin-, Thiomorpholin-, Thiomorpholin-S-oxid- oder Thiomorpholin-S-dioxid-Typ bilden, wobei dieser Heterocyclus gegebenenfalls durch eine (C₁-C₆)Alkylgruppe substituiert ist;
R6 und R7 jeweils für eine Phenylgruppe stehen, die gegebenenfalls durch ein oder mehrere Atome bzw. eine oder mehrere Gruppen, die unter einem Wasserstoffatom und einem Halogenatom und einer (C₁-C₆₎Alkyl-, Halogen(C₁-C₆)alkyl-, (C₁-C₆)Alkoxy-, Halogen(C₁-C₆)alkoxy- oder Cyanogruppe ausgewählt sind, substituiert ist;
Y für ein Wasserstoffatom oder ein Halogenatom oder eine (C₁-C₆)Alkyl-, Halogen(C₁-C₆)alkyl-, (C₁-C₆)Alkoxy-, Halogen (C₁-C₆)alkoxy-, (C₁-C₆)-AlkylS(O)ₚ- oder Cyanogruppe steht;
R8 für ein Wasserstoffatom, eine (C₁-C₆)Alkylgruppe, eine Halogen(C₁-C₆)alkylgruppe, eine Allylgruppe oder eine Phenyl(C₁-C₆)alkylgruppe steht,
wobei die Phenylgruppe gegebenenfalls durch 1 oder 2 O-Methyl-Gruppen substituiert ist;
p für eine ganze zahl steht, die unter 0, 1 oder 2 ausgewählt ist;
in Basenform oder in Säure- oder Basenadditionssalzform.

2. Verbindung der Formel (I) nach Anspruch 1, **dadurch gekennzeichnet, dass**:
R' für ein NR4R5 oder OR8 steht;
R für ein Methyl steht;
A und B, wenn sie vorhanden sind, unabhängig voneinander für ein -CH₂- stehen;
R1 für ein Wasserstoffatom steht;
R2 und R3 unabhängig voneinander für ein Wasserstoffatom oder eine (C₁-C₆)-Alkylgruppe stehen; wobei die (C₁-C₆)-Alkylgruppe gegebenenfalls durch eine Hydroxygruppe substituiert ist;
R4 und R5 unabhängig voneinander für ein Wasserstoffatom oder ein Methyl stehen oder zusammen mit dem Stickstoffatom, an das sie gebunden sind, ein Morpholin bilden;
R6 und R7 jeweils für eine Phenylgruppe stehen, die gegebenenfalls durch ein oder mehrere Atome bzw. eine oder mehrere Gruppen, die unter einem Halogenatom und einer (C₁-C₆)Alkyl-, Halogen (C₁-C₆)alkyl-, (C₁-C₆)Alkoxy-, Halogen(C₁-C₆)alkoxy-oder Cyanogruppe ausgewählt sind, in para-Stellung substituiert ist;
R8 für ein Wasserstoffatom oder eine (C₁-C₆)Alkyl-gruppe steht;
Y für ein Wasserstoffatom oder ein Halogenatom oder eine (C₁-C₆)Alkoxygruppe; eine (C₁-C₆)Alkyl-gruppe, Cyano oder eine Halogen(C₁-C₆)alkylgruppe steht;
in Form von Enantiomeren oder Diastereomeren oder Gemischen dieser Formen;
in Basenform oder in Säure- oder Basenadditionssalzform.

3. Verbindung der Formel (I) nach Anspruch 1, **dadurch gekennzeichnet, dass**:
R' für ein NR4R5 oder OR8 steht;
R für ein Methyl steht;
A und B, wenn sie vorhanden sind, unabhängig voneinander für ein -CH₂- stehen;
R1 für ein Wasserstoffatom steht;
R2 und R3 unabhängig voneinander für ein Wasserstoffatom oder eine (C₁-C₆)-Alkylgruppe stehen;
wobei die (C₁-C₆)-Alkylgruppe gegebenenfalls durch eine Hydroxygruppe substituiert ist;
R4 und R5 unabhängig voneinander für ein Wasserstoffatom oder ein Methyl stehen oder zusammen mit dem Stickstoffatom, an das sie gebunden sind, ein Morpholin bilden;
R6 und R7 jeweils für eine Phenylgruppe stehen, die durch ein Chlor-, Fluor- oder Bromatom oder eine Me-, OMe-, CN-, CF₃- oder OCF₃-Gruppe in para-Stellung substituiert ist;
R8 für ein Wasserstoffatom oder eine (C₁-C₆)Alkyl-gruppe steht;
Y für ein Wasserstoffatom oder ein Halogenatom oder eine (C₁-C₆)Alkoxygruppe; eine (C₁-C₆)Alkyl-gruppe, Cyano oder eine Halogen(C₁-C₆)alkylgruppe steht;
in Form von Enantiomeren oder Diastereomeren oder Gemischen dieser Formen;
in Basenform oder in Säure- oder Basenadditionssalzform.

4. Verbindung der Formel (I) nach Anspruch 1, **dadurch gekennzeichnet, dass**:
R' für ein NR4R5 oder OR8 steht;
R für ein Methyl steht;
A und B, wenn sie vorhanden sind, unabhängig voneinander für ein -CH₂- stehen;
R1 für ein Wasserstoffatom steht;
R2 und R3 unabhängig voneinander für ein Wasserstoffatom oder eine (C₁-C₆)-Alkylgruppe stehen; wobei die (C₁-C₆)-Alkylgruppe gegebenenfalls durch eine Hydroxygruppe substituiert ist;
R4 und R5 unabhängig voneinander für ein Wasserstoffatom oder ein Methyl stehen oder zusammen mit dem Stickstoffatom, an das sie gebunden sind, ein Morpholin bilden;
R6 und R7 jeweils für eine Phenylgruppe stehen, die gegebenenfalls durch ein oder mehrere Atome bzw. eine oder mehrere Gruppen, die unter einem Halogenatom und einer (C₁-C₆)Alkyl-, Halogen(C₁-C₆)alkyl-, (C₁-C₆)Alkoxy-, Halogen(C₁-C₆)alkoxy-oder Cyanogruppe ausgewählt sind, in para-Stellung substituiert ist;
R8 für ein Wasserstoffatom oder eine (C₁-C₆)Alkyl-gruppe steht;
Y für ein Wasserstoff-, Chlor-, Fluor- oder Bromatom oder eine Me-, OMe-, CN- oder CF₃-Gruppe steht;
in Form von Enantiomeren oder Diastereomeren oder Gemischen dieser Formen;
in Basenform oder in Säure- oder Basenadditionssalzform.

5. Verbindung der Formel (I) nach Anspruch 1, **dadurch gekennzeichnet, dass**:
R' für ein NR4R5 oder OR8 steht;
R für ein Methyl steht;
A und B, wenn sie vorhanden sind, unabhängig voneinander für ein -CH₂- stehen;
R1 für ein Wasserstoffatom steht;
R2 und R3 unabhängig voneinander für ein Wasserstoffatom oder eine (C₁-C₆)-Alkylgruppe stehen; wobei die (C₁-C₆)-Alkylgruppe gegebenenfalls durch eine Hydroxygruppe substituiert ist;
R4 und R5 unabhängig voneinander für ein Wasserstoffatom oder ein Methyl stehen oder zusammen mit dem Stickstoffatom, an das sie gebunden sind, ein Morpholin bilden;
R6 und R7 jeweils für eine Phenylgruppe stehen, die durch ein Chlor-, Fluor- oder Bromatom oder eine Me-, OMe-, CN-, CF₃- oder OCF₃-Gruppe in para-Stellung substituiert ist;
R8 für ein Wasserstoffatom oder eine (C₁-C₆)Alkyl-gruppe steht;
Y für ein Wasserstoff-, Chlor-, Fluor- oder Bromatom oder eine Me-, OMe-, CN- oder CF₃-Gruppe steht;
in Form von Enantiomeren oder Diastereomeren oder Gemischen dieser Formen;
in Basenform oder in Säure- oder Basenadditionssalzform.

6. Verbindung der Formel (I) nach Anspruch 1, ausgewählt unter:
3-({1-[Bis(4-chlorphenyl)methyl]azetidin-3-yl}-methansulfonylamino)-*N*-((*S*)-1-carbamoylethyl)-benzamid
3-({1-[Bis(4-chlorphenyl)methyl]azetidin-3-yl}-methansulfonylamino)-*N*-carbamoylmethyl-5-fluorbenzamid
3-({1-[Bis(4-chlorphenyl)methyl]azetidin-3-yl}-methansulfonylamino)-*N*-((S)-1-carbamoylethyl)-5-fluorbenzamid
3-({1-[Bis(4-chlorphenyl)methyl]azetidin-3-yl}-methansulfonylamino)-*N*-((R)-1-carbamoylethyl)-5-fluorbenzamid
3-({1-[Bis(4-chlorphenyl)methyl]azetidin-3-yl}-methansulfonylamino)-*N*-((R)-1-carbamoylethyl)benz-amid
3-({1-[Bis(4-chlorphenyl)methyl]azetidin-3-yl}-methansulfonylamino)-*N*-((*S*)-1-carbamoyl-2-hydroxy-ethyl)benzamid
3-({1-[Bis(4-chlorphenyl)methyl]azetidin-3-yl}-methansulfonylamino)-*N*-(2-carbamoylethyl)benzamid 3-({1-[Bis(4-chlorphenyl)methyl]azetidin-3-yl}-methansulfonylamino)-*N*-(2-morpholin-4-yl-2-oxo-ethyl)benzamid
3-({1-[Bis(4-chlorphenyl)methyl]azetidin-3-yl}-methansulfonylamino)-*N*-((*S*)-1-methylcarbamoyl-ethyl)benzamid
3-({1-[Bis(4-chlorphenyl)methyl]azetidin-3-yl}-methansulfonylamino)-*N*-((*S*)-1-carbamoylpropyl)-benzamid
3-({1-[Bis(4-chlorphenyl)methyl]azetidin-3-yl}-methansulfonylamino)-*N*-((*S*)-1-carbamoyl-3-methyl-butyl)benzamid
3-({1-[Bis(4-chlorphenyl)methyl]azetidin-3-yl}-methansulfonylamino)-*N*-((*S*)-1-carbamoyl-2-methyl-propyl)-5-fluorbenzamid
3-({1-[Bis(4-chlorphenyl)methyl]azetidin-3-yl}-methansulfonylamino)-*N*-((*S*)-1-carbamoyl-2-hydroxy-ethyl)-5-fluorbenzamid
3-({1-[Bis(4-chlorphenyl)methyl]azetidin-3-yl}-methansulfonylamino)-*N*-((*S*)-1-carbamoylpropyl)-5-fluorbenzamid
3-({1-[Bis(4-chlorphenyl)methyl]azetidin-3-yl}-methansulfonylamino)-*N*-(methylcarbamoylmethyl)-benzamid
3-({1-[Bis(4-chlorphenyl)methyl]azetidin-3-yl}-methansulfonylamino)-*N*-(carbamoylmethyl)benzamid (*S*)-2-[3-({1-[Bis(4-chlorphenyl)methyl]azetidin-3-yl}methansulfonylamino)benzoylamino]propionsäure-tert.-butylester
(*S*)-2-[3-({1-[Bis(4-chlorphenyl)methyl]azetidin-3-yl}methansulfonylamino)benzoylamino]propionsäure 3-({1-[Bis(4-chlorphenyl)methyl]azetidin-3-yl}-methansulfonylamino)benzoylaminoessigsäure-tert.-butylester
4-[3-({1-[Bis(4-chlorphenyl)methyl]azetidin-3-yl}-methansulfonylamino)-5-fluorbenzoylamino]butter-säure-tert.-butylester
(*S*)-2-[3-({1-[Bis(4-chlorphenyl)methyl]azetidin-3-yl}methansulfonylamino)-5-fluorbenzoylamino]-propionsäure-tert.-butylester
3-({1-[Bis(4-chlorphenyl)methyl]azetidin-3-yl}-methansulfonylamino)-*N*-((*S*)-1-carbamoylethyl)-5-methoxybenzamid
3-({1-[Bis(4-chlorphenyl)methyl]azetidin-3-yl}-methansulfonylamino)-N-((S)-1-dimethylcarbamoyl-ethyl)benzamid
4-[3-({1-[Bis(4-chlorphenyl)methyl]azetidin-3-yl}-methansulfonylamino)benzoylamino]buttersäure-tert.-butylester
(*S*)-2-[3-({1-[Bis(4-chlorphenyl)methyl]azetidin-3-yl}methansulfonylamino)-5-fluorbenzoylamino]-propionsäure und das Natriumsalz davon 3-({1-[Bis(4-chlorphenyl)methyl]azetidin-3-yl}-methansulfonylamino)-5-fluor-*N*-((*S*)-1-methyl-carbamoylethyl)benzamid
3-({1-[Bis(4-chlorphenyl)methyl]azetidin-3-yl}-methansulfonylamino)-*N*-((1*S*,2R)-1-carbamoyl-2-hydroxypropyl)benzamid
3-({1-[Bis(4-chlorphenyl)methyl]azetidin-3-yl}-methansulfonylamino)-N-(3-carbamoylpropyl)-5-fluorbenzamid
[3-({1-[Bis(4-chlorphenyl)methyl]azetidin-3-yl}-methansulfonylamino)benzoylamino]essigsäure 3-({1-[Bis(4-chlorphenyl)methyl]azetidin-3-yl}-methansulfonylamino)-*N*-((*S*)-1-carbamoylethyl)-2-fluorbenzamid
3-({1-[Bis(4-chlorphenyl)methyl]azetidin-3-yl}-methansulfonylamino)-*N*-((*S*)-1-methylcarbamoyl-ethyl)-2-fluorbenzamid
3-({1-[Bis(4-chlorphenyl)methyl]azetidin-3-yl}-methansulfonylamino)-*N*-((*S*)-1-carbamoylethyl)-6-fluorbenzamid
3-({1-[Bis(4-chlorphenyl)methyl]azetidin-3-yl}-methansulfonylamino)-*N*-((*S*)-1-carbamoylpropyl)-6-fluorbenzamid
3-({1-[Bis(4-chlorphenyl)methyl]azetidin-3-yl}-methansulfonylamino)-*N*-((*S*)-1-carbamoyl-2-methylpropyl)-6-fluorbenzamid
3-({1-[Bis(4-chlorphenyl)methyl]azetidin-3-yl}-methansulfonylamino)-*N*-((*S*)-1-methylcarbamoyl-ethyl)-6-fluorbenzamid
3-({1-[Bis(4-chlorphenyl)methyl]azetidin-3-y1}-methansulfonylamino)-*N*-((*S*)-1-carbamoylpropyl)-4-fluorbenzamid
3-({1-[Bis(4-chlorphenyl)methyl]azetidin-3-yl}-methansulfonylamino)-N-carbamoylmethyl-5-methyl-benzamid
3-({1-[Bis(4-chlorphenyl)methyl]azetidin-3-yl}-methansulfonylamino)-*N*-((*S*)-1-carbamoylethyl)-5-methylbenzamid
3-({1-[Bis(4-chlorphenyl)methyl]azetidin-3-yl}-methansulfonylamino)-*N*-((*S*)-1-carbamoylpropyl)-5-methylbenzamid
3-({1-[Bis(4-chlorphenyl)methyl]azetidin-3-yl}-methansulfonylamino)-*N*-((*S*)-1-carbamoyl-2-methyl-propyl)-5-methylbenzamid
3-({1-[Bis(4-chlorphenyl)methyl]azetidin-3-yl}-methansulfonylamino)-*N*-((*S*)-1-methylcarbamoyl-ethyl)-5-methylbenzamid
3-({1-[Bis(4-chlorphenyl)methyl]azetidin-3-yl}-methansulfonylamino)-*N*-((*S*)-1-carbamoyl-2-hydroxy- ethyl)-5-methylbenzamid
3-({1-[Bis(4-chlorphenyl)methyl]azetidin-3-yl}-methansulfonylamino)-*N*-((*S*)-1-carbamoylethyl)-5-chlorbenzamid
3-({1-[Bis(4-chlorphenyl)methyl]azetidin-3-yl}-methansulfonylamino)-*N*-((*S*)-1-carbamoylpropyl)-5-chlorbenzamid
3-({1-[Bis(4-chlorphenyl)methyl]azetidin-3-yl}-methansulfonylamino)-*N*-((*S*)-1-carbamoyl-2-methyl-propyl)-5-chlorbenzamid
3-({1-[Bis(4-chlorphenyl)methyl]azetidin-3-yl}-methansulfonylamino)-*N*-((*S*)-1-methylcarbamoyl-ethyl)-5-chlorbenzamid
3-({1-[Bis(4-chlorphenyl)methyl]azetidin-3-yl}-methansulfonylamino)-*N*-((*S*)-1-carbamoyl-2-hydroxyethyl)-5-chlorbenzamid
3-({1-[Bis(4-chlorphenyl)methyl]azetidin-3-yl}-methansulfonylamino)-*N*-((*S*)-1-carbamoylethyl)-5-brombenzamid
3-({1-[Bis(4-chlorphenyl)methyl]azetidin-3-yl}-methansulfonylamino)-*N*-((*S*)-1-carbamoylpropyl)-5-brombenzamid
3-({1-[Bis(4-chlorphenyl)methyl]azetidin-3-yl}-methansulfonylamino)-*N*-((*S*)-1-carbamoyl-2-methyl-propyl)-5-brombenzamid
3-({1-[Bis(4-chlorphenyl)methyl]azetidin-3-yl}-methansulfonylamino)-*N*-((*S*)-1-methylcarbamoyl-ethyl)-5-brombenzamid
3-({1-[Bis(4-chlorphenyl)methyl]azetidin-3-yl}-methansulfonylamino)-*N*-((*S*)-1-carbamoyl-2-hydroxyethyl)-5-brombenzamid
3-({1-[Bis(4-chlorphenyl)methyl]azetidin-3-yl}-methansulfonylamino)-*N*-carbamoylmethyl-5-trifluor-methylbenzamid
3-({1-[Bis(4-chlorphenyl)methyl]azetidin-3-yl}-methansulfonylamino)-*N*-((*S*)-1-carbamoylethyl)-5-trifluormethylbenzamid
3-({1-[Bis(4-chlorphenyl)methyl]azetidin-3-yl}-methansulfonylamino)-*N*-((*S*)-1-carbamoylpropyl)-5-trifluormethylbenzamid
3-({1-[Bis(4-chlorphenyl)methyl]azetidin-3-yl}-methansulfonylamino)-*N*-((*S*)-1-carbamoyl-2-methylpropyl)-5-trifluormethylbenzamid
3-({1-[Bis(4-chlorphenyl)methyl]azetidin-3-yl}-methansulfonylamino)-*N*-((*S*)-1-methylcarbamoyl-ethyl)-5-trifluormethylbenzamid
3-({1-[Bis(4-chlorphenyl)methyl]azetidin-3-yl}-methansulfonylamino)-*N*-((*S*)-1-carbamoyl-2-hydroxyethyl)-5-trifluormethylbenzamid
3-({1-[Bis(4-chlorphenyl)methyl]azetidin-3-yl}-methansulfonylamino)-*N*-((*S*)-1-carbamoylethyl)-5-cyanobenzamid
3-({1-[Bis(4-chlorphenyl)methyl]azetidin-3-yl}-methansulfonylamino)-*N*-((*S*)-1-carbamoylpropyl)-5-cyanobenzamid
3-({1-[Bis(4-chlorphenyl)methyl]azetidin-3-yl}-methansulfonylamino)-*N*-((*S*)-1-carbamoyl-2-methylpropyl)-5-cyanobenzamid
3-({1-[Bis(4-chlorphenyl)methyl]azetidin-3-yl}-methansulfonylamino)-*N*-((*S*)-1-methylcarbamoyl-ethyl)-5-cyanobenzamid
3-({1-[Bis(4-chlorphenyl)methyl]azetidin-3-yl}-methansulfonylamino)-*N*-((*S*)-1-carbamoyl-2-hydroxyethyl)-5-cyanobenzamid
(*S*)-2-[3-({1-[Bis(4-chlorphenyl)methyl]azetidin-3-yl}methansulfonylamino)-6-fluorbenzoylamino]-propionsäure und das Trifluoressigsäuresalz davon
(*S*)-2-[3-({1-[Bis(4-chlorphenyl)methyl]azetidin-3-yl}methansulfonylamino)-5-methylbenzoylamino]-propionsäure und das Trifluoressigsäuresalz davon
(*S*)-2-[3-({1-[Bis(4-chlorphenyl)methyl]azetidin-3-yl}methansulfonylamino)-5-chlorbenzoylamino]-propionsäure und das Trifluoressigsäuresalz davon
(*S*)-2-[3-({1-[Bis(4-chlorphenyl)methyl]azetidin-3-yl}methansulfonylamino)-5-brombenzoylamino]-propionsäure und das Trifluoressigsäuresalz davon
(*S*)-2-[3-({1-[Bis(4-chlorphenyl)methyl]azetidin-3-yl}methansulfonylamino)-3-trifluormethylbenzoyl-amino]propionsäure und das Trifluoressigsäuresalz davon
(*S*)-2-[3-({1-[Bis(4-chlorphenyl)methyl]azetidin-3-yl}methansulfonylamino)-5-cyanobenzoylamino]-propionsäure und das Trifluoressigsäuresalz davon
3-({1-[Bis(4-bromphenyl)methyl]azetidin-3-yl}-methansulfonylamino)-*N*-((*S*)-1-carbamoylethyl)-5-flüorbenzamid
3-({1-[Bis(4-trifluormethylphenyl)methyl]azetidin-3-yl}methansulfonylamino)-*N*-((*S*)-1-carbamoyl-ethyl)-5-fluorbenzamid
3-({1-[Bis(4-cyanophenyl)methyl]azetidin-3-yl}-methansulfonylamino)-*N*-((*S*)-1-carbamoylethyl)-5-fluorbenzamid
3-({1-[Bis(4-methoxyphenyl)methyl]azetidin-3-yl}-methansulfonylamino)-*N*-((*S*)-1-carbamoylethyl)-5-fluorbenzamid
3-({1-[Bis(4-fluorphenyl)methyl]azetidin-3-yl}-methansulfonylamino)-*N*-((*S*)-1-carbamoylethyl)-5-fluorbenzamid
(*S*)-2-[3-({1-[Bis(4-chlorphenyl)methyl]azetidin-3-yl}methansulfonylamino)-5-fluorbenzoylamino]-propionsäuremethylester
3-({1-[Bis(4-methylphenyl)methyl]azetidin-3-yl}-methansulfonylamino)-*N*-((*S*)-1-carbamoylpropyl)-5-fluorbenzamid
3-({1-[Bis(4-bromphenyl)methyl]azetidin-3-yl}-methansulfonylamino)-*N*-((*S*)-1-methylcarbamoyl-ethyl)-5-fluorbenzamid
(*S*)-2-[3-({1-[Bis(4-bromphenyl)methyl]azetidin-3-yl}methansulfonylamino)-5-fluorbenzoylamino]-propionsäure
(*S*)-2-[3-({1-[Bis(4-chlorphenyl)methyl]azetidin-3-yl}methansulfonylamino)-5-fluorbenzoylamino]-propionsäureethylester
3-({1-[Bis(4-trifluormethoxyphenyl)methyl]-azetidin-3-yl}methansulfonylamino)-*N*-((*S*)-1-carbamoylpropyl)-5-fluorbenzamid,
optische Isomere davon und pharmazeutisch unbedenkliche Salze davon.

7. Medikament, **dadurch gekennzeichnet, dass** es eine Verbindung der Formel (I) gemäß einem der Ansprüche 1 bis 6 umfasst.

8. Pharmazeutische Zusammensetzung, **dadurch gekennzeichnet, dass** sie eine Verbindung der Formel (I) gemäß einem der Ansprüche 1 bis 6 umfasst.

9. Verwendung einer Verbindung der Formel (I) gemäß einem der Ansprüche 1 bis 6 zur Herstellung eines Medikaments zur Behandlung oder Prävention von psychiatrischen Störungen, Substanzabhängigkeit und -entwöhnung, Tabakentwöhnung, kognitiven Störungen und Aufmerksamkeitsstörungen und akuten und chronischen neurodegenerativen Erkrankungen.

10. Verwendung einer Verbindung der Formel (I) gemäß einem der Ansprüche 1 bis 6 zur Herstellung eines Medikaments zur Behandlung oder Prävention von Stoffwechselstörungen, Appetenzstörungen, Appetitstörungen, Obesitas, Diabetes, metabolischem Syndrom, Dyslipidämie und Schlafapnoe.

11. Verwendung einer Verbindung der Formel (I) gemäß einem der Ansprüche 1 bis 6 zur Herstellung eines Medikaments zur Behandlung oder Prävention von Schmerzen, neuropathischen Schmerzen und durch Antikrebsbehandlung induzierten neuropathischen Schmerzen.

12. Verwendung einer Verbindung der Formel (I) gemäß einem der Ansprüche 1 bis 6 zur Herstellung eines Medikaments zur Behandlung oder Prävention von Magen-Darm-Störungen, Erbrechen, Geschwüren, Durchfallstörungen, Blasen- und Harnstörungen, Störungen endokrinen Ursprungs, Herz-Kreislauf-Störungen, Hypotonie, hämorrhagischem Schock, septischem Schock, Lebererkrankungen, chronischer Leberzirrhose, Fibrosen, nichtalkoholischer Steatohepatitis (NASH), Steatohepatitis und Lebersteatose, unabhängig von der Ätiologie dieser Leiden (Alkohol, Medikament, chemisches Produkt, Autoimmunerkrankung, Obesitas, Diabetes, kongenitale Stoffwechselerkrankung).

13. Verwendung einer Verbindung der Formel (I) gemäß einem der Ansprüche 1 bis 6 zur Herstellung eines Medikaments zur Behandlung oder Prävention von Erkrankungen des Immunsystems, rheumatoider Arthritis, Demyelinisierung, multipler Sklerose und entzündlichen Erkrankungen.

14. Verwendung einer Verbindung der Formel (I) gemäß einem der Ansprüche 1 bis 6 zur Herstellung eines Medikaments zur Behandlung oder Prävention von Alzheimer-Krankheit, Parkinson-Krankheit, Schizophrenie und mit Schizophrenie, Diabetes, Obesitas und metabolischem Syndrom assoziierten kognitiven Störungen.

15. Verwendung einer Verbindung der Formel (I) gemäß einem der Ansprüche 1 bis 6 zur Herstellung eines Medikaments zur Behandlung oder Prävention von Asthma, chronisch-obstruktiven Lungenerkrankungen, Raynaud-Syndrom, Glaukom und Fruchtbarkeitsstörungen.

16. Verwendung einer Verbindung der Formel (I) gemäß einem der Ansprüche 1 bis 6 zur Herstellung eines Medikaments zur Behandlung oder Prävention von Infektions- und Viruserkrankungen wie Enzephalitis, Schlaganfällen, Guillain-Barré-Syndrom, Osteoporose und Schlafapnoe und für die Antikrebschemotherapie.

17. Verfahren zur Herstellung von Verbindungen der Formel (I), für die R' für NR4R5 steht, 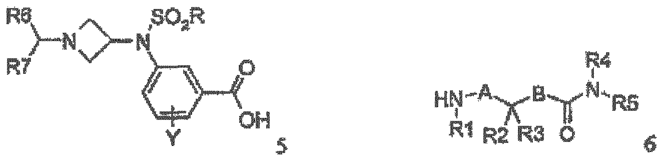 bei dem man ein Säurederivat 5 und ein Aminderivat 6 in einem inerten Lösungsmittel in Gegenwart eines Kupplungsmittels und gegebenenfalls eines die Racemisierung verhindernden Additivs umsetzt und das Produkt isoliert und gegebenenfalls in ein Säureadditionssalz umwandelt.

18. Verfahren zur Herstellung von Verbindungen der Formel (I), für die R' für OR8 steht, 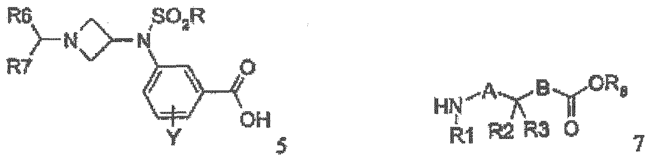 bei dem man ein Säurederivat 5 und ein Aminderivat 7 in einem inerten Lösungsmittel in Gegenwart eines Kupplungsmittels und gegebenenfalls eines die Racemisierung verhindernden Additivs umsetzt und das Produkt isoliert und gegebenenfalls in ein Säureadditionssalz umwandelt.

19. Verfahren zur Herstellung von Verbindungen der Formel (I), für die R' für OR8 steht und R8 für H steht, bei dem man die OR8-Funktion, wobei R8 für eine (C₁-C₆)Alkylgruppe, eine Halogen(C₁-C₆)alkyl-gruppe, eine Allylgruppe oder eine Phenyl(C₁-C₆)alkylgruppe steht, wobei die Phenylgruppe gegebenenfalls durch 1 oder 2 O-Methyl-Gruppen substituiert ist, hydrolysiert, das so hydrolysierte Produkt isoliert und gegebenenfalls in ein Basenadditionssalz umwandelt.

20. Verfahren zur Herstellung von Verbindungen der Formel (I), für die R' für NR4R5 steht, bei dem man eine Verbindung der Formel (I), für die R' für OR8 steht und R8 für H steht, und ein Aminderivat HNR4R5 in einem inerten Lösungsmittel in Gegenwart eines Kupplungsmittels und gegebenenfalls eines die Racemisierung verhindernden Additivs umsetzt und das Produkt isoliert und gegebenenfalls in ein Säureadditionssalz umwandelt.

21. Verfahren zur Herstellung von Verbindungen der Formel 5, für die Y, R, R6 und R7 die in Anspruch 1 angegebene Bedeutung besitzen, durch Hydrolyse der Esterfunktion der Verbindungen der Formel 4, für die Y, R, R6 und R7 die in Anspruch 1 angegebene Bedeutung besitzen und R'' für eine (C₁-C₆)Alkylgruppe, eine Halogen(C₁-C₆)alkylgruppe, eine Allylgruppe oder eine Phenyl(C₁-C₆)alkylgruppe steht, wobei die Phenylgruppe gegebenenfalls durch 1 oder 2 O-Methyl-Gruppen substituiert ist

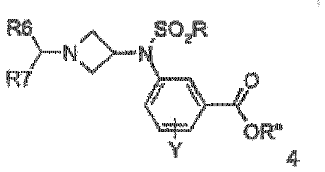

22. Zwischenprodukte der Synthese von Verbindungen der Formel (I)
3-({1-[Bis(4-chlorphenyl)methyl]azetidin-3-yl}-methansulfonylamino)-5-fluorbenzoesäuremethylester
3-({1-[Bis(4-fluorphenyl)methyl]azetidin-3-yl}-methansulfonylamino)-5-fluorbenzoesäuremethylester
3-({1-[Bis(4-fluorphenyl)methyl]azetidin-3-yl}-methansulfonylamino)-5-fluorbenzoesäure
3-({1-[Bis(4-bromphenyl)methyl]azetidin-3-yl}-methansulfonylamino)-5-fluorbenzoesäureethylester
3-({1-[Bis(4-bromphenyl)methyl]azetidin-3-yl}-methansulfonylamino)-5-fluorbenzoesäure
3-({1-[Bis(4-trifluormethylphenyl)methyl]azetidin-3-yl}methansulfonylamino)-5-fluorbenzoesäureethyl-ester
3-({1-[Bis(4-trifluormethylphenyl)methyl]azetidin-3-yl}methansulfonylamino)-5-fluorbenzoesäure
3-({1-[Bis(4-methoxyphenyl)methyl]azetidin-3-yl}-methansulfonylamino)-5-fluorbenzoesäureethylester
3-({1-[Bis(4-methoxyphenyl)methyl]azetidin-3-yl}-methansulfonylamino)-5-fluorbenzoesäure
3-({1-[Bis(4-methylphenyl)methyl]azetidin-3-yl}-methansulfonylamino)-5-fluorbenzoesäuremethylester
3-({1-[Bis(4-methylphenyl)methyl]azetidin-3-yl}-methansulfonylamino)-5-fluorbenzoesäure
3-({1-[Bis(4-cyanophenyl)methyl]azetidin-3-yl}-methansulfonylamino)-5-fluorbenzoesäureethylester
3-({1-[Bis(4-cyanophenyl)methyl]azetidin-3-yl}-methansulfonylamino)-5-fluorbenzoesäure
3-({1-[Bis(4-trifluormethoxyphenyl)methyl] azetidin-3-yl}methansulfonylamino)-5-fluorbenzoe-säuremethylester
3-({1-[Bis(4-trifluormethoxyphenyl)methyl]-azetidin-3-yl}methansulfonylamino)-5-fluorbenzoesäure
3-({1-[Bis(4-chlorphenyl)methyl]azetidin-3-yl}-methansulfonylamino)-2-fluorbenzoesäuremethylester
3-({1-[Bis(4-chlorphenyl)methyl]azetidin-3-yl}-methansulfonylamino)-2-fluorbenzoesäure
3-({1-[Bis(4-chlorphenyl)methyl]azetidin-3-yl}-methansulfonylamino)-4-fluorbenzoesäureethylester
3-({1-[Bis(4-chlorphenyl)methyl]azetidin-3-yl}-methansulfonylamino)-4-fluorbenzoesäure
3-({1-[Bis(4-chlorphenyl)methyl]azetidin-3-yl}-methansulfonylamino)-6-fluorbenzoesäuremethylester
3-({1-[Bis(4-chlorphenyl)methyl]azetidin-3-yl}-methansulfonylamino)-6-fluorbenzoesäure
3-({1-[Bis(4-chlorphenyl)methyl]azetidin-3-yl}-methansulfonylamino)-5-methoxybenzoesäuremethylester
3-({1-[Bis(4-chlorphenyl)methyl]azetidin-3-yl}-methansulfonylamino)-5-methoxybenzoesäure
3-({1-[Bis(4-chlorphenyl)methyl]azetidin-3-yl}-methansulfonylamino)-5-methylbenzoesäureallylester
3-({1-[Bis(4-chlorphenyl)methyl]azetidin-3-yl}-methansulfonylamino)-5-methylbenzoesäure
3-({1-[Bis(4-chlorphenyl)methyl]azetidin-3-yl}-methansulfonylamino)-5-chlorbenzoesäuremethylester
3-({1-[Bis(4-chlorphenyl)methyl]azetidin-3-yl}-methansulfonylamino)-5-chlorbenzoesäure
3-({1-[Bis(4-chlorphenyl)methyl]azetidin-3-yl}-methansulfonylamino)-5-brombenzoesäuremethylester
3-({1-[Bis(4-chlorphenyl)methyl]azetidin-3-yl}-methansulfonylamino)-5-brombenzoesäure
3-({1-[Bis(4-chlorphenyl)methyl]azetidin-3-yl}-methansulfonylamino)-5-trifluormethylbenzoesäure-methylester
3-({1-[Bis(4-chlorphenyl)methyl]azetidin-3-yl}-methansulfonylamino)-5-trifluormethylbenzoesäure
3-({1-[Bis(4-chlorphenyl)methyl]azetidin-3-yl}-methansulfonylamino)-5-cyanobenzoesäuremethylester
3-({1-[Bis(4-chlorphenyl)methyl]azetidin-3-yl}-methansulfonylamino)-5-cyanobenzoesäure.

## Claims

1. Compound of formula (I) 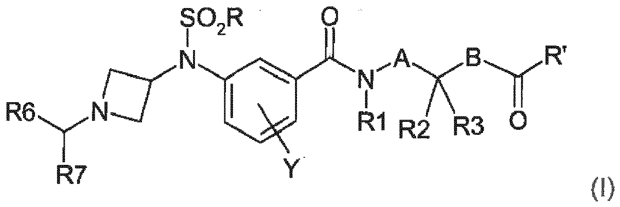 in which:
R is a (C₁-C₆)alkyl or halo(C₁-C₆)alkyl group;
R' is an NR4R5 or OR8 group;
A and B, if they are present, are, independently of one another, one or two carbon atoms, these carbon atoms being substituted with one or more hydrogens or (C₁-C₆)alkyl groups; the (C₁-C₆)alkyl group(s) being optionally substituted with one or more hydroxyl, (C₁-C₆)alkoxy, halo(C₁-C₆)alkyl, (C₁-C₆)alkylS(O)ₚ, NR4R5 or CONR4R5 groups;
A+B is at most two carbons;
R1 is a hydrogen atom or a (C₁-C₆)alkyl group;
R2 and R3 are, independently of one another, a hydrogen atom or a (C₁-C₆)alkyl group; the (C₁-C₆)alkyl group being optionally substituted with one or more hydroxyl, (C₁-C₆)alkoxy, halo(C₁-C₆)alkyl, (C₁-C₆)alkylS(O)ₚ, NR4R5 or CONR4R5 groups;
R4 and R5 are, independently of one another, a hydrogen atom or a (C₁-C₆)alkyl group, or form, together with the nitrogen atom which bears them, a heterocycle of the azetidine, pyrrolidine, piperidine, azepane, piperazine, homopiperazine, morpholine, thiomorpholine, thiomorpholine S-oxide or thiomorpholine S-dioxide type, this heterocycle being optionally substituted with a (C₁-C₆)alkyl group;
R6 and R7 are each a phenyl group, optionally substituted with one or more atoms or groups chosen from a hydrogen atom, a halogen, and a (C₁-C₆)alkyl, halo(C₁-C₆)alkyl, (C₁-C₆)alkoxy, halo(C₁-C₆)alkoxy or cyano group;
Y is a hydrogen atom, a halogen, or a (C₁-C₆)alkyl, halo(C₁-C₆)alkyl, (C₁-C₆)alkoxy, halo(C₁-C₆)alkoxy, (C₁-C₆)alkylS(O)ₚ or cyano group;
R8 is a hydrogen atom, a (C₁-C₆)alkyl group, a halo(C₁-C₆)alkyl group, an allyl group or a phenyl(C₁-C₆)alkyl group, the phenyl group being optionally substituted with 1 or 2 O-methyl groups;
p is an integer chosen from 0, 1 or 2;
in the form of a base or of an addition salt with an acid or with a base.

2. Compound of formula (I) according to Claim 1, **characterized in that**:
R' is an NR4R5 or OR8;
R is a methyl;
A and B, if they are present, are, independently of one another, a -CH₂-;
R1 is a hydrogen atom;
R2 and R3 are, independently of one another, a hydrogen atom or a (C₁-C₆)alkyl group; the (C₁-C₆)alkyl group being optionally substituted with a hydroxyl group;
R4 and R5 are, independently of one another, a hydrogen atom or a methyl, or form, together with the nitrogen atom which bears them, a morpholine;
R6 and R7 are each a phenyl group, optionally substituted with one or more atoms or groups chosen from a halogen, and a (C₁-C₆)alkyl, halo(C₁-C₆)alkyl, (C₁-C₆)alkoxy, halo(C₁-C₆)alkoxy or cyano group, in the para-position;
R8 is a hydrogen atom or a (C₁-C₆)alkyl group;
Y is a hydrogen, a halogen, a (C₁-C₆)alkoxy group; a (C₁-C₆)alkyl group; a cyano; or a halo(C₁-C₆)alkyl group; in the form of enantiomers or diastereoisomers or of mixtures of these forms;
in the form of a base or of an addition salt with an acid or with a base.

3. Compound of formula (I) according to Claim 1, **characterized in that**:
R' is an NR4R5 or OR8;
R is a methyl;
A and B, if they are present, are, independently of one another, a -CH₂-;
R1 is a hydrogen atom;
R2 and R3 are, independently of one another, a hydrogen atom or a (C₁-C₆)alkyl group; the (C₁-C₆)alkyl group being optionally substituted with a hydroxyl group;
R4 and R5 are, independently of one another, a hydrogen atom or a methyl, or form, together with the nitrogen atom which bears them, a morpholine;
R6 and R7 are each a phenyl group substituted with a chlorine, fluorine or bromine atom or a Me, OMe, CN, CF₃ or OCF₃ group, in the para-position;
R8 is a hydrogen atom or a (C₁-C₆)alkyl group;
Y is a hydrogen, a halogen, a (C₁-C₆)alkoxy group; a (C₁-C₆)alkyl group; a cyano; or a halo(C₁-C₆)alkyl group; in the form of enantiomers or diastereoisomers or of mixtures of these forms;
in the form of a base or of an addition salt with an acid or with a base.

4. Compound of formula (I) according to Claim 1, **characterized in that**:
R' is an NR4R5 or OR8;
R is a methyl;
A and B, if they are present, are, independently of one another, a -CH₂-;
R1 is a hydrogen atom;
R2 and R3 are, independently of one another, a hydrogen atom or a (C₁-C₆)alkyl group; the (C₁-C₆)alkyl group being optionally substituted with a hydroxyl group;
R4 and R5 are, independently of one another, a hydrogen atom or a methyl, or form, together with the nitrogen atom which bears them, a morpholine;
R6 and R7 are each a phenyl group, optionally substituted with one or more atoms or groups chosen from a halogen, and a (C₁-C₆)alkyl, halo(C₁-C₆)alkyl, (C₁-C₆)alkoxy, halo(C₁-C₆)alkoxy or cyano group, in the para-position;
R8 is a hydrogen atom or a (C₁-C₆)alkyl group;
Y is a hydrogen, chlorine, fluorine or bromine atom or a Me, OMe, CN or CF₃ group;
in the form of enantiomers or diastereoisomers or of mixtures of these forms;
in the form of a base or of an addition salt with an acid or with a base.

5. Compound of formula (I) according to Claim 1, **characterized in that**:
R' is an NR4R5 or OR8;
R is a methyl;
A and B, if they are present, are, independently of one another, a -CH₂-;
R1 is a hydrogen atom;
R2 and R3 are, independently of one another, a hydrogen atom or a (C₁-C₆)alkyl group; the (C₁-C₆)alkyl group being optionally substituted with a hydroxyl group;
R4 and R5 are, independently of one another, a hydrogen atom or a methyl or form, together with the nitrogen atom which bears them, a morpholine;
R6 and R7 are each a phenyl group substituted with a chlorine, fluorine or bromine atom or a Me, OMe, CN, CF₃ or OCF₃ group, in the para-position;
R8 is a hydrogen atom or a (C₁-C₆)alkyl group;
Y is a hydrogen, chlorine, fluorine or bromine atom or a Me, OMe, CN or CF₃ group,
in the form of enantiomers or diastereoisomers or of mixtures of these forms;
in the form of a base or of an addition salt with an acid or with a base.

6. Compound of formula (I) according to Claim 1, chosen from:
3-({1-[bis(4-chlorophenyl)methyl]azetidin-3-yl}methanesulphonylamino)-*N*-((*S*)-1-carbamoylethyl)benzamide
3-({1-[bis(4-chlorophenyl)methyl]azetidin-3-yl}methanesulphonylamino)-*N*-carbamoylmethyl-5-fluorobenzamide
3-({1-[bis(4-chlorophenyl)methyl]azetidin-3-yl}methanesulphonylamino)-*N*-((*S*)-1-carbamoylethyl)-5-fluorobenzamide
3-({1-[bis(4-chlorophenyl)methyl]azetidin-3-yl}methanesulphonylamino)-*N*-((R)-1-carbamoylethyl)-5-fluorobenzamide
3-({1-[bis(4-chlorophenyl)methyl]azetidin-3-yl}methanesulphonylamino)-*N*-((R)-1-carbamoylethyl)benzamide
3-({1-[bis(4-chlorophenyl)methyl]azetidin-3-yl}methanesulphonylamino)-*N*-((*S*)-1-carbamoyl-2-hydroxyethyl)benzamide
3-({1-[bis(4-chlorophenyl)methyl]azetidin-3-yl}methanesulphonylamino)-*N*-(2-carbamoylethyl)benzamide
3-({1-[bis(4-chlorophenyl)methyl]azetidin-3-yl}methanesulphonylamino)-*N*-(2-morpholin-4-yl-2-oxoethyl)benzamide
3-({1-[bis(4-chlorophenyl)methyl]azetidin-3-yl}methanesulphonylamino)-*N*-((*S*)-1-methylcarbamoylethyl)benzamide
3-({1-[bis(4-chlorophenyl)methyl]azetidin-3-yl}methanesulphonylamino)-*N*-((*S*)-1-carbamoylpropyl)benzamide
3-({1-[bis(4-chlorophenyl)methyl]azetidin-3-yl}methanesulphonylamino)-*N*-((*S*)-1-carbamoyl-3-methylbutyl)benzamide
3-({1-[bis(4-chlorophenyl)methyl]azetidin-3-yl}methanesulphonylamino)-*N*-((*S*)-1-carbamoyl-2-methylpropyl)-5-fluorobenzamide
3-({1-[bis(4-chlorophenyl)methyl]azetidin-3-yl}methanesulphonylamino)-*N*-((*S*)-1-carbamoyl-2-hydroxyethyl)-5-fluorobenzamide
3-({1-[bis(4-chlorophenyl)methyl]azetidin-3-yl}methanesulphonylamino)-*N*-((*S*)-1-carbamoylpropyl)-5-fluorobenzamide
3-({1-[bis(4-chlorophenyl)methyl]azetidin-3-yl}methanesulphonylamino)-*N-*(methylcarbamoylmethyl)benzamide
3-({1-[bis(4-chlorophenyl)methyl]azetidin-3-yl}methanesulphonylamino)-*N*-(carbamoylmethyl)benzamide
(*S*)-2-[3-({1-[bis(4-chlorophenyl)methyl]azetidin-3-yl}methanesulphonylamino)benzoylamino]propionic acid tert-butyl ester
(*S*)-2-[3-({1-[bis(4-chlorophenyl)methyl]azetidin-3-yl}methanesulphonylamino)benzoylamino]propionic acid
3-({1-[bis(4-chlorophenyl)methyl]azetidin-3-yl}methanesulphonylamino)benzoylaminoacetic acid tert-butyl ester
4-[3-({1-[bis(4-chlorophenyl)methyl]azetidin-3-yl}methanesulphonylamino)-5-fluorobenzoylamino]butyric acid tert-butyl ester
(*S*)-2-[3-({1-[bis(4-chlorophenyl)methyl]azetidin-3-yl}methanesulphonylamino)-5-fluorobenzoylamino]propionic acid tert-butyl ester
3-({1-[bis(4-chlorophenyl)methyl]azetidin-3-yl}methanesulphonylamino)-*N*-((*S*)-1-carbamoylethyl)-5-methoxybenzamide
3-({1-[bis(4-chlorophenyl)methyl]azetidin-3-yl}methanesulphonylamino)-*N*-((*S*)-1-dimethylcarbamoylethyl)benzamide
4-[3-({1-[bis(4-chlorophenyl)methyl]azetidin-3-yl}methanesulphonylamino)benzoylamino]butyric acid tert-butyl ester
(*S*)-2-[3-({1-[bis(4-chlorophenyl)methyl]azetidin-3-yl}methanesulphonylamino)-5-fluorobenzoylamino]propionic acid and the sodium salt thereof
3-({1-[bis(4-chlorophenyl)methyl]azetidin-3-yl}methanesulphonylamino)-5-fluoro-*N*-((*S*)-1-methylcarbamoylethyl)benzamide
3-({1-[bis(4-chlorophenyl)methyl]azetidin-3-yl}methanesulphonylamino)-*N*-((1*S*,2R)-1-carbamoyl-2-hydroxypropyl)benzamide
3-({1-[bis(4-chlorophenyl)methyl]azetidin-3-yl}methanesulphonylamino)-*N*-(3-carbamoylpropyl)-5-fluorobenzamide
[3-({1-[bis(4-chlorophenyl)methyl]azetidin-3-yl}methanesulphonylamino)benzoylamino]acetic acid
3-({1-[bis(4-chlorophenyl)methyl]azetidin-3-yl}methanesulphonylamino)-*N*-((*S*)-1-carbamoylethyl)-2-fluorobenzamide
3-({1-[bis(4-chlorophenyl)methyl]azetidin-3-yl}methanesulphonylamino)-*N*-((*S*)-1-methylcarbamoylethyl)-2-fluorobenzamide
3-({1-[bis(4-chlorophenyl)methyl]azetidin-3-yl}methanesulphonylamino)-*N*-((*S*)-1-carbamoylethyl)-6-fluorobenzamide
3-({1-[bis(4-chlorophenyl)methyl]azetidin-3-yl}methanesulphonylamino)-*N*-((*S*)-1-carbamoylpropyl)-6-fluorobenzamide
3-({1-[bis(4-chlorophenyl)methyl]azetidin-3-yl}methanesulphonylamino)-*N*-((*S*)-1-carbamoyl-2-methylpropyl)-6-fluorobenzamide
3-({1-[bis(4-chlorophenyl)methyl]azetidin-3-yl}methanesulphonylamino)-*N*-((*S*)-1-methylcarbamoylethyl)-6-fluorobenzamide
3-({1-[bis(4-chlorophenyl)methyl]azetidin-3-yl}methanesulphonylamino)-*N*-((*S*)-1-carbamoylpropyl)-4-fluorobenzamide
3-({1-[bis(4-chlorophenyl)methyl]azetidin-3-yl}methanesulphonylamino)-*N*-carbamoylmethyl-5-methylbenzamide
3-({1-[bis(4-chlorophenyl)methyl]azetidin-3-yl}methanesulphonylamino)-*N*-((*S*)-1-carbamoylethyl)-5-methylbenzamide
3-({1-[bis(4-chlorophenyl)methyl]azetidin-3-yl}methanesulphonylamino)-*N*-((*S*)-1-carbamoylpropyl)-5-methylbenzamide
3-({1-[bis(4-chlorophenyl)methyl]azetidin-3-yl}methanesulphonylamino)-*N*-((*S*)-1-carbamoyl-2-methylpropyl)-5-methylbenzamide
3-({1-[bis(4-chlorophenyl)methyl]azetidin-3-yl}methanesulphonylamino)-*N*-((*S*)-1-methylcarbamoylethyl)-5-methylbenzamide
3-({1-[bis(4-chlorophenyl)methyl]azetidin-3-yl}methanesulphonylamino)-*N*-((*S*)-1-carbamoyl-2-hydroxyethyl)-5-methylbenzamide
3-({1-[bis(4-chlorophenyl)methyl]azetidin-3-yl}methanesulphonylamino)-*N*-((*S*)-1-carbamoylethyl)-5-chlorobenzamide
3-({1-[bis(4-chlorophenyl)methyl]azetidin-3-yl}methanesulphonylamino)-*N*-((*S*)-1-carbamoylpropyl)-5-chlorobenzamide
3-({1-[bis(4-chlorophenyl)methyl]azetidin-3-yl}methanesulphonylamino)-*N*-((*S*)-1-carbamoyl-2-methylpropyl)-5-chlorobenzamide
3-({1-[bis(4-chlorophenyl)methyl]azetidin-3-yl}methanesulphonylamino)-*N*-((*S*)-1-methylcarbamoylethyl)-5-chlorobenzamide
3-({1-[bis(4-chlorophenyl)methyl]azetidin-3-yl}methanesulphonylamino)-*N*-((*S*)-1-carbamoyl-2-hydroxyethyl)-5-chlorobenzamide
3-({1-[bis(4-chlorophenyl)methyl]azetidin-3-yl}methanesulphonylamino)-*N*-((*S*)-1-carbamoylethyl)-5-bromobenzamide
3-({1-[bis(4-chlorophenyl)methyl]azetidin-3-yl}methanesulphonylamino)-*N*-((*S*)-1-carbamoylpropyl)-5-bromobenzamide
3-({1-[bis(4-chlorophenyl)methyl]azetidin-3-yl}methanesulphonylamino)-*N*-((*S*)-1-carbamoyl-2-methylpropyl)-5-bromobenzamide
3-({1-[bis(4-chlorophenyl)methyl]azetidin-3-yl}methanesulphonylamino)-*N*-((*S*)-1-methylcarbamoylethyl)-5-bromobenzamide
3-({1-[bis(4-chlorophenyl)methyl]azetidin-3-yl}methanesulphonylamino)-*N*-((*S*)-1-carbamoyl-2-hydroxyethyl)-5-bromobenzamide
3-({1-[bis(4-chlorophenyl)methyl]azetidin-3-yl}methanesulphonylamino)-*N*-carbamoylmethyl-5-trifluoromethylbenzamide
3-({1-[bis(4-chlorophenyl)methyl]azetidin-3-yl}methanesulphonylamino)-*N*-((*S*)-1-carbamoylethyl)-5-trifluoromethylbenzamide
3-({1-[bis(4-chlorophenyl)methyl]azetidin-3-yl}methanesulphonylamino)-*N*-((*S*)-1-carbamoylpropyl)-5-trifluoromethylbenzamide
3-({1-[bis(4-chlorophenyl)methyl]azetidin-3-yl}methanesulphonylamino)-*N*-((*S*)-1-carbamoyl-2-methylpropyl)-5-trifluoromethylbenzamide
3-({1-[bis(4-chlorophenyl)methyl]azetidin-3-yl}methanesulphonylamino)-*N*-((*S*)-1-methylcarbamoylethyl)-5-trifluoromethylbenzamide
3-({1-[bis(4-chlorophenyl)methyl]azetidin-3-yl}methanesulphonylamino)-*N*-((*S*)-1-carbamoyl-2-hydroxyethyl)-5-trifluoromethylbenzamide
3-({1-[bis(4-chlorophenyl)methyl]azetidin-3-yl}methanesulphonylamino)-*N*-((*S*)-1-carbamoylethyl)-5-cyanobenzamide
3-({1-[bis(4-chlorophenyl)methyl]azetidin-3-yl}methanesulphonylamino)-*N*-((*S*)-1-carbamoylpropyl)-5-cyanobenzamide
3-({1-[bis(4-chlorophenyl)methyl]azetidin-3-yl}methanesulphonylamino)-*N*-((*S*)-1-carbamoyl-2-methylpropyl)-5-cyanobenzamide
3-({1-[bis(4-chlorophenyl)methyl]azetidin-3-yl}methanesulphonylamino)-*N*-((*S*)-1-methylcarbamoylethyl)-5-cyanobenzamide
3-({1-[bis(4-chlorophenyl)methyl]azetidin-3-yl}methanesulphonylamino)-*N*-((*S*)-1-carbamoyl-2-hydroxyethyl)-5-cyanobenzamide
(*S*)-2-[3-({1-[bis(4-chlorophenyl)methyl]azetidin-3-yl}methanesulphonylamino)-6-fluorobenzoylamino]propionic acid and the trifluoroacetic acid salt thereof
(*S*)-2-[3-({1-[bis(4-chlorophenyl)methyl]azetidin-3-yl}methanesulphonylamino)-5-methylbenzoylamino]propionic acid and the trifluoroacetic acid salt thereof
(*S*)-2-[3-({1-[bis(4-chlorophenyl)methyl]azetidin-3-yl}methanesulphonylamino)-5-chlorobenzoylamino]propionic acid and the trifluoroacetic acid salt thereof
(*S*)-2-[3-({1-[bis(4-chlorophenyl)methyl]azetidin-3-yl}methanesulphonylamino)-5-bromobenzoylamino]propionic acid and the trifluoroacetic acid salt thereof
(*S*)-2-[3-({1-[bis(4-chlorophenyl)methyl]azetidin-3-yl}methanesulphonylamino)-3-trifluoromethylbenzoylamino]propionic acid and the trifluoroacetic acid salt thereof
(*S*)-2-[3-({1-[bis(4-chlorophenyl)methyl]azetidin-3-yl}methanesulphonylamino)-5-cyanobenzoylamino]propionic acid and the trifluoroacetic acid salt thereof
3-({1-[bis(4-bromophenyl)methyl]azetidin-3-yl}methanesulphonylamino)-*N*-((*S*)-1-carbamoylethyl)-5-fluorobenzamide
3-({1-[bis(4-trifluoromethylphenyl)methyl]azetidin-3-yl}methanesulphonylamino)-*N*-((*S*)-1-carbamoylethyl)-5-fluorobenzamide
3-({1-[bis(4-cyanophenyl)methyl]azetidin-3-yl}methanesulphonylamino)-*N*-((*S*)-1-carbamoylethyl)-5-fluorobenzamide
3-({1-[bis(4-methoxyphenyl)methyl]azetidin-3-yl}methanesulphonylamino)-*N*-((*S*)-1-carbamoylethyl)-5-fluorobenzamide
3-({1-[bis(4-fluorophenyl)methyl]azetidin-3-yl}methanesulphonylamino)-*N*-((*S*)-1-carbamoylethyl)-5-fluorobenzamide
(*S*)-2-[3-({1-[bis(4-chlorophenyl)methyl]azetidin-3-yl}methanesulphonylamino)-5-fluorobenzoylamino]propionic acid methyl ester
3-({1-[bis(4-methylphenyl)methyl]azetidin-3-yl}methanesulphonylamino)-*N*-((*S*)-1-carbamoylpropyl)-5-fluorobenzamide
3-({1-[bis(4-bromophenyl)methyl]azetidin-3-yl}methanesulphonylamino)-*N*-((*S*)-1-methylcarbamoylethyl)-5-fluorobenzamide
(*S*)-2-[3-({1-[bis(4-bromophenyl)methyl]azetidin-3-yl}methanesulphonylamino)-5-fluorobenzoylamino]propionic acid
(*S*)-2-[3-({1-[bis(4-chlorophenyl)methyl]azetidin-3-yl}methanesulphonylamino)-5-fluorobenzoylamino]propionic acid ethyl ester
3-({1-[bis(4-trifluoromethoxyphenyl)methyl]azetidin-3-yl}methanesulphonylamino)-*N*-((*S*)-1-carbamoylpropyl)-5-fluorobenzamide
the optical isomers thereof and the pharmaceutically acceptable salts thereof.

7. Medicament, **characterized in that** it comprises a compound of formula (I) as defined in Claims 1 to 6.

8. Pharmaceutical composition, **characterized in that** it comprises a compound of formula (I) as defined in Claims 1 to 6.

9. Use of a compound of formula (I) as defined in Claims 1 to 6, for the preparation of a medicament for the treatment or prevention of psychiatric disorders, substance dependence and withdrawal, tobacco withdrawal, cognitive and attention disorders, and acute and chronic neurodegenerative diseases.

10. Use of a compound of formula (I) as defined in Claims 1 to 6, for the preparation of a medicament for the treatment or prevention of metabolic disorders, appetence disorders, appetite disorders, obesity, diabetes, metabolic syndrome, dyslipidemia and sleep apnoea.

11. Use of a compound of formula (I) as defined in Claims 1 to 6, for the preparation of a medicament for the treatment or prevention of pain, neuropathic pain and neuropathic pain induced by anticancer drugs.

12. Use of a compound of formula (I) as defined in Claims 1 to 6, for the preparation of a medicament for the treatment or prevention of gastrointestinal disorders, vomiting, ulcers, diarrhoea disorders, bladder and urinary disorders, disorders of endocrine origin, cardiovascular disorders, hypotension, haemorrhagic shock, septic shock, liver diseases, chronic liver cirrhosis, fibrosis, non-alcoholic steatohepatitis (NASH), steatohepatitis and hepatic steatosis, irrespective of the etiology of these conditions (alcohol, medicament, chemical product, autoimmune disease, obesity, diabetes, congenital metabolic disease).

13. Use of a compound of formula (I) as defined in Claims 1 to 6, for the preparation of a medicament for the treatment or prevention of immune system diseases, rheumatoid arthritis, demyelination, multiple sclerosis and inflammatory diseases.

14. Use of a compound of formula (I) as defined in Claims 1 to 6, for the preparation of a medicament for the treatment or prevention of Alzheimer's disease, Parkinson's disease, schizophrenia and cognitive disorders associated with schizophrenia, with diabetes, with obesity or with metabolic syndrome.

15. Use of a compound of formula (I) as defined in Claims 1 to 6, for the preparation of a medicament for the treatment or prevention of asthma, chronic obstructive pulmonary diseases, Raynaud's syndrome, glaucoma and fertility disorders.

16. Use of a compound of formula (I) as defined in Claims 1 to 6, for the preparation of a medicament for the treatment or prevention of infectious and viral diseases such as encephalitis, cerebral strokes, Guillain-Barré syndrome, osteoporosis and sleep apnoea, and for anticancer chemotherapy.

17. Process for preparing compounds of formula (I) for which R' is NR4R5 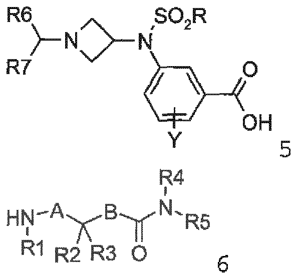 an acid derivative 5 and an amino derivative 6 are reacted in an inert solvent, in the presence of a coupling agent and optionally of an additive for preventing racemization, and the product is isolated and optionally converted to an addition salt with an acid.

18. Process for preparing compounds of formula (I) for which R' is OR8 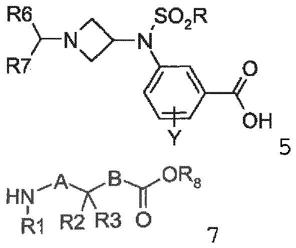 an acid derivative 5 and an amino derivative 7 are reacted in an inert solvent, in the presence of a coupling agent and optionally of an additive for preventing racemization, and the product is isolated and optionally converted to an addition salt with an acid.

19. Process for preparing compounds of formula (I) for which R' is OR8 and R8 is H, by hydrolysis of the OR8 function where R8 is a (C₁-C₆)alkyl group, a halo(C₁-C₆)alkyl group, an allyl group or a phenyl(C₁-C₆)alkyl group, the phenyl group being optionally substituted with 1 or 2 O-methyl groups; the product thus hydrolysed is isolated and optionally converted to an addition salt with a base.

20. Process for preparing compounds of formula (I) for which R' is NR4R5, by reacting a compound of formula (I) for which R' is OR8 and R8 is H and an amino derivative HNR4R5 in an inert solvent, in the presence of a coupling agent and optionally of an additive for preventing racemization, and the product is isolated and optionally converted to an addition salt with an acid.

21. Process for preparing compounds of formula 5 for which Y, R, R6 and R7 are as defined in Claim 1, by hydrolysis of the ester function of the compounds of formula 4 for which Y, R, R6 and R7 are as defined in Claim 1 and R'' is a (C₁-C₆)alkyl group, a halo(C₁-C₆)alkyl group, an allyl group or a phenyl(C₁-C₆)alkyl group, the phenyl group being optionally substituted with 1 or 2 O-methyl groups

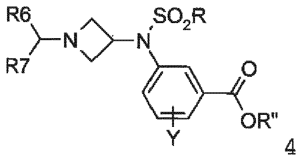

22. Intermediates for synthesizing compounds of formula (I)
3-({1-[bis(4-chlorophenyl)methyl]azetidin-3-yl}methanesulphonylamino)-5-fluorobenzoic acid methyl ester
3-({1-[bis(4-fluorophenyl)methyl]azetidin-3-yl}methanesulphonylamino)-5-fluorobenzoic acid methyl ester
3-({1-[bis(4-fluorophenyl)methyl]azetidin-3-yl}methanesulphonylamino)-5-fluorobenzoic acid
3-({1-[bis(4-bromophenyl)methyl]azetidin-3-yl}methanesulphonylamino)-5-fluorobenzoic acid ethyl ester
3-({1-[bis(4-bromophenyl)methyl]azetidin-3-yl}methanesulphonylamino)-5-fluorobenzoic acid
3-({1-[bis(4-trifluoromethylphenyl)methyl]azetidin-3-yl}methanesulphonylamino)-5-fluorobenzoic acid ethyl ester
3-({1-[bis(4-trifluoromethylphenyl)methyl]azetidin-3-yl}methanesulphonylamino)-5-fluorobenzoic acid
3-({1-[bis(4-methoxyphenyl)methyl]azetidin-3-yl}methanesulphonylamino)-5-fluorobenzoic acid ethyl ester
3-({1-[bis(4-methoxyphenyl)methyl]azetidin-3-yl}methanesulphonylamino)-5-fluorobenzoic acid
3-({1-[bis(4-methylphenyl)methyl]azetidin-3-yl}methanesulphonylamino)-5-fluorobenzoic acid methyl ester
3-({1-[bis(4-methylphenyl)methyl]azetidin-3-yl}methanesulphonylamino)-5-fluorobenzoic acid
3-({1-[bis(4-cyanophenyl)methyl]azetidin-3-yl}methanesulphonylamino)-5-fluorobenzoic acid ethyl ester
3-({1-[bis(4-cyanophenyl)methyl]azetidin-3-yl}methanesulphonylamino)-5-fluorobenzoic acid
3-({1-[bis(4-trifluoromethoxyphenyl)methyl]azetidin-3-yl}methanesulphonylamino)-5-fluorobenzoic acid methyl ester
3-({1-[bis(4-trifluoromethoxyphenyl)methyl]azetidin-3-yl}methanesulphonylamino)-5-fluorobenzoic acid
3-({1-[bis(4-chlorophenyl)methyl]azetidin-3-yl}methanesulphonylamino)-2-fluorobenzoic acid methyl ester
3-({1-[bis(4-chlorophenyl)methyl]azetidin-3-yl}methanesulphonylamino)-2-fluorobenzoic acid
3-({1-[bis(4-chlorophenyl)methyl]azetidin-3-yl}methanesulphonylamino)-4-fluorobenzoic acid ethyl ester
3-({1-[bis(4-chlorophenyl)methyl]azetidin-3-yl}methanesulphonylamino)-4-fluorobenzoic acid
3-({1-[bis(4-chlorophenyl)methyl]azetidin-3-yl}methanesulphonylamino)-6-fluorobenzoic acid methyl ester
3-({1-[bis(4-chlorophenyl)methyl]azetidin-3-yl}methanesulphonylamino)-6-fluorobenzoic acid
3-({1-[bis(4-chlorophenyl)methyl]azetidin-3-yl}methanesulphonylamino)-5-methoxybenzoic acid methyl ester
3-({1-[bis(4-chlorophenyl)methyl]azetidin-3-yl}methanesulphonylamino)-5-methoxybenzoic acid
3-({1-[bis(4-chlorophenyl)methyl]azetidin-3-yl}methanesulphonylamino)-5-methylbenzoic allyl ester
3-({1-[bis(4-chlorophenyl)methyl]azetidin-3-yl}methanesulphonylamino)-5-methylbenzoic acid
3-({1-[bis(4-chlorophenyl)methyl]azetidin-3-yl}methanesulphonylamino)-5-chlorobenzoic acid methyl ester
3-({1-[bis(4-chlorophenyl)methyl]azetidin-3-yl}methanesulphonylamino)-5-chlorobenzoic acid
3-({1-[bis(4-chlorophenyl)methyl]azetidin-3-yl}methanesulphonylamino)-5-bromobenzoic acid methyl ester
3-({1-[bis(4-chlorophenyl)methyl]azetidin-3-yl}methanesulphonylamino)-5-bromobenzoic acid
3-({1-[bis(4-chlorophenyl)methyl]azetidin-3-yl}methanesulphonylamino)-5-trifluoromethylbenzoic acid methyl ester
3-({1-[bis(4-chlorophenyl)methyl]azetidin-3-yl}methanesulphonylamino)-5-trifluoromethylbenzoic acid
3-({1-[bis(4-chlorophenyl)methyl]azetidin-3-yl}methanesulphonylamino)-5-cyanobenzoic acid methyl ester
3-({1-[bis(4-chlorophenyl)methyl]azetidin-3-yl}methanesulphonylamino)-5-cyanobenzoic acid
